(19) Europäisches Patentamt
European Patent Office
Office européen des brevets

(11) Publication number: 0 233 686 A2

(12) EUROPEAN PATENT APPLICATION

(21) Application number: 87300191.1

(22) Date of filing: 09.01.87

(51) Int.Cl.[3]: C 07 C 93/14
C 07 C 91/14, C 07 C 103/44
C 07 C 101/42, C 07 C 147/12
C 07 D 307/79, A 61 K 31/135
A 61 K 31/16, A 61 K 31/24

(30) Priority: 11.01.86 GB 8600644
09.05.86 GB 8611345

(43) Date of publication of application:
26.08.87 Bulletin 87/35

(84) Designated Contracting States:
BE CH DE ES FR GB GR IT LI LU NL SE

(71) Applicant: BEECHAM GROUP PLC
Beecham House Great West Road
Brentford Middlesex TW8 9BD(GB)

(72) Inventor: Ainsworth, Anthony Trevor
Beecham Pharmaceuticals Coldharbour Road
The Pinnacles Harlow Essex CM19 5AD(GB)

(72) Inventor: Smith, David Glynn Beecham Pharmaceuticals
Great Burgh Yew Tree Bottom Road
Epsom Surrey KT18 5XQ(GB)

(74) Representative: Russell, Brian John et al,
European Patent Attorney Beecham Pharmaceuticals
Great Burgh Yew Tree Bottom Road
Epsom Surrey KT18 5XQ(GB)

(54) **Bis phenyl ethanol amines and bis phenyoxypropanolamines having a beta-agonist activity.**

(57) A compound of formula (I):

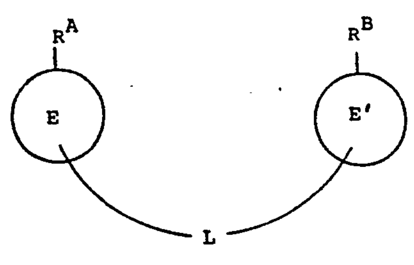

(1)

or a pharmaceutically acceptable salt, ester or amide thereof, wherein $R^A$ represents a moiety of formula (a):

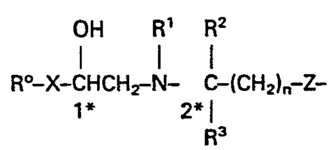

(a)

and $R^B$ represents a moiety of formula (b):

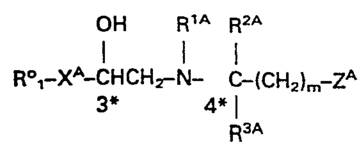

(b)

wherein

$R^o$ and $R^o_1$ each independently represents a substituted or unsubstituted aryl group or a substituted or unsubstituted benzofuranyl group,

X and $X^A$ each independently represents a bond or $-O-CH_2-$

$R^1$ represents a hydrogen atom or a moiety:

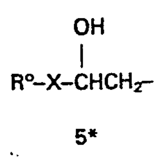

wherein X and $R^o$ are as defined above; ./...

EP 0 233 686 A2

Croydon Printing Company Ltd.

TITLE MODIFIED

0233686



B1995

# NOVEL COMPOUNDS

This invention relates to certain ethanolamine derivatives having β-agonist activity, to a process for preparing such compounds, to pharmaceutical compositions containing such compounds and to the use of such compounds and compositions in medicine and agriculture.

European Patent Application, Publication Number 0,196,849 discloses certain bridged bis(arylethanolamines) which are described as having activity as anti-obesity and/or anti-hyperglycaemic agents.

It has now been discovered that a novel series of arylethanolamine derivatives have β-agonist activity and show good anti-obesity and anti-hyperglycaemic activity coupled with good selectivity from cardiac side effects. These compounds also show potential as growth promoters for livestock and for decreasing birth mortality rate and increasing the post-natal survival rate in livestock.

These compounds may also be of use in increasing the high-density-lipoprotein (HDL) cholesterol concentration and decreasing the triglyceride concentration in human blood serum and are therefore of potential use in the treatment and/or prophylaxis of atherosclerosis.

Accordingly the present invention provides a compound of formula (I):

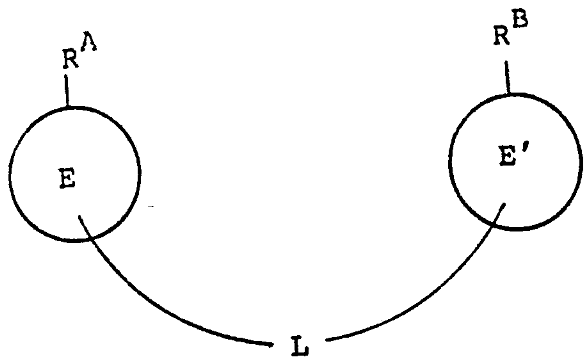

(I)

or a pharmaceutically acceptable salt, ester or amide thereof,

wherein $R^A$ represents a moiety of formula (a):

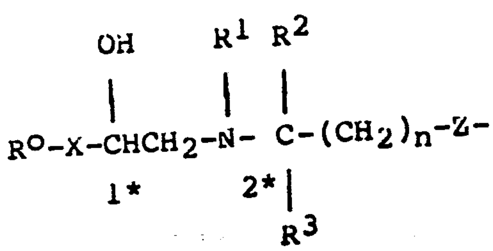

(a)

and $R^B$ represents a moiety of formula (b):

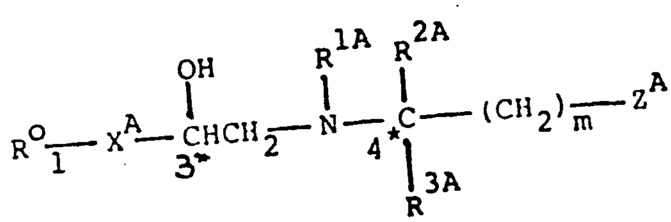

(b)

wherein
$R^o$ and $R^o{}_1$ each independently represents a substituted or unsubstituted aryl group or a substituted or unsubstituted benzofuranyl group,
X and $X^A$ each independently represents a bond or $-O-CH_2-$ ,
$R^1$ represents a hydrogen atom or a moiety:

OH
|
$R^o$-X-CHCH$_2$- wherein X and $R^o$ are as defined above;
5*

$R^{1A}$ represents a hydrogen atom or a moiety:

OH
|
$R^o{}_1$-$X^A$-CHCH$_2$- wherein $X^A$ and $R^o{}_1$ are as
6*

defined above;

$R^2$, $R^3$, $R^{2A}$ and $R^{3A}$ each independently represent a hydrogen atom or an alkyl group,

Z and $Z^A$ each independently represent a bond or a moiety $-CH_2-O-$,

n and m each independently represent an integer 1 or 2;

E and E′ each independently represent substituted or unsubstituted aryl; and L represents a linking moiety.

Preferably, $R^o$ and $R^o{}_1$ each independently represent a substituted or unsubstituted aryl group.

Suitable aryl groups include phenyl, naphthyl, phenylene and naphthylene groups optionally substituted with up to five, preferably up to three, groups selected from halogen, substituted or unsubstituted alkyl, alkenyl, alkynyl or phenyl; hydroxy, alkoxy, amino, nitro, nitrile or carboxy.

Preferably the aryl group $R^o$ or $R^o{}_1$ is a substituted or unsubstituted phenyl group.

Preferred optional substituents for the aryl group $R^o$ or $R^o{}_1$ include up to three substituents selected from halogen, hydroxy, $C_{1-6}$ alkoxy, hydroxy-$C_{1-6}$alkyl, amino, nitrile and trifluoromethyl.

When $R^o$ or $R^o{}_1$ represents a benzofuranyl group it is preferably a benzofuran-2-yl group.

Suitably E or $E^1$ represents a substituted or unsubstituted phenylene or naphthylene group; preferably a substituted or unsubstituted phenylene group.

Suitable substituents for any aryl group E or $E^1$ are those indicated in relation to the aryl groups $R^o$ or $R^o{}_1$.

When the benzofuranyl group is substituted, it is preferably substituted in the phenylene ring; a suitable substituent for the phenylene ring being a $C_{1-6}$ alkyl group. Suitably, the phenylene ring in the benzofuranyl moiety is substituted in the 7-position, suitably with a $C_{1-6}$ alkyl group such as for example methyl or ethyl.

Preferably, when $R^o$ represents a benzofuranyl group X represents a bond; preferably, when $R^o{}_1$ represents a benzofuranyl group $X^A$ represents a bond.

Suitably, X represents a bond. Suitably $X^A$ represents a bond.

Preferably X and $X^A$ both represent a bond.

Favourably, $R^1$ represents a hydrogen atom. Favourably $R^{1A}$ represents a hydrogen atom.

Preferably, $R^1$ and $R^{1A}$ both represent hydrogen.

Suitably, $R^2$ represents a $C_{1-6}$ alkyl group, preferably a methyl group. Suitably, $R^{2A}$ represents a $C_{1-6}$ alkyl group, preferably a methyl group.

Preferably, $R^2$ and $R^{2A}$ both represent methyl.

Suitably, $R^3$ represents a hydrogen atom. Suitably, $R^{3A}$ represents a hydrogen atom.

Preferably, $R^3$ and $R^{3A}$ both represent hydrogen.

Preferably, Z represents a bond. Preferably, $Z^A$ represents a bond.

Most preferably, Z and $Z^A$ both represent a bond.

Preferably, n represents the integer 1. Preferably, m represents the integer 1.

Most preferably, n and m both represent 1.

Suitably, $R^0$ and $R^0_1$ each independently represent a moiety of formula (c):

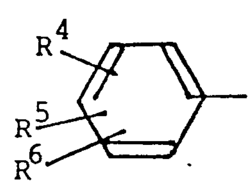

(c)

wherein $R^4$, $R^5$ and $R^6$ each independently represent hydrogen, halogen, alkyl, alkenyl, alkynyl, phenyl, alkoxy, trifluoromethyl, hydroxyalkyl, hydroxy, alkoxy amino, nitro, nitrile or carboxy.

Preferably, $R^4$, $R^5$, and $R^6$ each independently represent hydrogen, halogen, trifluoromethyl, amino or hydroxy.

Preferably, $R^4$ and $R^5$ both represent hydrogen.

In a particularly preferred aspect $R^4$ and $R^5$ both represent hydrogen and $R^6$ represents hydrogen, chlorine or trifluoromethyl.

Most preferably the moiety (c) represents phenyl, 3-chlorophenyl, 3-(trifluoromethyl)phenyl, 4-hydroxyphenyl or 3,5-dihydroxyphenyl; especially 3-chlorophenyl.

Preferably, $R^o = R^o{}_1$.
Favourably $R^A = R^B$.

In one preferred aspect the present invention provides a compound of formula (II):

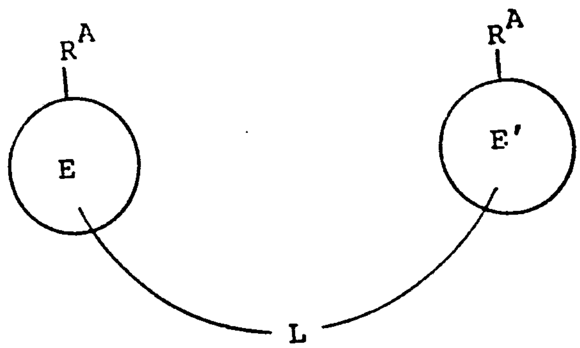

(II)

or a pharmaceutically acceptable salt, ester or amide thereof, wherein $R^A$, E, E' and L are as defined in relation to formula (I).

In a particularly preferred aspect the present invention provides a compound of formula (IIA):

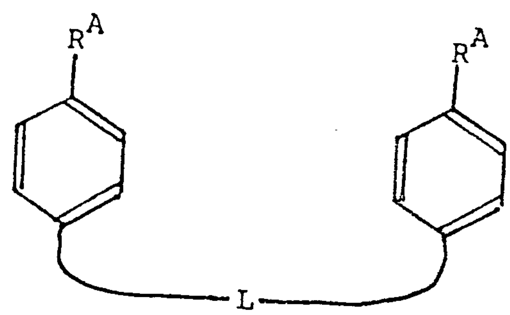

(IIA)

or a pharmaceutically acceptable salt, ester or amide thereof, wherein $R^A$ and L are as defined in relation to formula (I).

A suitable linking group L comprises a substituted or unsubstituted hydrocarbon; or a chain of at least two atoms in length comprising at least one hetero atom selected from oxygen or substituted or unsubstituted nitrogen or sulphur, or L represents oxygen, an amino group or SOz wherein z is zero or 1 or 2.

A suitable amino group is a group $>NR$ wherein R is hydrogen, alkyl, aryl, or alkylcarbonyl or aryl carbonyl.

A suitable substituent for the nitrogen atom is a group R defined in relation to $>NR$ above.

A suitable substituent for the sulphur atom is an oxo group.

A suitable linking group L comprises a substituted or unsubstituted hydrocarbon; or a chain of at least two

atoms in length comprising at least one hetero atom selected from oxygen or substituted or unsubstituted nitrogen or sulphur; or L represents oxygen or $SO_z$ wherein z is zero or 1 or 2.

Suitably, L represents a linking moiety attached to a carbon atom of a moiety E in the 3- or 4- position relative to $R^A$.

Suitably, L represents a linking moiety attached to a carbon atom of a moiety E′ in the 3- or 4- position relative to $R^B$.

Favourably, E represents phenylene and L represents a linking moiety attached to a phenylene carbon atom in the 3- or 4- position, relative to $R^A$.

Favourably , E represents phenylene and L represents a linking moiety attached to a phenyl carbon atom in the 3- or 4- position, relative to $R^B$.

More favourably, L represents a linking moiety linking the carbon atom in the 3- or 4- position, relative to $R^A$, to the carbon atom in the 3- or 4- position relative to $R^B$.

A suitable hydrocarbon linking moiety L, is a substituted or unsubstituted alkylene, alkenylene or alkynylene group, preferably a substituted or unsubstituted alkylene group.

A suitable linking moiety L is a chain of from 2 to 30 atoms in length comprising at least one hetero atom selected from oxygen, nitrogen or sulphur and a substituted or unsubstituted alkylene, alkenylene or alkynylene group, preferably an alkylene group.

Favourable linking moieties L are these comprising

-O-, -S-, -SO-, $-SO_2-$, -C(O)-, -CR(OH)-, -CO.O-, -CON(R′)- or -N(R′)-, wherein R represents hydrogen, alkyl or hydroxyalkyl and R′ represents hydrogen or alkyl, as part of the chain of from 2 to 30 atoms, especially as part of a chain also comprising a substituted or unsubstituted alkylene, alkenylene or alkynylene group.

Favourable linking moieties L are -O-, -S-, -SO or $-SO_2$.

Particularly favourable linking moieties L are those of formula $-X^1-X^2-X^3-$ wherein $X^1$ and $X^3$ each independently represent a bond, -C(O)-, RCOH, -CO.O- $-OX^{2A}CO_2-$, -CO.N(R′)-, $-X^{2A}CO.N(R')-$, $-OX^{2A}CO.N(R')-$, $-OX^{2B}O-$, $-X^2N(R')-$, $-OX^{2A}N(R')-$, O, S, $-SO_2$, -N(R′)-, $RC(OH)X^{2A}-$ or $-N(R')X^{2B}O-$; wherein R and R′are as defined above, $X^{2A}$ represents alkylene and $X^{2B}$ represents $C_{2-10}$ alkylene; and $X^2$ represents a substituted or unsubstituted alkylene, alkenylene, alkynylene or a moiety $-X^4-Z^1-X^5-$ wherein $X^4$ and $X^5$ each independently represent a bond, $C_{1-6}$ alkylene, $C_{2-6}$ alkenylene or $C_{2-6}$ alkynylene, and wherein $Z^1$ represents -O-, S, -SO, $-SO_2$ or -NR′ wherein R′ is defined above.

In particular $X_1$ or $X_3$ may independently represent a bond, -O-, -CO.O-, -CO.N(R')-, $-X^{2A}.CO.N(R')-$,

$-O-X^{2A}.CO.N(R')-$, $-O-X^{2A}N(R')-$ or $-X^{2}N(R')-$ wherein $X^2$, $X^{2A}$ and $R^1$ are as defined above.

Preferably, $X^1$ or $X^3$ represent -O-.

Most preferably, $X^1$ and $X^3$ both represent -O-.

In particular $X^2$ represents alkylene or a moiety $X^4-Z^1-X^5$ wherein $X^4$ and $X^5$ each independently represent $C_{1-6}$ alkylene and $Z^1$ represents -O-.

Preferably, $X^2$ represents alkylene.

Most preferably $X_2$ represents $-(CH_2)_6-$.

A preferred linking group L, is that represented by a moiety of the formula:

$-O(CH_2)_yCONH-(CH_2)_x-NHCO(CH_2)_yO-$,
$-O(CH_2)_yCONH-(CH_2)_x-NH(CH_2)_xO-$,
$-O(CH_2)_xNH-(CH_2)_x-NH(CH_2)_xO-$,
$-CONH(CH_2)_xNHCO-$,
$-(CH_2)_yCONH-(CH_2)_x-NHCO(CH_2)_y-$,
$-(CH_2)_yCONH-(CH_2)_x-NH(CH_2)_y-$,
$-(CH_2)_yNH-(CH_2)_x-NH(CH_2)_y-$,
$-O-(CH_2)_{y+1}-O-$,
$-(CH_2)_y-O-(CH_2)_y-$,
$-O(CH_2)_{y+1}-O-(CH_2)_{y+1}-O-$,
$-CO.O-(CH_2)_{y+1}-O.OC-$,
$-(CH_2)_y-$,
|
$-C(OH)-CH_2OH$,
-O-, or
$SO_z$;

wherein

x represents an integer from 2 to 6;
y represents an integer from 1 to 10, and
z represents zero or an integer 1 or 2.

Preferably x represents an integer 2, 3 or 4.

Preferably y represents an integer 1,2,3,4,5,6,7, or 8.

Preferably z represents the integer 2.

A particularly preferred linking moiety L is $O(CH_2)_yCO\ NH(CH_2)_x\ NH.CO.(CH_2)_yO-$ wherein x and y are as defined above; especially when x is 2, 3 or 4 and y is an integer from 1 to 6.

A particularly preferred linking moiety L is $O(CH_2)_xNH(CH_2)_xNH(CH_2)_xO-$ wherein x is as defined above; especially when x is 2, 3 or 4.

A particularly preferred linking moiety L is $(CH_2)_yNH(CH_2)_xNH(CH_2)_y$ wherein x and y are as defined above; specially when x is 2,3 or 4 and y is an integer from 1 to 6.

A particularly preferred linking moiety L is $-O-(CH_2)_{y+1}-O-$ wherein y is as defined above; especially when y is 1,2,3,4,5,6,7 or 8; and preferably when y is 5.

A particularly preferred linking moiety L is $-O-(CH_2)_{y+1}-O-(CH_2)_{y+1}-O-$ wherein y is as defined above; especially when y is 1,2,3,4,5,6,7 or 8.

A particularly preferred linking moiety L is $-CO.O-(CH_2)_{y+1}O.OC-$ wherein y is as defined above;

especially when y is an integer from 1 to 6.

A particularly preferred linking moiety is L $-(CH_2)_y-$ wherein y is as defined above; especially when y is an integer from 1 to 6.

A particularly preferred linking moiety L is

$$\begin{array}{l}\ |\\ -C(OH)CH_2OH.\end{array}$$

A particularly preferred linking moiety L is -O-.

A particularly preferred linking moiety L is $SO_z$ wherein z is as defined above; especially when z is 2.

In an especially preferred aspect the linking group L is $O-(CH_2)_{y+1}-O$ as defined above; preferably $-O-(CH_2)_6-O-$.

In a particularly preferred aspect the present invention provides a compound selected from the list consisting of:

[R,R,R,R]-N,N'-(1,2-ethanediyl)bis[2-[4-[2-[(2-(3-chlorophenyl)-2-hydroxyethyl)amino]propyl]phenoxy] acetamide];

[R,R,R,R]-α,α'[1,2-ethanediylbis(imino-2,1-ethanediyloxy-4,1-phenylene (1-methyl-2,1-ethanediyl) iminomethylene]]bis[3-chlorobenzenemethanol];

[R,R,R,R]-α,α'[1,6-hexanediylbis[oxy-4,1-phenylene(1-methyl-2,1-ethanediyl)iminomethylene]]bis[3-chlorobenzenemethanol],

[R,R,R,R]-α,α'-[oxybis[4,1-phenylene(1-methyl-2,1-ethanediyl)iminomethylene]]bis[3-chlorobenzenemethanol];

[R,R,R,R]-α,α'-[methylenebis[4,1-phenylene(1-methyl-2,1-ethanediyl)iminomethylene]]bis[3-chlorobenzenemethanol],

[R,R,R,R]-α,α'-[sulphonylbis[4,1-phenylene(1-methyl-2,1-ethanediyl)iminomethylene]]bis[3-chlorobenzenemethanol];

[R,R,R,R]-1,1-di[4-[2-[[2-(3-chlorophenyl)-2-hydroxyethyl]amino]propyl]phenyl]-1,2-ethanediol;

[R,R,R,R]-α,α'-[1,8-octanediylbis[oxy-4,1-phenylene (1-methyl-2,1-ethanediyl)iminomethylene]]bis[3-chlorobenzenemethanol];

1,2-ethanediyl di[4-[2-[[2-hydroxy-2-(3-trifluoromethyl)phenylethyl]amino]propyl]benzoate],

1,2-ethanediyl di[4-[2-[[2-hydroxy-2-phenylethyl]amino] propyl] benzoate];

[R,R,R,R,]-α,α'[1,4-butanediylbis[oxy-4,1-phenylene (1-methyl-2,1-ethanediyl)iminomethylene]]bis[3-chlorobenzenemethanol], dihydrochloride.

11,R,R,R,]-α,α'[1,3-propanediylbis[oxy-4,1-phenylene (1-methyl-2,1-ethanediyl)iminomethylene]]bis[3-chlorobenzenemethanol];

[R,R,R,R,]-α,α'[1,9-nonanediylbis[oxy-4,1-phenylene (1-methyl-2,1-ethanediyl)iminomethylene]]bis[3-chlorobenzenemethanol];

[R,R,R,R,]-3-chloro-α-[[[[2-[4-[6-[4-[2-[2-(4-hydroxyphenyl)-2-hydroxyethyl]amino]propyl]phenoxy]hexyloxy]phenyl]-1-methylethyl]amino]methyl]-benzenemethanol;

[R,R,R,R,]-α,α'-[1,2-ethanediylbis[iminomethylene-4,1-phenylene(1-methyl-2,1-ethanediyl)iminomethylene]]bis[3-chlorobenzenemethanol;

[R,R,R,R]-α,-α'-[1,4-butanediylbis[iminomethylene-4,1-phenylene(1-methyl-2,1-ethanediyl)iminomethylene]]bis[3-chlorobenzenemethanol;

[R,R,R,R]-α,α'-[1,6-hexanediylbis[iminomethylene-4,1-phenylene (1-methyl-2,1-ethanediyl)iminomethylene]]bis[3-chlorobenzenemethanol];

[R,R,R,R]-α,α'-[1,2-ethanediylbis[4,1-phenylene(1-methyl-2,1-ethanediyl)iminomethylene]]bis[3-chlorobenzenemethanol];

[R,R,R,R]-α,α'-[1,6-hexanediylbis[4,1-phenylene(1-methyl-2,1-ethanediyl)iminomethylene]]bis[3-chlorobenzenemethanol];

[R,R,R,R]-α,α'-[1,5-pentanediyl bis[oxy-4,1-phenylene(1-methyl-2,1-ethanediyl)iminomethylene]]bis[3-chlorobenzenemethanol];

[R,R,R,R]-α,α'-[oxybis[2,1-ethanediyloxy-4,1-phenylene(1-methyl-2,1-ethanediyl)iminomethylene]]bis[3-chlorobenzenemethanol];

[R,R,R,R]-5,5'-[1,6-hexanediylbis[oxy-4-1-phenylene(1-methyl-2,1-ethanediyl)imino(1-hydroxy-2,1-ethanediyl)]]bis[benzene-1,3-diol]; and

[R,R,R,R]-α,α'[1,7-heptanediylbis[oxy-4,1-phenylene-(1-methyl-2,1-ethanediyl)iminomethylene]]bis[3-chlorobenzenemethanol], or a pharmaceutically acceptable acid addition salt thereof.

The compounds of the general formula (I) may have, depending on the meaning of $R^1$, $R^2$, $R^3$, $R^{1A}$, $R^{2A}$ and $R^{3A}$ up to six asymmetric carbon atoms, marked 1* to 6* in the formula. These compounds may, therefore, exist in up to sixty four stereoisomeric forms. The present invention encompasses all stereoisomers of the compounds of the general formula (I) whether free from other isomers or admixed with other isomers in any proportion, and thus includes for instance, racemic mixtures of enantiomers.

The term 'hydrocarbon' includes groups having up to 18 carbon atoms, suitably up to 10 carbon atoms, conveniently up to 6 carbon atoms. Suitable hydrocarbon groups include alkylene, alkenylene, alkynylene, $C_{3-7}$ cycloalkyl, $C_{3-7}$ cycloalkyl alkylene, aryl, and aryl alkylene; preferably alkylene, alkenylene and alkynylene.

Suitably substituents for any hydrocarbon, especially any alkylene, alkenylene or alkynylene group, include those indicated above in relation to suitable aryl groups.

When used herein the term 'alkyl', 'alkenyl', 'alkynyl', 'alkylene', 'alkenylene', 'alkynylene' or 'alkoxy' relates to groups having straight or branched chains containing up to 10 carbon atoms, conveniently up to 6 carbon atoms.

Suitable pharmaceutically acceptable esters of compounds of formula (I) are esters of carboxy groups or hydroxy groups.

Favoured pharmaceutically acceptable esters are in-vivo hydrolysable esters of carboxy groups or hydroxy groups.

Suitable in-vivo hydrolysable esters of carboxy groups are those of formula -CO.OR wherein R represents a $C_{1-6}$ alkyl group.

Suitable pharmaceutically acceptable amide groups are those of formula $-CO.NR^sR^t$ wherein $R^s$ and $R^t$ each independently represent hydrogen or $C_{1-6}$ alkyl or $R^s$ and $R^t$ together with the nitrogen to which they are attached form a saturated 5- or 6- membered ring.

When used herein the term ''halogen'' refers to fluorine, chlorine, bromine and iodine, preferably chlorine.

Suitably the hydroxy group present in the moieties

OH
|
(X or $X^A$)-CHCH- or any hydroxyl group present in the compound of formula (I) may be derivatised as an ester, by for example, an aryl carboxylic acid, an arylalkyl carboxylic acid or a $C_{1-6}$ alkyl carboxylic acid. Suitable esters are in-vivo hydrolysable esters. Such esters and pharmaceutically acceptable salts of such esters form further aspects of the present invention.

When used herein the term ''in-vivo hydrolysable ester'' relates to a pharmaceutically acceptable ester

which readily breaks down in the human or non-human animal body to leave the free hydroxy group. Suitable *in-vivo* hydrolysable ester groups are those used conventionally in the art; they are preferably those provided by lower alkyl carboxylic acids.

Preferably the above mentioned hydroxyl groups are present as free hydroxyl groups.

The absolute configuration of any compound of the general formula (I) may be determined by conventional X-ray crystallographic techniques.

Suitably, when $R^2 \neq R^3$ the 2* asymmetric carbon has the R-configuration.

Suitably, when $R^{2A} \neq R^{3A}$ the 4* asymmetric carbon has the R-configuration.

Suitably, when X represents a bond, the 1* asymmetric carbon has the R-configuration.

Suitably, when X represents $-O-CH_2-$, the 1* asymmetric carbon has the S-configuration.

Suitably, when $X^A$ represents a bond, the 3* asymmetric carbon has the R-configuration.

Suitably, when $X^A$ represents $-O-CH_2$, the 3* asymmetric carbon has the S-configuration.

When $R^1 = R^{1A} = H$ , $R^2 = R^3$ and $R^{2A} \neq R^{3A}$, a preferred enantiomer of the compound of formula (I) is that wherein the asymmetric carbons 1*3*4* have the following configurations:

RRR, SRR, RSR or SSR.

When $R^1 = R^{1A} = H$, $R^2 \neq R^3$ and $R^{2A} \neq R^{3A}$, a preferred enantiomer of the compound of formula (I) is that wherein the asymmetric carbons 1*2*3*4* have the following configurations:

RRRR, SRRR, SRSR or RRSR.

When $R^1 \neq H$, $R^{1A} \neq H$, $R^2 \neq R^3$ and $R^{2A} \neq R^{3A}$, a preferred enantiomer of the compound of formula (I) is that wherein:

either 1* has the R-configuration when
X represents a bond, or
1* has the S-configuration when
X represents $-O-CH_2-$, and

either 3* has the R-configuration when
$X^A$ represents a bond, or
3* has the S-configuration when
$X^A$ represents $-O-CH_2$.

Suitable pharmaceutically acceptable salts of the compounds of formula (I) include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine or quinoline.

Compounds of the general formula (I) also form acid addition salts.

Pharmaceutically acceptable acid addition salts may be, for example, salts with inorganic acids such, for example, as hydrochloric acid, hydrobromic acid, orthophosphoric acid or sulphuric acid, or with organic acids such, for example as methanesulphonic acid, toluenesulophonic acid, acetic acid, propionic acid, lactic acid, citric acid, fumaric acid, malic acid, succinic acid, salicylic acid or acetylsalicylic acid.

A preferred acid addition salt is a hydrochloride.

Solvates, preferably hydrates, of the compound of formula (I) are also encompassed by the invention.

The invention also provides a process for the preparation of a compound of the general formula (I), or a pharmaceutically acceptable salt, ester or amide thereof, which process comprises reacting a compound of formula (III):

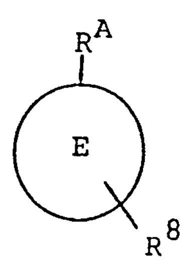

(III)

with a compound of formula (IV):

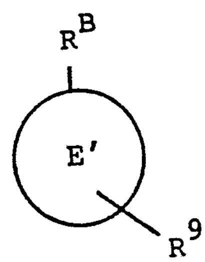

(IV)

wherein $R^A$ and $R^B$ are as defined in relation to formula (I) or may be protected forms thereof, E and E' are as defined in relation to formula (I); and

either $R^8$ represents a nucleophilic moiety and $R^9$ represents a moiety $- L^1 - R^x$ wherein $R^x$ represents a leaving moiety, $L^1$ representing a moiety such that $-R^8-L^1-$ represents the linking moiety L; or

$R^8$ represents the above defined moiety $L^1 - R^x$ and $R^9$ represents a nucleophilic moiety and $L^1$ is a moiety such that $-R^9-L^1$ represents the linking group L; and thereafter if necessary carrying out one or more of the following steps:

(i) removing any protecting group;

(ii) converting a compound of formula (I) into a further compound of formula (I);

(iii) converting a salt of formula (I) into a free compound of formula (I),

(iv) preparing a pharmaceutically acceptable ester or amide of a compound of formula (I);

(v) preparing a pharmaceutically acceptable salt of a compound of formula (I) or an ester or amide thereof.

The precise nature of $R^8$ and $R^9$ will of course depend upon the nature of the linking moiety L although any nucleophilic moiety or moiety $- L^1 - R^x$ which in the conventional art would provide the linking moiety L is encompassed by the above mentioned process.

The reaction between a compound of formula (III) and a compound of formula (IV) may be carried out under any suitable conditions appropriate to the nature of $R^8$ and $R^9$.

Preferably $R^x$ is a halogen atom, such as bromine, or an alkoxy group.

Suitable nucleophilic moieties $R^8$ or $R^9$ are nucleophilic groups comprising an anion such as $O^{\ominus}$, $S^{\ominus}$, $CO_2^{\ominus}$, $C{\equiv}C^{\ominus}$ or $^{\ominus}CH_2$; suitably the anions are provided in salted form, preferably with a metal cation such as sodium or as an appropriate Grignard Reagent.

Suitable nucleophilic moieties also include those wherein $R^8$ and $R^9$ represent a negative charge on a carbon atom of E or E' respectively; the said moiety suitably being provided as an appropriate Grignard Reagent.

Examples of suitable Grignard reagents are $(R^A-E-R^8)$ MgX and $(R^B-E-R^9)$ MgX wherein $R^A$, $R^B$, E and E' are as defined in relation to formula (I), $R^8$ and $R^9$ represent a negative charge on a carbon atom of E or E' repectively, and X represents a halide ion such as a bromide ion.

Further suitable nucleophilic moieties $R^8$ or $R^9$ are groups terminating in an amino group, preferably a primary amino group. Preferably when the nucleophilic moiety is a group terminating in an amino group, the moiety $-L^1-R^x$ is a group terminating in a halocarbonyl or alkoxycarbonyl group, the halo atom, preferably chlorine, or the alkoxy group, preferably ethoxy, being the leaving group $R^x$, thereby providing a moiety L comprising a -CO.NH- group.

A favourable nucleophilic group terminating in an amino group is a group terminating in an alkyleneamino group.

The nucleophilic moieties may be prepared by any conventional method appropriate to the nature of the rest of the molecule. Similarly the moiety $-L^1-R^x$ may be prepared by any convenient conventional method.

A preferred nucleophilic group $R^8$ or $R^9$ comprising an anion, is a moiety $(X^1)^\ominus$ or $(X^3)^\ominus$ wherein $X^1$ and $X^3$ are defined above; thus examples of preferred anionic nucleophilic groups are groups of formula $O^\ominus$, $S^\ominus$, $CO_2^\ominus$, $OX^{2A}CO_2^\ominus$, $-OX^{2B}O^\ominus$, or $-N(R)X^{2B}O^\ominus$ wherein $X^2$, $X^{2A}$, $X^{2B}$ and R are as defined above.

A further preferred nucleophilic group $R^8$ or $R^9$ comprising an anion is a group of formula $-X^1-X^{2\ominus}$ or $-X^3-X^{2\ominus}$ wherein $X^1$, $X^2$ and $X^3$ are as defined above.

A preferred nucleophilic moiety $R^8$ or $R^9$ terminating in an amino group is a group $-Y^1H$ wherein $Y^1$ is an appropriate moiety $X^1$ or $X^3$ such as $-N(R')-$, $-X^2N(R')-$ or $-OX^{2B}N(R')-$.

A further preferred nucleophilic moiety terminating in an amino group is a moiety $-X^1-X^2-NHR'$ or $-X^3-X^2-NHR'$ wherein $X^1$, $X^2$, $X^3$ and R' are as defined above.

When $X^1$ or $X^3$ represents a bond then the nucleophilic moiety $R^8$ or $R^9$ may be represented by a moiety $(X^2)^{\ominus}$; for example by moieties of formula $-(CH_2)_a-CH_2^{\ominus}$, $(CH_2)_b-C{\equiv}C^{\ominus}$ wherein a is zero or an integer 1 to 11 and b is zero or an integer 1 to 10.

When the moiety $R^8$ or $R^9$ represents $L^1-R^x$ then the value of $L^1$ depends upon the nature of the corresponding nucleophilic moiety in any reacting pair of compounds (III) and (IV).

Suitable values for the nucleophilic moiety and moiety $-L^1-R^x$ in any reacting pair of compounds (III) and (IV) are:

| nucleophilic moiety | $L^1-R^x$ |
|---|---|
| $-Y^1H$ | $R^x.OC.X^2-X^1-$ |
| $-Y^1H$ | $R^x.OC.X^2-X^3-$ |
| $-X^1-X^2-NHR'$ | $R^x.OC.Y^2-$ |
| $-X^3-X^2-NHR'$ | $R^x.OC.Y^2-$ |
| $-(X^1)^{\ominus}$ | $R^x-X^2-X^3-$ |
| $-X^1-(X^2)^{\ominus}$ | $R^x-X^3-$ |
| $-(X^3)^{\ominus}$ | $R^x-X^2-X^1-$ |
| $-X^3-(X^2)^{\ominus}$ | $R^x-X^1-$ . |

wherein $Y^1$, $X^1$, $X^2$, $X^3$, R' and $R^x$ are as defined above and $Y^2$ is a moiety such that $-Y^2-CO.NR'-$ is a moiety $X^1$ or $X^3$.

Particularly preferred values for the nucleophilic group and moiety $-L^1-R^x$ in any reacting pair of compounds (III) and (IV) are:

| nucleophilic moiety | $L^1-R^x$ |
|---|---|
| $-O(CH_2)_yCO.NH(CH_2)_x-NH_2$ | $R^x.CO.(CH_2)_xO-$ |
| $-O(CH_2)_xNH(CH_2)_x-NH_2$ | $R^x.CO.(CH_2)_xO-$ |
| $-CO.NH.(CH_2)_x-NH_2$ | $R^x.CO-$ |
| $-(CH_2)_yCO.NH(CH_2)_x-NH_2$ | $R^x.CO(CH_2)_y-$ |
| $-(CH_2)_yNH(CH_2)_x-NH_2$ | $R^x.CO.(CH_2)_y-$ |
| $-O^{\ominus}$ | $R^x.(CH_2)_{y+1}-O-$ |
| $-(CH_2)_y-O^{\ominus}$ | $R^x.(CH_2)_{y+1}-O-$ |
| $-O-(CH_2)_{y+1}-O^{\ominus}$ | $R^x.(CH_2)_{y+1}-O-$ |
| $-CO.O^{\ominus}$ | $R^x.(CH_2)_{y+1}.O.OC-$ |
| $(E \text{ or } E')^{\ominus}$ | $R^x-(CH_2)_y-$ |

wherein x, y, E and $E^1$ are as defined above.

Suitably, in the abovementioned reaction between compounds of formula (III) and (IV), $R^A = R^B$, thereby providing a process for preparing a compound of the hereinbefore defined formulae (II) and (IIA).

The compounds of the general formula (III) may be prepared by either:

(A) reacting a compound of the general formula (V)

$$T - Q \qquad (V)$$

(i) wherein T represents a moiety of formula $R^o-X-$ wherein $R^o$ and X are as defined in relation to formula (I), and Q represents a group of formula (c) or (d):

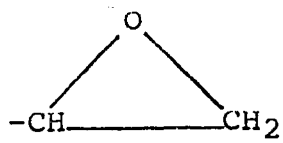 or 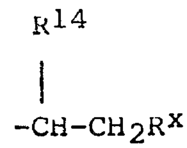

(c) (d)

wherein $R^{14}$ represents a hydroxyl group or a protected hydroxyl group, and $R^x$ represents a leaving group, with a compound of the general formula (VI):

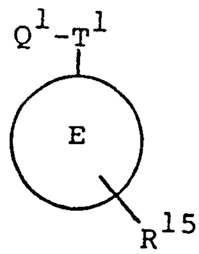

(VI)

wherein $T^1$ represents a moiety $(CH_2)_n$-Z wherein n and Z are as defined in relation to formula (I), $R^{15}$ represents a group $R^8$ or, preferably, a protected form thereof; and $Q^1$ represents a group of the formula (e):

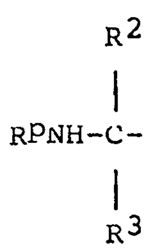

(e)

wherein $R^2$ and $R^3$ are as defined in relation to formula (I), and $R^p$ represents a hydrogen atom, a protecting group, preferably a benzyl group, or the hereinbefore defined moiety $R^1$; or

(ii) wherein T is as defined above and Q represents a group of formula (f):

$$-CO-CHO \qquad (f)$$

or (g):

$$\begin{array}{l} R^{14} \\ | \\ -CH-CO.OH \end{array} \qquad (g)$$

wherein $R^{14}$ is as defined above; with a compound of formula (VI) wherein $T^1$ is as defined above and in the moiety $Q^1$ the variables $R^2$, $R^3$ and $R^p$ are as defined above and subsequently treating with a reducing agent and if required carrying out reaction (B) below; or

(iii) wherein T is a defined above and Q represents a group of the formula (h):

$$\begin{array}{l} R^{14} \\ | \\ -CH-CH_2-NHR^p \end{array} \qquad (h)$$

wherein $R^{14}$ and $R^p$ have the meanings given above, with a compound of formula (VI) wherein $Q^1$ represents a group of the formula (j):

$$\begin{array}{c} R^2 \\ | \\ R^x-C- \\ | \\ R^3 \end{array} \qquad (j)$$

in which $R^2$, $R^3$ and $R^x$ have the meanings given above; or

(iv) wherein T is as defined above and Q represents a group of the formula (h) as defined above;

with a compound of formula (VI) wherein $Q^1$ represents a group of formula (k):

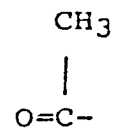 (k)

and subsequently treating with a reducing agent; or

(B) for compounds of formula (III) wherein $R^1$

OH

represents only the moiety $R^o$-X-$CHCH_2$- as defined above, by reacting a compound of formula (III) wherein $R^1$ represents a hydrogen atom, with either:

(i) a compound of formula (VA):

T - Q′ (VA)

wherein T is as defined above and Q′ represents a group of the hereinbefore defined formula (c) or (d); or

(ii) a compound of formula (VB):

T - Q′′

wherein T is as defined above and Q′ is a moiety (f) or (g) as defined above and subsequently treating with a reducing agent;

and thereafter if necessary carrying out one or more of the following steps;

i) removing any protecting group; or

ii) converting a compound of formula (III) into a further compound of formula (III).

Suitable protecting groups are those used conventionally in the art, for example $R^p$ is preferably a benzyl group and examples of groups $R^{15}$ as protected forms of groups $R^8$ are $-O-CH_2C_6H_5$ (convertible via conventional catalytic debenzylation to a salted hydroxyl group $R^8$) and $-CO_2R$, wherein R is $C_{1-6}$ alkyl, (convertible via conventional ester hydrolysis to a salted carboxyl group $R^8$).

Converting a compound of formula (III) into a further compound of formula (III) includes for example:

(i) converting $R^1$ in the moiety of formula (a), as defined above, from hydrogen to a moiety $R^0-X-CH(OH)CH_2-$, as defined above; or

(ii) converting one group $R^8$ to another group $R^8$.

Suitable methods of converting $R^1$ from hydrogen to $R^0-X-CH(OH)CH_2-$ include treating the appropriate compound of formula (III) with a compound of formula (V) as defined above, wherein Q represents a group of formula (c) or (d); preferably under the same reaction conditions as the analogous reaction between compounds of formula (V), wherein Q is (c) or (d), and compounds of formula (VI) wherein $Q^1$ is a moiety (e).

Suitable conversions of one group $R^8$ to another group $R^8$ include converting a group $R^8$ representing a nucleophilic moiety into a group $R^8$ representing a

moiety $L^1-R^x$, or converting a group $R^8$ representing a moiety $L^1-R^x$ into a nucleophilic moiety.

Suitably, when $R^8$ in a compound of formula (III) represents a nucleophilic moiety $-(X^1)^{\ominus}$, $-(X^3)^{\ominus}$ or $-Y^1H$ it may be converted into a moiety $L^1R^x$, wherein $-L^1$ is $-X^1-X^2-$, $-X^3-X^2$ or $-(X^1$ or $X^3)-X^2-CO-$ respectively, by treating the compound of formula (III) with a compound of formula (A):

$$R^z-X^2-R^z \qquad (A)$$

wherein $X^2$ is as defined above and $R^z$ is $R^x$ when $R^8$ is $-(X^1)^{\ominus}$ or $-(X^3)^{\ominus}$ or $R^z$ is $-COR^x$ when $R^8$ is $-Y^1H$.

Suitably when $R^8$ in a compound of formula (III) represents a moiety $-L^1-R^x$, such as the hereinbefore defined $-Y^2-CO-R^x$, it may be converted into a nucleophilic moiety $-Y^2-CO.N(R')-X^2-NHR'$ by treating the compound of formula (III) with a compound of formula (B):

$$R'HN-X^2-NHR' \qquad (B)$$

wherein $X^2$ and R' are as defined above.

In one preferred form of the process of the invention for preparing compounds of formula (I), wherein L is $-X^1-X^2-X^3-$, E = E' and $R^A = R^B$; a compound of formula (IIIA):

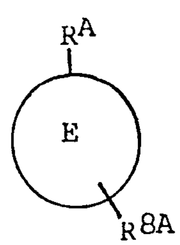

(IIIA)

wherein $R^A$ and E are as defined above, is reacted with a compound of formula (A) or (B), as defined above, providing that when compound (A) is used then $R^{8A}$ is an appropriate nucleophilic group $R^8$ and when compound B is used $R^{8A}$ is an appropriate moiety $-L^1-R^x$.

Suitably, when (A) is used and $R^z$ is $R^x$, then $R^8$ is $-(X^1)^{\ominus}$ or $-(X^3)^{\ominus}$.

Preferably, when (A) is used and $R^z$ is $R^x$, $R^8$ is $-CO_2^{\ominus}$, $O^{\ominus}$, or $S^{\ominus}$, suitably provided in salted form with for example an alkali metal cation such as a sodium ion, or $R^8$ represents a negative charge on a carbon atom of E, suitably provided as an appropriate Grignard reagent; most preferably $R^8$ is $O^{\ominus}$.

Suitably, when (A) is used and $R^z$ is $-CO.R^x$ then $R^8$ is $-Y^1H$.

Preferably, when (A) is used and $R^z$ is $-COR^x$, $R^8$ is $-OX^{2B}N(R')H$.

Suitably, when (B) is used, $R^8$ is $-Y^2.CO.R^x$ as defined above, preferably $R^8$ is $-OX^{2A}.CO.R^x$.

Preferably the molar ratio of (IIIA) to (A) or (B) is at least 2:1.

Preferably $X^2$ is alkylene especially $-(CH_2)_6-$.

A compound of formula (IV) may be prepared by using methods analogous to those used for preparing a compound of formula (III). Thus a compound of formula (IV) may be prepared by reacting a compound of formula

(V), wherein T represents a moiety $R^o{}_1-X^A$ wherein $R^o{}_1$ and $X^A$ are as defined in relation to formula (I); and Q is as defined above in relation to formula (V), with a compound of formula (VIA):

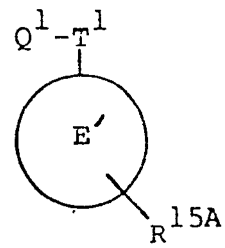

(VIA)

wherein $T^1$ represents a moiety $-(CH_2)_m-Z^A$ wherein m and $Z^A$ are as defined in relation to formula (I), and $R^{15A}$ represents a group $R^9$ or preferably a protected form thereof; and $Q^1$ is as defined above in relation to formula (VI).

The reactions between the compounds of formula (V) and (VIA) may be carried out under the same conditions as the analogous reaction between compounds of formula (V) and (VI).

Suitably, $R^{15A}$ is a protected form of a group $R^9$ as described above for $R^{15}$ in relation to $R^8$.

The present invention further provides a process for the preparation of a compound of formula (I) from a compound of formula (IX):

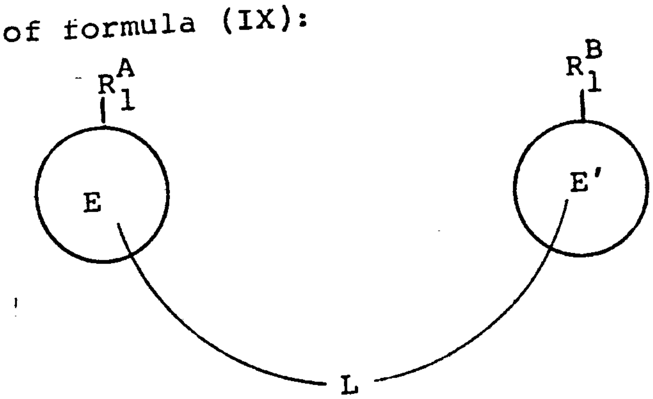

(IX)

wherein L, E and E' are as defined in relation to formula (I), $R^A{}_1$ represents $R^A$, as defined in relation to formula (I), or a moiety convertible to a moiety $R^A$, and $R^B{}_1$ represents $R^B$, as defined in relation to formula (I), or a moiety convertible to a group $R^B$; providing that $R^A{}_1$ is not $R^A$ when $R^B{}_1$ is $R^B$

which process comprises, where appropriate;

(a) converting any group $R^A{}_1$, to $R^A$; and/or

(b) converting any group $R^B{}_1$ to $R^B$;

and thereafter if necessary carrying out one or more of the following steps:

(i) removing any protecting group;

(ii) converting a compound of formula (I) into a further compound of formula (I);

(iii) converting a salt of formula (I) into a free compound of formula (I);

(iv) preparing a pharmaceutically acceptable ester or amide of a compound of formula (I);

(v) preparing a pharmaceutically acceptable salt of a compound of formula (I) or an ester or amide thereof.

Suitable compounds of the general formula (IX) are those of formula (IXA), wherein E, E' and L are as defined above, $R^{A}_{1}$ is a moiety convertible to $R^{A}$ and $R^{B}_{1}$ is $R^{B}$; the compounds of formula (IXA) being convertible to a compound of formula (I) by process step (a) as defined above.

Suitable compounds of the general formula (IX) are those of formula (IXB), wherein E, E' and L are as defined above, $R^{A}_{1}$ is a moiety $R^{A}$ and $R^{B}_{1}$ is a moiety convertible to $R^{B}$; the compounds of formula (IXB) being convertible to a compound of formula (I) by process step (b) as defined above.

Suitable compounds of the general formula (IX) are those of formula (IXC), wherein E, E' and L are as defined above, $R^{A}_{1}$ is a moiety convertible to $R^{A}$ and $R^{B}_{1}$ is a moiety convertible to $R^{B}$; the compounds of formula (IX) being convertible to compounds of formula (I) either:

(i) by carrying out process (a) (to prepare a compound (IXB)) and thereafter process (b) to prepare a compound of formula (I); or

(ii) by carrying out process (b) (to prepare a compound of formula (IXA)) and thereafter process (a) to prepare a compound of formula (I).

In a preferred aspect of the preparation of a compound of formula (I) from a compound of formula (IXC), the compound of formula (IXA) or (IXB) is not isolated and is converted *in-situ* to a compound of formula (I).

In a preferred aspect of the conversion of a compound of formula (IXC) to a compound of formula (I), the reaction steps (a) and (b) are carried out simultaneously using the same reagent; thus in a most preferred aspect of the process preferably when $R^{A}_{1}$ = $R^{B}_{1}$, a compound of formula (IXC) is converted to a compound of the hereinbefore defined formulae (II) or (IIA).

In a preferred form of the process of the invention there is provided a process for preparing a compound of formula (I), or a pharmaceutically acceptable salt, ester or amide thereof, which process comprises;

(A) reacting a compound of formula (IX) with a compound of formula (V), wherein in the compound of formula (IX) L is as defined above, $R^{A}_{1}$ is $R^{A}$ or $-T^{1}-Q^{1}-$ and $R^{B}_{1}$ is $R^{B}$ or $T^{1}-Q^{1}-$, providing that at least one of $R^{A}1$ or $R^{B}_{1}$ represents $-T^{1}-Q^{1}-$ and wherein, either:

(i) in $-T^{1}-Q^{1}$, $T^{1}$ is $-(CH_2)_n-Z-$ and $Q^{1}$ is a moiety (e) as defined in relation to formula (VI) and in the compound of formula (V), T is $R^{O}-X$ and Q represents a group of formula (c) or (d); or

(ii) in $T^{1}-Q^{1}$, $T^{1}$ is $(CH_2)_n-Z-$ and $Q^{1}$ is a moiety (e) and in the compound of formula (V), T is $R^{O}-X$ and Q represents a group of formula (f) or (g), and subsequently treating with a reducing agent; or

(iii) in $-T^1-Q^1$, $T^1$ is $(CH_2)_n-Z$ and $Q^1$ represents a group of formula (j) as defined in relation to formula (VI) and in the compound of formula (V), T is $R^o-X$ and Q represents a group of formula (h); or

(iv) in $-T^1-Q^1$, $T^1$ is $(CH_2)_n-Z$ and $Q^1$ represents a group of formula (k) as defined in relation to formula (VI) and in the compound of formula (V), T is $R^o-X$ and Q represents a group of formula (h) and subsequently treating with a reducing agent;

(B) for compounds of formula (I) wherein $R^1$

OH
|
represents only the moiety $R^o-X-CH-CH_2$ as defined above, by reacting a compound of formula (I) wherein $R^1$ represents a hydrogen atom, with either

(i) a compound of formula (VA):

$$T - Q' \qquad (VA)$$

wherein T is as defined above and $Q'$ represents a group of the hereinbefore defined formula (c) or (d); or

(ii) a compound of formula (VB):

$$T - Q''$$

wherein T is as defined above and $Q''$ is a moiety (f) or (g) as defined above and subsequently treating with a reducing agent;

and thereafter if necessary carrying out one or more of the following steps:

(i) removing any protecting group;

(ii) converting a compound of formula (I) into a further compound of formula (I);

(iii) converting a salt of formula (I) into a free compound of formula (I);

(iv) preparing a pharmaceutically acceptable ester or amide of a compound of formula (I);

(v) preparing a pharmaceutically acceptable salt of a compound of formula (I) or an ester or amide thereof.

It will be appreciated that the present process encompasses the preparation of compounds of formula (I) wherein $R^A$ and $R^B$ are the same or different.

Preferably a compound of formula (I) is prepared by reacting a compound of formula (IX) with a compound of formula (V) as defined in (A) (ii) above, especially when Q represents a group of formula (g).

Most preferably $R^{A}_{1}$ and $R^{B}_{1}$ both represent $-T^1-Q^1$ and the compound of formula (IX) is reacted with at least two molar equivalents of the compound of formula (V) thereby providing a compound of formula (II) or (IIA).

The abovementioned reactions between the compounds of formulae (V) and (IX) are carried out under the same conditions as the analogous reactions between compounds of formulae (V) and (VI).

The abovementioned reaction between a compound of formula (I) (wherein $R^1 = H$) and (VA) or (VB) is carried out under the same conditions as the analogous reaction between a compound of formula (III) (wherein $R^1 = H$) and (VA) or (VB).

The conversion of one compound of formula (I) to a further compound of formula (I) includes for example:

(i) converting $R^1$ in a moiety of formula (a), as defined above, from hydrogen to the moiety $R^o-X-\overset{OH}{\overset{|}{C}}HCH_2-$ as defined above, and similarly for $R^{1A}$;

(ii) converting one group L to a further group L.

Suitable methods for converting $R^1$ from hydrogen to $R^0-X-\overset{OH}{\overset{|}{C}}HCH_2$ are as defined above in (B).

Suitable methods for converting one group L to another group L are those wherein a group L comprising a reduceable moiety such as -C=C-, -C≡C-, -CO-, -C=N- or -CO-N(R')- is reduced, using the appropriate conventional conditions and reagents; or where a group L comprising an oxidisable moiety such as -S- is oxidised using the appropriate conventional conditions and reagents.

Examples of preferred conversions are:

$$-OCH_2CO.NH- \xrightarrow{(a)} -OCH_2CH_2NH-$$

$$-CO- \xrightarrow{(b)} -CH(OH)- \xrightarrow{(c)} CH_2-$$

$$-S- \xrightarrow{(d)} -SO_2-$$

(a) = borane-methylsulphide

(b) = lithium aluminium hydride

(c) = catalytic hydrogenolysis

(d) = 3-chloroperbenzoic acid.

The compounds of formula (IX) may be prepared by reacting a compound of formula (X):

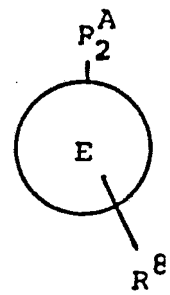

(X)

wherein $R^A{}_2$ is $R^A{}_1$ as defined in relation to formula (IX) or, preferably, a protected form thereof, E is as defined in relation to formula (I) and $R^8$ is as defined in relation to formula (III), with a compound of formula (XI):

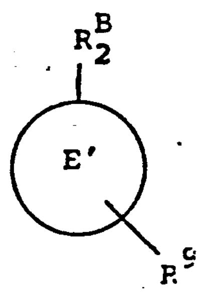

(XI)

wherein $R^B{}_2$ is $R^B{}_1$ as defined in relation to formula (IX) or, preferably, a protected form thereof, E' is as defined in relation to formula (I) and $R^9$ is as defined in relation to formula (IV); and thereafter, if required:

(i) removing any protecting group;

(ii) converting a group L to another group L.

A preferred group $R^{A}_{2}$ in the compound of formula (X) is a group $-T^{1}-Q^{1}$ as defined in relation to formula (VI), or, preferably, a protected form thereof; for example $R^{A}_{2}$ is a group $-T^{1}-Q^{1}$ wherein $Q^{1}$ is a group (e) wherein $R^{p}$ is a protecting group such as benzyl or if $R^{p}$ is $R^{1}$ then $Q^{1}$ is an N-protected form of such a group (e), such as an N-benzylated form. Similarly for $R^{B}_{2}$ in the compound of formula (XI).

A group L may be converted to another group L using methods described above for the conversion of one group L to another group L in the compound of formula (I).

The reaction between the compounds of formula (X) and (XI) may be carried out under any suitable conditions appropriate to the nature of $R^{8}$ and $R^{9}$.

Any protecting groups used in the above reactions are those used conventionally in the art, the said protecting groups being prepared and removed by conventional procedures. For example when $R^{o}$ or $R^{o}_{1}$ represents a phenyl group substituted with a hydroxy group, any conventional hydroxy protecting group may be used. Preferably the hydroxy group being protected by etherification; the ether group being converted into a free hydroxy group by methods known per se. For example, an unsubstituted or substituted benzyloxy protecting group, may be converted by hydrogenolysis into a free hydroxy group.

The hydrogenolysis reaction may be carried out, for example in the presence of a palladium-on-carbon catalyst in a solvent, for example a mixture of ethyl acetate and methanol.

A leaving group $R^x$ is any group that will, under the reaction conditions, cleave from the starting material, thus promoting reaction at a specified site. Suitable examples of such groups are halogen atoms, mesyloxy groups, tosyloxy groups or an alkoxy moiety of an ester group. Preferably in formula (d) of compound (V) or in formula (j) of compound (VI) $R^x$ represents a mesyloxy or tosyloxy group or a bromine atom.

Compounds of formulae (V), (VA), (VI), (VIA), (X) and (XI) are either known compounds or can be prepared from known compounds by known processes or processes analogous to known processes.

As stated above the reaction conditions for the reaction between the compounds of formulae (III) and (IV) or (X) and (XI) will depend largely upon the nature of the nucleophilic moiety and moiety $-L^1-R^x$ (represented by the variables $R^8$ and $R^9$).

Suitably for reacting pairs of compounds (III) and (IV) or (X) and (XI) the reaction between the nucleophilic moiety comprising an anion and the appropriate $-L^1-R^x$ may be carried out in any convenient solvent such as diethylether, tetrahydrofuran or dimethylformamide at a low to elevated temperature; preferred reaction conditions are those set out hereinafter in the appropriate Example.

Suitably for reacting pairs of compounds of formulae (III) and (IV) or (X) and (XI) the reaction between the nucleophilic moiety terminating with an amino group and the appropriate $-L^1-R^x$ may be carried out under conventional peptide forming conditions such as in a $C_{1-6}$ alkanol for example methanol or ethanol at a low

to elevated temperature; preferred conditions are those set out hereinafter in the appropriate Example.

The reaction of compounds of the general formulae (V) and (VI) in which Q and $Q^1$ have formulae (c) and (e) respectively is advantageously carried out in a protic solvent, e.g. an alkanol, especially a lower alkanol having at least 2 carbon atoms, at reflux, preferably in ethanol. The reaction between the compounds of formula (III) (wherein $R^1$ = H) and (VA) (wherein Q′ = (c)) may be carried out under similar conditions.

Reaction of compounds of the general formulae (V) and (VI) in which Q and $Q^1$ have formulae (d) and (e) or (h) and (j) respectively is advantageously carried out in dimethyl sulphoxide, for example at a temperature in the range of from 30 to 80°C, e.g. substantially 50°C, and advantageously for a period of time of 1 to 4 days, e.g. about 3 days. The reaction between the compounds of formula (III) (wherein $R^1$ = H) and (VA) (wherein Q′ = (d)) may be carried out under similar conditions.

The reaction between the compounds of formula (V) and (VI) in which Q and $Q^1$ have formulae (f) and (e) respectively, or between (III) (wherein $R^1$ = H) and (V) (wherein Q = (f)) is preferably carried out in methanol at ambient temperature, the subsequent reduction being carried out, for example, with sodium cyanoborohydride.

The reaction between the compounds of formula (V) and (VI) in which Q and $Q^1$ have formulae (g) or (e)′ respectively, or between (III) (wherein $R^1$ = H) and (V) (wherein Q = (g)) is preferably carried out in the presence of dicyclohexyl carbodimide or other suitable condensing agent in any suitable solvent such as

dimethylformamide at ambient temperature; the subsequent reduction may be carried out with, for example, lithium aluminium hydride or a borane reducing agent, for example borane methyl sulphide complex.

The reductions with sodium cyanoborohydride and sodium borohydride are preferably performed in a lower alkanol, e.g. methanol. The reductions with lithium aluminium hydride or a borane methyl sulphide complex are preferably carried out in diethylether or tetrohydrofuran.

The salts of compounds of the general formula (I) may be produced by methods conventional in the art, for example, acid addition salts may be prepared by treating a compound of general formula (I) or an ester or amide with the appropriate acid . (It will be appreciated from the foregoing that in the case of acid addition salts, the relevant salt may be found from a compound of formula (I) or an ester and/or amide thereof.)

Compounds of the general formula (I) and pharmaceutically acceptable salts, esters or amides thereof, produced by the above processes, may be recovered by conventional methods.

If required compounds of the general formula (I) may be separated into diastereoisomeric pairs of enantiomers by, for example, fractional crystallisation from a suitable solvent, for example methanol or ethyl acetate or a mixture thereof. The enantiomers thus obtained may be separated into individual stereoisomers by conventional means, for example by the use of an optically active acid as a resolving agent. Suitable

optically active acids which maybe used as resolving agents are described in 'Topics in Stereochemistry', Vol. 6, Wiley Interscience, 1971, Allinger, N.L. and Eliel, W.L. Eds.

Alternatively, any enantiomer of a compound of the general formula (I) or a pharmaceutically acceptable salt, ester or amide thereof may be obtained by conventional stereospecific synthesis using optically pure starting materials of known configuration.

The esters and amides of the compounds of formula (I) may be prepared by conventional methods. For example esters may be prepared by treatment of the appropriate acid with an appropriate alcohol suitably in the presence of an acidic catalyst. An amide may be prepared by treating the appropriate acid or acid derivative with the appropriate amine.

As previously indicated, the compounds of the present invention have valuable pharmacological properties.

The present invention also provides a compound of the general formula (I), or a pharmaceutically acceptable salt, ester or amides thereof, for use as an active therapeutic substance.

In one aspect, the present invention provides a compound of the general formula (I), or a pharmaceutically acceptable salt, ester or amide thereof, for use in the treatment of obesity in human or non-human animals. The aforementioned use also encompasses the treatment of obesity for cosmetic purposes where appropriate.

The present invention further provides a compound of the general formula (I), or pharmaceutically acceptable salt, ester or amide thereof, for use in the treatment of hyperglycaemia in human or non-human animals.

The present invention further provides a compound of formula (I), or a pharmaceutically acceptable salt, ester or amide thereof, for use in the treatment and/or prophylaxis of atheroscleroris in humans.

The present invention also encompasses the use of a compound of formula (I), or a pharmaceutically acceptable salt, ester or amide thereof, for the manufacture of a medicament for the treatment of obesity in human or non-human animals.

The invention further provides the use of a compound of formula (I), or a pharmaceutically acceptable salt, ester or amide thereof for the manufacture of a medicament for the treatment of hyperglycaemia in humans or non-human animals.

The invention further extends to the use of a compound of formula (I), or a pharmaceutically acceptable salt, ester or amide thereof, for the manufacture of a medicament for the treatment of atherosclerosis in humans.

A compound of the general formula (I), or a pharmaceutically acceptable salt, esters or amides hereof, may be administered <u>per se</u> or, preferably, as a pharmaceutical composition also comprising a pharmaceutically acceptable carrier.

Suitable non-human animals are non-human mammals, especially domestic animals such as dogs or cats.

Accordingly, the present invention also provides a pharmaceutical composition comprising a compound of the general formula (I), or a pharmaceutically acceptable salt, ester or amide thereof, and a pharmaceutically acceptable carrier therefor.

As used herein the term ''pharmaceutically acceptable'' embraces compounds, compositions and ingredients for both human and veterinary use: for example the term ''pharmaceutically acceptable salt'' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

The present invention further provides a method for treating obesity in a human or non-human animal, which comprises administering an effective, non-toxic, amount of a compound of the general formula (I), or a pharmaceutically acceptable salt, ester or amide thereof, to an obese human or non-human animal.

The present invention further provides a method for treating hyperglycaemia in a human or non-human animal which comprises administering an effective, non-toxic, amount of a compound of the general formula (I), or a pharmaceutically acceptable salt, ester or amide thereof, to a hyperglycaemic human or non-human animal.

The present invention further provides a method for treating atherosclerosis by increasing high-density lipoprotein (HDL) cholesterol concentration and/or decreasing triglyceride concentration in human blood serum, which method comprises the administration of an effective non-toxic amount of a compound of formula (I) or a pharmaceutically acceptable salt, ester or amide thereof to a human in need thereof.

Conveniently, the active ingredient may be administered as a pharmaceutical composition hereinbefore defined, and this forms a particular aspect of the present invention.

In treating hyperglycaemic or obese humans the compound of the general formula (I), or a pharmaceutically

acceptable salt, ester or amide thereof, may be taken in doses, such as those described above, one to six times a day in a manner such that the total daily dose for a 70 kg adult will generally be in the range of from 0.1 to 6000 mg, and more usually about 1 to 1500 mg.

In treating hyperglycaemic or obese non-human animals, especially dogs, the active ingredient may be adminstered by mouth, usually once or twice a day and in an amount in the range of from about 0.025 mg/kg to 25 mg/kg, for example 0.1 mg/kg to 20 mg/kg.

In treating atherosclerosis in humans the compound of the general formula (I), or a pharmaceutically acceptable salt, ester or amide thereof, may be taken in doses, such as those described above, one to six times a day in a manner such that the total daily dose for a 70 kg adult will generally be in the range of from 0.1 to 6000 mg, and more usually about 1 to 1500 mg.

In a further aspect the present invention also provides a method for increasing weight gain and/or improving the feed utilisation efficiency and/or increasing lean body mass and/or decreasing birth mortality rate and increasing post-natal survival rate; of livestock, which method comprises the administration to livestock of an effective non-toxic amount of a compound of formula (I) or a veterinarily acceptable salt, ester or amide thereof.

Whilst the compounds of formula (I) and the veterinarily acceptable salts, esters or amides thereof; may be administered to any livestock in the

abovementioned method, they are particularly suitable for increasing weight gain and/or feed utilisation efficiency and/or lean body mass and/or decreasing birth mortality rate and increasing post-natal survival rate; in poultry, especially turkeys and chickens, cattle, pigs and sheep.

In the preceding method the compounds of formula (I) or veterinarily acceptable salts, esters or amides thereof will normally be administered orally although non-oral modes of administration, for example injection or implantation, are also envisaged. Suitably the compounds are administered in the feed-stuff or drinking water provided for the livestock. Conveniently these are administered in the feed-stuff at from $10^{-3}$ ppm - 500ppm of total daily fed intake, more usually 0.01ppm to 250ppm, suitably less than 100ppm.

The particular formulations used will of course depend upon the mode of administration but will be those used conventionally in the mode of administration chosen.

For administration in feed-stuff the drugs are conveniently formulated as a premix in association with a suitable carrier.

Accordingly, the present invention also provides a veterinarily acceptable premix formulation comprising a compound of formula (I), or a veterinarily acceptable salt, ester or amide thereof and a veterinarily acceptable carrier therefore.

Suitable carriers are inert conventional agents such as powdered starch. Other conventional feed-stuff premix carriers may also be employed.

No toxicological effects are indicated when a compound of formula (I) or a pharmaceutically acceptable salt, ester or amide thereof is administered in any of the abovementioned dosage ranges.

The following Examples illustrate the invention but do not limit it in any way.

Example 1

[R,R,R,R]-N,N'-(1,2-Ethanediyl)bis[2-[4-[2-[(2-(3-chlorophenyl)-2-hydroxyethyl)amino]propyl]phenoxy]acetamide]

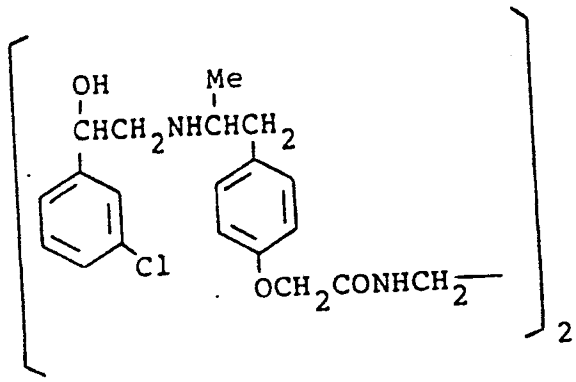

Ethylene diamine (0,053g, 0.89mmol) was added to a stirred solution of [R,R,R,R]-ethyl 2-[4-[2-[2-(3-chlorophenyl)-2-hydroxyethyl)amino]propyl]phenoxy]-acetate (0.7g, 1.79mmol) in ethanol (10ml). The solution was heated under reflux for 4 days, cooled, evaporated to dryness, and the residue chromatographed on silica using chloroform/methanol (95:5) as eluent. When unreacted starting material had eluted, the eluent was changed to chloroform/methanol (88:12) to give (R,R,R,R)-N,N'-(1,2-ethanediyl)bis[2-[4-[2-[(2-(3-chlorophenyl)-2-hydroxyethyl)amino]propyl]phenoxy]acetamide] (0.3g) as an oil, which was crystallised from methanol, m.p. 124-6°.

$^1H$ nmr ($d_6$-DMSO)ppm

0.88 (6H,d), 1.65 (2H,broad, exchanges with $D_2O$), 2.3 (2H,m), 2.7 (8H,m), 3.5 (4H,s), 4.4 (4H,s), 4.56 (2H,m), 5.31 (2H, broad, exchanges with $D_2O$), 6.83 (4H,d), 7.05 (4H,d), 7.25-7.32 (8H,m) 8.10 (2H, broad, exchanges slowly with $D_2O$).

Example 2

[R,R,R,R]-α,α'[1,2-Ethanediylbis(imino-2,1-ethanediyloxy-4,1-phenylene (1-methyl-2,1-ethanediyl) iminomethylene]]bis[3-chlorobenzenemethanol], tetrahydrochloride.

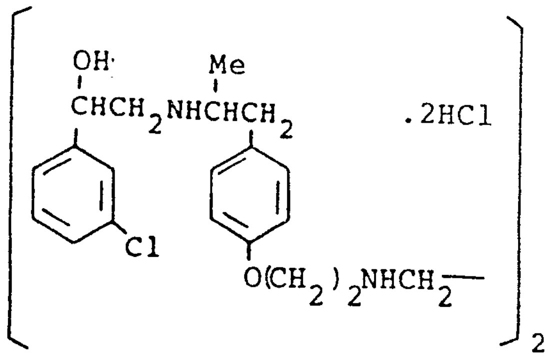

To a solution of [R,R,R,R]-N,N'-[1,2-ethanediylbis [imino(2-oxo-2,1-ethanediyl)oxy-4,1-phenylene (1-methyl-2,1-ethanediyl)]]bis[3-chloro-α-hydroxybenzeneacetamide (0.4g, 0.5mmol) in dry THF (25ml), was added dropwise, borane-methyl sulphide (2ml, 20mmol) as a neat liquid. The reaction mixture was stirred and heated at 80°C under a nitrogen atmosphere for 64h during which time a colourless solution was formed. Methanol was added dropwise to destroy excess borane-methyl sulphide and then hydrogen chloride was bubbled through the reaction mixture until the solution was acidic. The solvent was evaporated, the residue dissolved in aqueous potassium carbonate solution and extracted with ethyl acetate. The organic layer was washed with water, dried ($MgSO_4$) and evaporated to leave a colourless oil. This was chromatographed on silica using chloroform/methanol/ammonia (89.5:10: 0.5) as eluent to give a colourless oil (0.2g) which was converted to [R,R,R,R]-α,α'[1,2-ethanediylbis (imino-2,1- ethanediyloxy-4,1-phenylene (1-methyl-2,1-ethanediyl)iminomethylene]]bis

[3-chlorobenzenemethanol], tetrahydrochloride m.p. 246-251° (methanol-ethylacetate).

1H nmr ($d_6$-DMSO)ppm:
1.13 (6H,d), 2.63 (2H,dd), 3.0-3.5(18H,m), 4.28 (4H,m), 5.09 (2H,bd), 6.35 (2H,bs, replaceable with $D_2O$), 6.99 (4H,d), 7.19 (4H,d), 7.35-7.47 (8H,m), 8.83 (2H,bs, replaceable by $D_2O$), 9.41 (2H, bs, replaceable by $D_2O$), 9.75 (4H, bs, replaceable by $D_2O$).

Example 3

[R,R,R,R]-α,α'[1,6-Hexanediylbis[oxy-4,1-phenylene(1-methyl-2,1-ethanediyl)iminomethylene]]bis[3-chlorobenzene methanol], dihydrochloride.

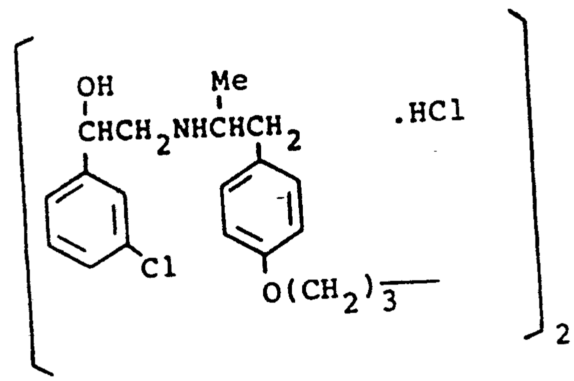

To a solution of [R,R,R,R]-N,N'-[1,6-hexanediylbis [oxy-4,1-phenylene(1-methyl-2,1-ethanediyl)]] bis[3-chloro-α-hydroxybenzeneacetamide] (2.45g) in dry tetrahydrofuran (30ml) was added dropwise a solution of borane-methyl sulphide (6.8ml, 6.8mmols) in dry tetrahydrofuran (10ml). After 3 hours at reflux, the solution was cooled to ambient temperature. Methanol was added dropwise to destroy excess borane methyl sulphide followed by a solution of hydrogen chloride gas in methanol until the solution was acidic. The solvent was evaporated *in vacuo* to leave a white crystalline residue which was recrystallised from

ethanol to give[R,R,R,R]-α,α'[1,6-hexanediylbis [oxy-4,1-phenylene(1-methyl-2,1-ethanediyl) iminomethylene]]bis[3-chlorobenzenemethanol], dihydrochloride salt. m.p. 203-208°C
$[\alpha]_D^{25}$: -44.3° (C 0.56; MeOH)

'H nmr, $d_6$-DMSO ppm:
1.1(6H,d), 1.46(4H,m), 1.72(4H,m), 2.6(2H,dd), 3.0-3.5(8H,m), 3.95(4H,m), 5.1(2H,dd), 6.3(2H,bs, disappears with $D_2O$), 6.87(4H,d), 7.37(4H,d), 7.39-7.51(8H,m), 8.7-9.7(4H,broad,disappears on $D_2O$).

Example 4

[R,R,R,R]-α,α′-[Oxybis[4,1-phenylene(1-methyl-2,1-ethanediyl)iminomethylene]]bis[3-chlorobenzene-methanol],dihydrochloride.

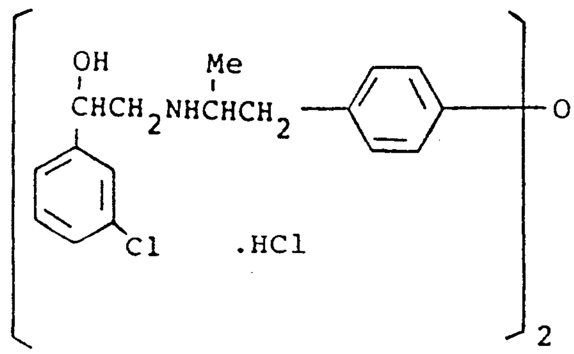

[R,R,R,R]-α,α′-[Oxybis[4,1-phenylene(1-methyl-2,1-ethanediyl)iminomethylene]]bis[3-chlorobenzenemethanol], dihydrochloride, m.p. 248-50° (ethyl acetate-methanol) was obtained from [R,R,R,R]-N,N′-[4,4′-oxybis[4,1,-phenylene(1-methyl-2,1-ethanediyl)]] bis[3-chloro-α-hydroxybenzeneacetamide], (2.8g) by an analogous procedure to that described in Example 3.
$[\alpha]_D^{25}$ :-33.8° (EtOH).

1H-nmr ($d_6$-DMSO), ppm:
1.14(6H,d), 2.68(2H,dd), 2.9-3.6 (8H,m), 5.12 (2H,bd), 6.37 (2H, bd, exchanges with $D_2O$), 6.95 (4H,d), 7.26 (4H,d), 7.3-7.6 (8H,m), 8.7-9.1 (2H, bs, exchanges with $D_2O$), 9.3-9.7 (2H, bs, exchanges with $D_2O$).

## Example 5

[R,R,R,R]-α,α'-[Methylenebis[4,1-phenylene(1-methyl-2,1-ethanediyl)iminomethylene]]bis[3-chlorobenzene-methanol]dihydrochloride

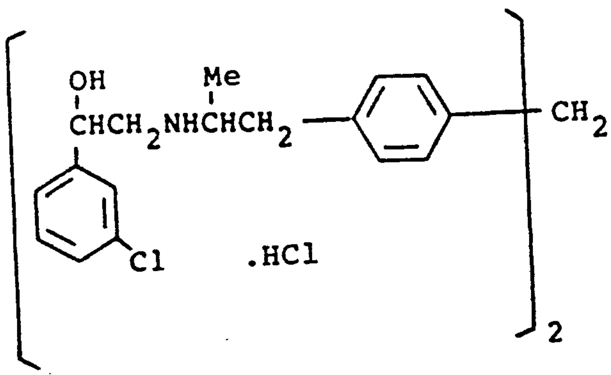

[R,R,R,R]-α,α'-[methylenebis[4,1-phenylene(1-methyl-2,1-ethanediyl)iminomethylene]]bis[3-chlorobenzenemethanol]dihydrochloride m.p. 262-64° (ethyl acetate-methanol) was obtained from [R,R,R,R]-N,N'-[methylenebis[4,1-phenylene(1-methyl-2,1-ethanediyl]] bis[3-chloro-α-hydroxybenzeneacetamide] (1.9g) by an analogous procedure to that described in Example 3. $[\alpha]_D^{25}$: -37.4° (EtOH).

1H-NMR ($d_6$-DMSO), ppm.

1.09 (6H,d); 2.62 2H,dd); 3.0-3.5 (8H,m); 3.89 (2H,s); 5.10 (2H,bd); 6.37 (2H,d, exchanges with $D_2O$); 7.17 (8H,d), 7.3-7.5 (8H,m) 8.7-9.0 (2H, bs exchanges with $D_2O$), 9.3-9.6 (2H, bs, exchanges with $D_2O$).

Example 6

[R,R,R,R]-α,α'-[Sulphonylbis[4,1-phenylene(1-methyl-2,1-ethanediyl)iminomethylene]]bis[3-chlorobenzene-methanol] dihydrochloride

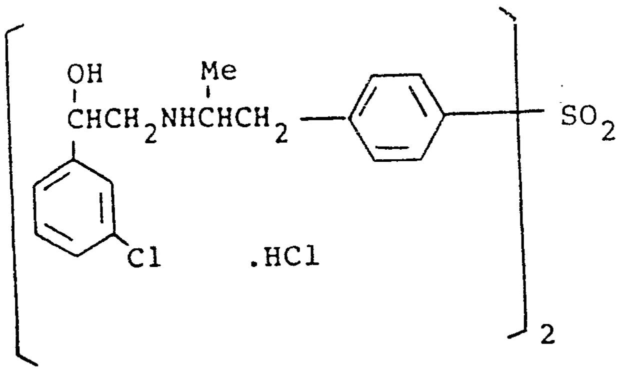

[R,R,R,R]-α,α'-[Sulphonylbis[4,1-phenylene(1-methyl-2,1-ethanediyl)iminomethylene]]bis[3-chlorobenzenemethanol] dihydrochloride m.p. 255-58° (ethyl acetate-methanol) was obtained from [R,R,R,R]-N,N'-[sulphonylbis[4,1-phenylene(1-methyl-2,1-ethanediyl]] bis[3-chloro-α-hydroxybenzeneacetamide] (9.0g) by an analogous procedure to that described in Example 3. $[\alpha]_D^{25}$: -25.8° (EtOH).

1H-nmr ($d_6$-DMSO), ppm

1.11 (6H,d); 2.83 (2H,dd); 3.0-3.3 (4H,m); 3.3-3.4 (4H,m); 5.15 (2H,d), 6.43 (2H, d, exchanges with $D_2O$); 7.3-7.6 (12H,m); 7.94 (4H,d); 8.9-9.2 (2H, bs, exchanges with $D_2O$); 9.7-10.0 (2H, bs, exchanges with $D_2O$).

Example 7

[R,R,R,R]-1,1-di[4-[2-[[2-(3-chlorophenyl)-2-hydroxyethyl]amino]propyl]phenyl]-1,2-ethanediol, dihydrochloride.

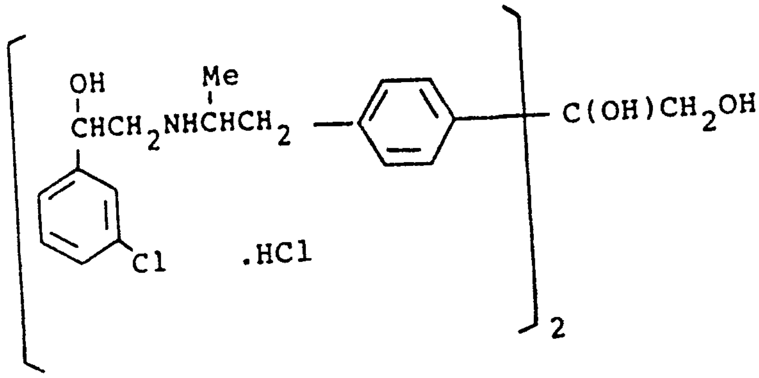

[R,R,R,R]-1,1-di[4-[2-[[2-(3-chlorophenyl)-2-hydroxyethyl]amino]propyl]phenyl]-1,2-ethanediol, dihydrochloride, m.p. 123-26° was obtained from [R,R,R,R]-ethyldi[4-[2-[2-(3-chlorophenyl)-2-hydroxy-1-oxoethyl)amino]propyl]phenyl]-α-hydroxyacetate (2.0g) by an analogous procedure that described for Example 3.

1H-nmr ($d_6$-DMSO), ppm.

1.09 (6H,d); 2.61 (2H,dd); 3.0-3.3 (6H,m); 3.3-3.5 (2H,m); 3.92 (2H,bd); 4.0 (1H,m); 4.79 (1H,bs, exchanges with $D_2O$); 5.06 (2H,bd); 6.35 (2H,d); 7.15 (4H,d); 7.23 (4H,d); 7.3-7.5 (8H,m); 8.7-9.0 (2H,bs, exchanges with $D_2O$); 9.4-9.6 (2H, bs, exchanges with $D_2O$).

Example 8

[R,R,R,R]-α,α'-[1,8-Octanediylbis[oxy-4,1-phenylene (1-methyl-2,1-ethanediyl)iminomethylene]]bis[3-chloro-benzenemethanol], dihydrochloride.

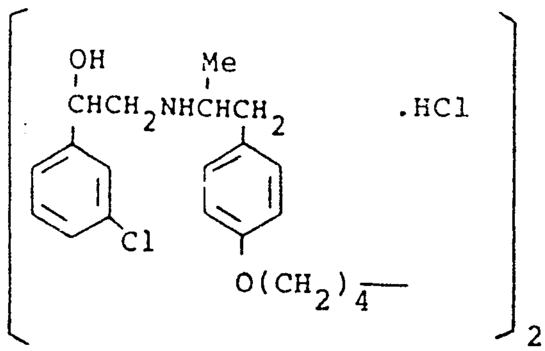

[R,R,R,R]-α,α'-[1,8-Octanediylbis[oxy-4,1-phenylene (1-methyl-2,1-ethanediyl)iminomethylene]]bis[3-chlorobenzenemethanol], dihydrochloride, m.p. 189-195 was prepared from [R,R,R,R]-N,N'-[(1,8-octanediyl)bis-[oxy-4,1-phenylene(1-methyl-2,1-ethanediyl)]]bis [3-chloro-α-hydroxybenzeneocetamide] by an analogous procedure to that described in Example 3.
$[\alpha]_D^{25}$: -37.6° (C. 0.5, MeOH)

1H nmr, ($D_6$-DMSO, ppm

1.10 (6H,d); 1.39 (8H,m); 1.70 (4H,m); 2.59 (2H,m), 3.00-3.40 (8H,m); 3.93 (4H,m); 5.08 (2H,dd); 6.35 (2H, bs, disappears with $D_2O$); 6.87 (4H,d); 7.14 (4H,d); 7.35-7.50 (8H,m); 8.85 and 9.30 (4H, broad, disappears with $D_2O$).

Example 9

1,2-Ethanediyl di[4-[2-[[2-hydroxy-2-(3-trifluoromethyl)phenylethyl]amino]propyl]benzoate]

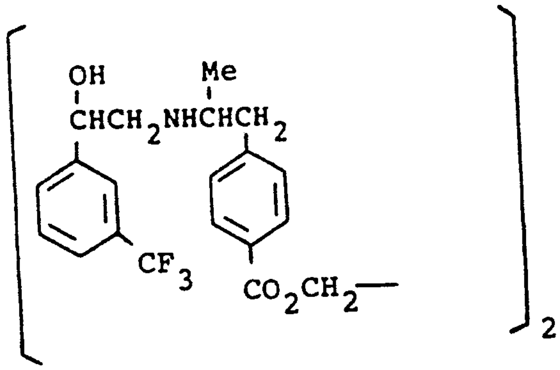

(E9

A mixture of (R*,R*)-(±)-4-[2-[(2-hydroxy-2-(3-trifluoromethyl)phenylethyl)amino]propyl]benzoic acid (0.734g), 1,2-dibromoethane (0.188g) and 1,8-diazabicyclo[5.4.0]undec-7-ene (0.304g) was stirred in dimethylformamide (5ml) for 16 h at ambient temperature. The solvent was removed and the residue was partitioned between water and ethyl acetate. The organic layer was separated, washed sequentially with 2N sodium hydroxide solution and water, dried and evaporated to give 1,2-ethanediyl di [4-[2-[[2-hydroxy-2-(3-trifluoromethyl)phenylethyl] amino]propyl]benzoate]as a white solid (0.2g) m.p. 115-120° (ethyl acetate-diisopropylether).

$^1H$ nmr ($CDCl_3$), ppm

0.90 (6H,d); 2.49-2.56 (4H,m); 2.65-2.77 (4H,m); 2.81-2.88 (2H,m); 3.3 (2H, broad, disappears with $D_2O$); 4.61 (4H,s); 4.65 (2H,t); 5.4 (2H, broad, disappears with $D_2O$), 7.26 (4H,d); 7.45-7.64 (8H,m); 7.83 (4H,d).

Example 10

1,2-Ethanediyl di[4-[2-[[2-hydroxy-2-phenylethyl]amino] propyl] benzoate]

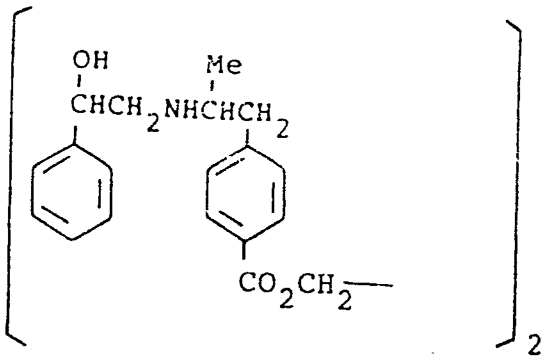

1,2-Ethanediyl di[4-[2-[[2-hydroxy-2-phenylethyl]amino] propyl] benzoate] m.p. 133-5° (ethylacetate-diisopropylether) was prepared from (R*,R*)-(±)-4-[2-[(2-hydroxy-2-phenylethyl)amino]propyl]benzoic acid in an analogous manner to the compound described in Example 9.

$^1H$ nmr ($CDCl_3$), ppm

0.91 (6H,d); 2.5-2.68 (4H,m); 2.71-2.78(4H,m): 2.82-2.89 (2H,m); 3.31 (2H, broad, disappears with $D_2O$); 4.54 (2H,t); 4.61 (4H,s); 5.2 (2H, broad, disappears with $D_2O$); 7.18-7.29 (14H,m); 7.85 (4H,d).

Example 11

[R,R,R,R,]-α,α'[1,4-Butanediylbis[oxy-4,1-phenylene (1-methyl-2,1-ethanediyl)iminomethylene]]bis[3-chlorobenzenemethanol], dihydrochloride.

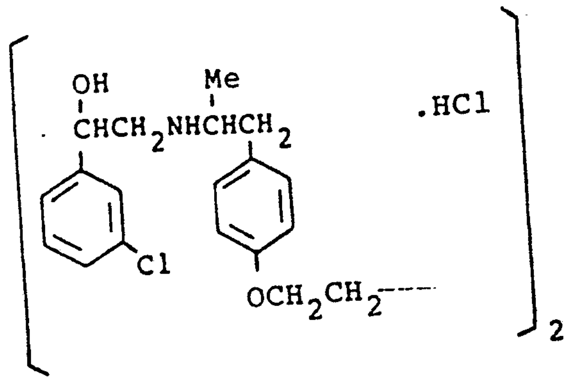

(E11)

[R,R,R,R,]-α,α'[1,4-Butanediylbis[oxy-4,1-phenylene (1-methyl-2,1-ethanediyl)iminomethylene]]bis[3-chlorobenzenemethanol], dihydrochloride, m.p. 206-211° was prepared using [R,R,R,R]-N,N'-[1,4 butanediyl bis [oxy-4,1-phenylene(1-methyl-2,1-ethanediyl)]]bis[3-chloro-α-hydroxybenzeneacetamide](1.8g) by an analogous procedure to that described in Example 3.

$[\alpha]_D^{25}$ : (MeOH) = -46.9°

$^1H$ nmr, (DMSO-$d_6$) ppm

1.1(6H,d); 1.8(4H,broad s); 2.6(2H,t); 3.0-3.5(8H,m), 4.0(4H,broad s); 5.1(2H,m); 6.4(2H,m-exch $D_2O$); 6.9 (4H,d); 7.15(4H,d); 7.3-7.5(8H,m); 8.6-9.25(2H, very broad exch. $D_2O$); 9.25-10.0(2H, very broad exch. $D_2O$).

Example 12

11,R,R,R,]-α,α'[1,3-Propanediylbis[oxy-4,1-phenylene (1-methyl-2,1-ethanediyl)iminomethylene]]bis[3-chlorobenzenemethanol], dihydrochloride.

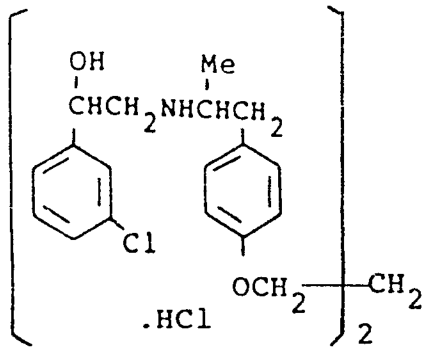

...2)

[R,R,R,R,]-α,α'[1,3-Propanediylbis[oxy-4,1-phenylene (1-methyl-2,1-ethanediyl)iminomethylene]]bis[3-chlorobenzenemethanol], dihydrochloride m.p. 189-193°
was prepared using [R,R,R,R,)-N,N'-[1,3-propanediyl bis[oxy-4,1-phenylene (1-methyl-2,1-ethanediyl)]]bis[3-chloro-α-hydroxybenzeneacetamide] (1.8g) by an analogous procedure to that described in Example 3.

$[\alpha]_D^{25}$ : (MeOH) = -43.1°

$^1H$ nmr, (DMSO-$d_6$) ppm

1.1(6H,d); 2.1(2H,m); 2.6(2H,t); 3.0-3.5(8H,m); 4.1(4H,m); 5.1(2H,m); 6.4(2H,m,exch $D_2O$); 6.9(4H,d); 7.2(4H,d); 7.3-7.6(8H,m); 8.5-9.25(2H, very broad exch. $D_2O$); 9.25-10.0(2H, very broad exch. $D_2O$).

Example 13

[R,R,R,R,]-α,α'[1,9-Nonanediylbis[oxy-4,1-phenylene (1-methyl-2,1-ethanediyl)iminomethylene]]bis[3-chlorobenzenemethanol], dihydrochloride.

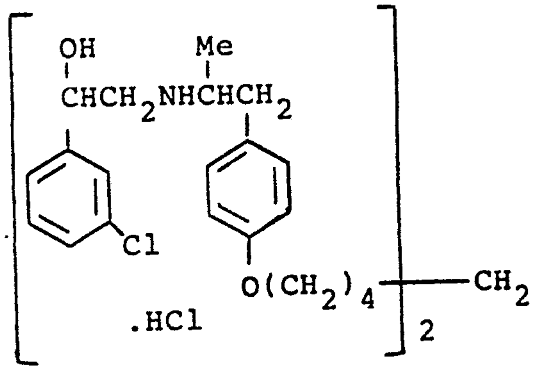

[R,R,R,R,]-α,α'[1,9-Nonanediylbis[oxy-4,1-phenylene (1-methyl-2,1-ethanediyl)iminomethylene]]bis [3-chlorobenzenemethol], dihydrochloride, m.p. 181-187$^{o}$ was prepared in an analogous manner to that described in Example 3.

$^{1}$H nmr, ($d_6$-DMSO) ppm

1.1(6H,d); 1.3-1.5(10H,m); 1.65-1.8(4H,m); 2.5-2.7(2H,m); 3.0-3.5(8H,m); 3.92(4H,t); 5.0(2H,dd) 6.32(2H,broad,disappears with $D_2O$); 6.88(4H,d); 7.12(4H,d); 7.3-7.5(8H,m), 8.5-9.4.(4H,broad, disappears with $D_2O$).

Example 14

[R,R,R,R,]-3-Chloro-α-[[[[2-[4-[6-[4-[2-[2-(4-hydroxyphenyl)-2-hydroxyethyl]amino]propyl]phenoxy]hexyloxy]phenyl]-1-methylethyl]amino]methyl]-benzenemethanol.

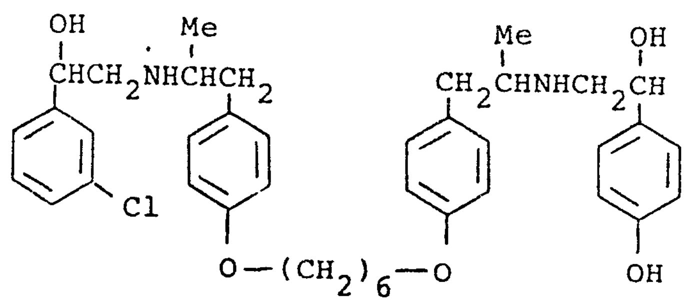

[R,R,R,R,]-α-[[[[2-[4-[6-[4-[2-[2-(4-Benzyloxyphenyl)-2-hydroxyethyl]amino]propyl]phenoxy]hexyloxy]phenyl]-1-methylethyl]-amino]methyl]-3-chlorobenzenemethanol (680mg) was dissolved in glacial acetic acid (10ml) and treated with 10% Pd-C (100mg). The mixture was shaken under a hydrogen atmosphere at ambient temperature and pressure until hydrogen uptake ceased. The catalyst was filtered off and the filtrate evaporated to dryness. The residue was chromatographed on silica using chloroform/methanol/ammonia (94:5:1) as eluent to give [R,R,R,R,]-3-Chloro-α-[[[[2-[4-[6-[4-[2-[2-(4-hydroxyphenyl)-2-hydroxyethyl]amino]propyl]phenoxy]hexyloxy] phenyl]-1-methylethyl]amino]methyl]benzene-methanol as a foam, m.p. 55-66°

$^1H$ nmr, ($d_6$-DMSO), ppm

0.90(3H,d); 0.91(3H,d); 1.46(4H,m); 1.71(4H,m), 2.3-2.51(2H,m); 2.6-3.0(8H,m); 3.3(2H,broad, replaceable by $D_2O$), 3.92(4H,t), 4.51(1H,dd), 4.59(1H,dd), 5.0-5.6(2H,broad, replaceable by $D_2O$); 6.65-7.5(16H,m); 9.25(1H,broad, replaceable by $D_2O$).

Example 15

[R,R,R,R,]-α,α'-[1,2-Ethanedlylbis[iminomethylene-4,1-phenylene(1-methyl-2,1-ethanediyl)iminomethylene]]bis[3-chlorobenzenemethanol, tetrahydrochloride.

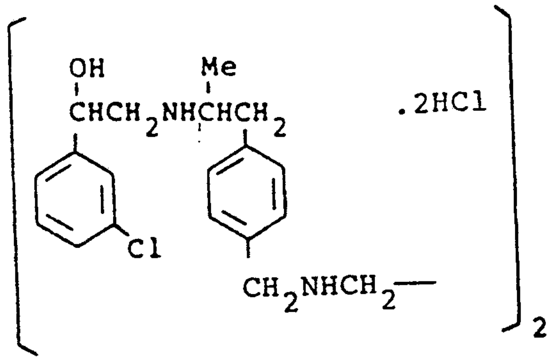

[R,R,R,R,]-N,N'-[1,2-Ethanediylbis[iminocarbonyl-4,1-phenylene(1-methyl-2,1-ethanediyl)]]bis[3-chloro-α-hydroxybenzeneacetamide] (0.71g, 0.99 mmol) was suspended in dioxan (45ml) and borane-dimethylsulphide complex (75 equivalents) added slowly with stirring. After addition was complete the solution was heated to reflux for 24 h, cooled and the excess borane destroyed with methanol. The resulting solution was concentrated and then dissolved in methanol through which hydrogen chloride was passed for 5 minutes. [R,R,R,R,]-α,α'-[1,2-Ethanediylbis-[iminomethylene-4,1-phenylene(1-methyl-2,1-ethanediyl)iminomethylene]]bis[3-chlorobenzenemethanol, tetrahydrochloride, 0.23g, was isolated by filtration.

$[\alpha]_D^{25}$ : -26.1° (DMSO)

$^1H$ nmr, ($d_6$-DMSO), ppm

1.1(6H,d); 2.7(2H,br t); 2.9-3.55(12H,m); 4.2(4H,s), 5.15(2H,d); 6.4(2H,br s); 7.2-7.6(16H,m); 8.6-10.2(8H,br m).

Example 16

[R,R,R,R]-α,-α'-[1,4-Butanediylbis[iminomethylene-4,1-phenylene(1-methyl-2,1-ethanediyl)iminomethylene]]bis[3-chlorobenzenemethanol, tetrahydrochloride.

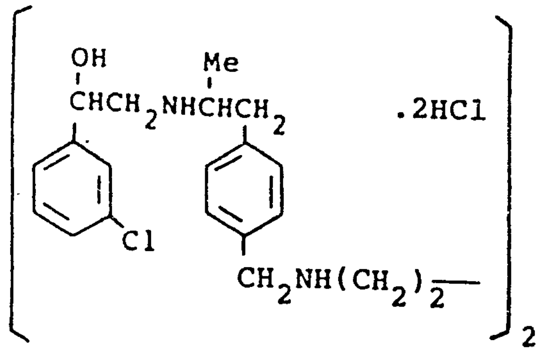

[R,R,R,R]-α,-α'-[1,4-Butanediylbis[iminomethylene-4,1-phenylene(1-methyl-2,1-ethanediyl)iminomethylene]]bis[3-chlorobenzenemethanol, tetrahydrochloride was prepared in an analogous manner to the compound described in Example 15.

$[\alpha]_D^{25}$: -26.5° (DMSO).

$^1$H nmr ($d_6$-DMSO), ppm

1.1(6H,d); 1.7 (4H,br,s); 2.4-3.5 (14H,m); 4.05 (4H,s); 5.05 (2H,br d); 6.3 (2H,br, s); 7.25-7.6 (16H,m); 8.5-9.6 (8H,br).

Example 17

[R,R,R,R]-α,α'-[1,6-Hexanediylbis[iminomethylene-4,1-phenylene (1-methyl-2,1-ethanediyl)iminomethylene]] bis[3-chlorobenzenemethanol], tetrahydrochloride.

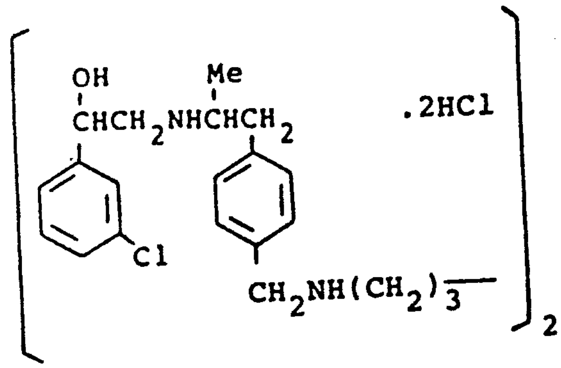

[R,R,R,R]-α,α'-[1,6-Hexanediylbis[iminomethylene-4,1-phenylene (1-methyl-2,1-ethanediyl)iminomethylene]] bis[3-chlorobenzenemethanol], tetrahydrochloride was prepared in an analogous manner to the compound described in Example 15. $[\alpha]_D^{25}$: -27.4° (DMSO).

$^1H$ nmr ($d_6$-DMSO), ppm

1.15(6H,d); 1.25(4H,br s); 1.7(4H,br s); 2.6-3.6(14H,m); 4.1(4H,s); 5.2(2H,d); 6.4(2H,d); 7.25-7.65(16H,m); 8.75-10.0(8H,br).

Example 18

[R,R,R,R]-α,α'-[1,2-Ethanediylbis[4,1-phenylene (1-methyl-2,1-ethanediyl)iminomethylene]] bis[3-chlorobenzenemethanol], dihydrochloride.

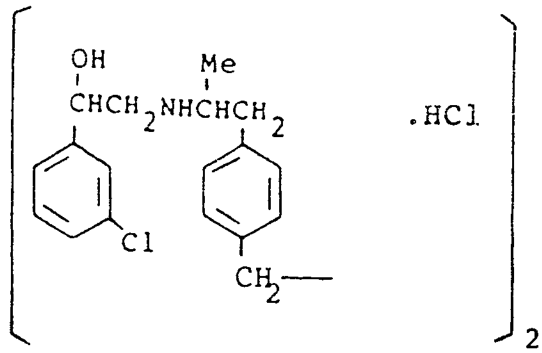

[R,R,R,R]-α,α'-[1,2-Ethanediylbis[4,1-phenylene (1-methyl-2,1-ethanediyl)iminomethylene]] bis[3-chlorobenzenemethanol], dihydrochloride, m.p. 233-234°, was prepared in an analogous manner to the compound described in Example 15, except that tetrahydrofuran replaced dioxan as solvent.

$[\alpha]_D^{25}$: -31.9° (DMSO).

$^1H$ nmr ($d_6$-DMSO), ppm

1.05(6H,d); 2.6(2H,m); 2.75(4H,s); 3.0-3.5(8H,m), 5.1(2H,m); 6.4(2H,d); 7.05-7.25(8H,m); 7.25-7.55(8H,m); 8.8(2H,br s); 9.6(2H,br s).

Example 19

[R,R,R,R]-α,α'-[1,6-Hexanediylbis[4,1-phenylene (1-methyl-2,1-ethanediyl)iminomethylene]] bis[3-chlorobenzenemethanol], dihydrochloride.

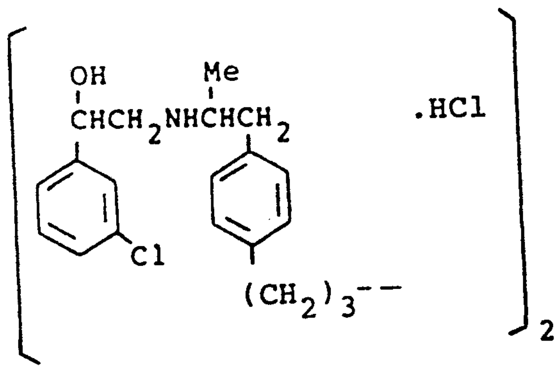

[R,R,R,R]-α,α'-[1,6-Hexanediylbis[4,1-phenylene (1-methyl-2,1-ethanediyl)iminomethylene]] bis[3-chlorobenzenemethanol], dihydrochloride was prepared in an analogous manner to the compound described in Example 15, except that tetrahydrofuran replaced dioxan as solvent.

$[\alpha]_D^{25}$: -27.2° (DMSO).

$^1H$ nmr ($CDCl_3$), ppm

1.3(10H,m); 1.55(4H,br s); 2.55(4H,t); 2.8(2H,m), 3.2(4H,m); 3.45(4H,m); 5.45(2H,d); 6.0(2H,d); 7.0-7.5(16H,m); 9.1(2H,br s); 10.0(2H,br s).

Example 20

[R,R,R,R]-α,α'-[1,5-Pentanediyl bis[oxy-4,1-phenylene (1-methyl-2,1-ethanediyl)iminomethylene]] bis[3-chlorobenzenemethanol], dihydrochloride.

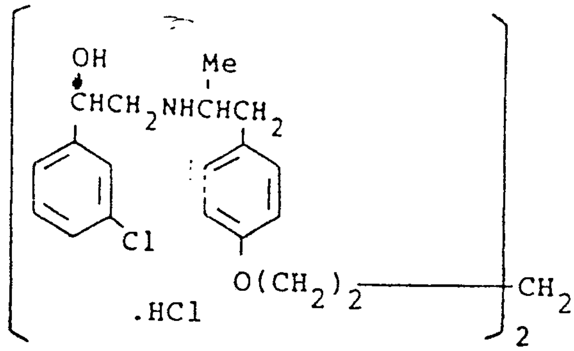

(E20)

[R,R,R,R]-α,α'-[1,5-Pentanediyl bis[oxy-4,1-phenylene (1-methyl-2,1-ethanediyl)iminomethylene]] bis[3-chlorobenzenemethanol], dihydrochloride was prepared in an analogous manner to the compound described in Example 15.

$[\alpha]_D^{25}$: -29.1° (DMSO).

$^1H$ nmr ($d_6$-DMSO), ppm

1.15(6H,d); 1.3-2.0(6H,m); 1.7(2H,br.d); 3.0-3.7(8H,m); 4.0(6H,br,t); 5.2(2H,m); 6.4(2H,br d); 6.85(4H,d); 7.15(4H,d); 7.35-7.6(8H,m); 8.6-10.0(4H,br).

Example 21

[R,R,R,R]-α,α'-[oxybis[2,1-ethanediyloxy-4,1-phenylene (1-methyl-2,1-ethanediyl)iminomethylene]] bis[3-chlorobenzenemethanol], dihydrochloride.

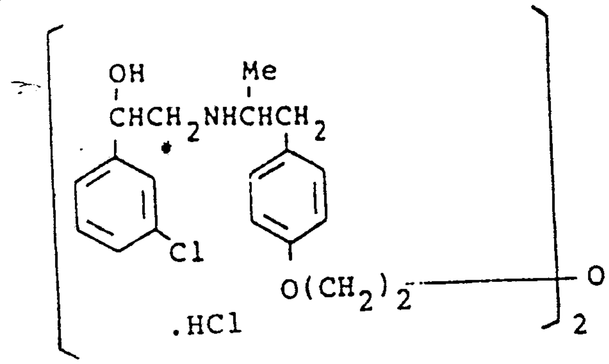

[R,R,R,R]-α,α'-[oxybis[2,1-ethanediyloxy-4,1-phenylene (1-methyl-2,1-ethanediyl)iminomethylene]] bis[3-chlorobenzenemethanol], dihydrochloride, m.p. 68-9° (dichloromethane), was prepared in an analogous manner to the compound described in Example 3.

$[\alpha]_D^{25}$: -28.3° (EtOH).

$^1H$ nmr ($d_6$-DMSO + $D_2O$), ppm

1.25(6H,d); 2.65(2H,); 2.9-3.5(8H, complex); 3.6(4H, complex); 4.1(4H, complex); 5.0(2H, complex); 6.8(4H,d); 7.2(4H,d); 7.4(8H,complex).

Example 22

[R,R,R,R]-5,5'-[1,6-Hexanediylbis[oxy-4-1-phenylene (1-methyl-2,1-ethanediyl)imino(1-hydroxy-2,1-ethanediyl)]]bis[benzene-1,3-diol], dihydrochloride.

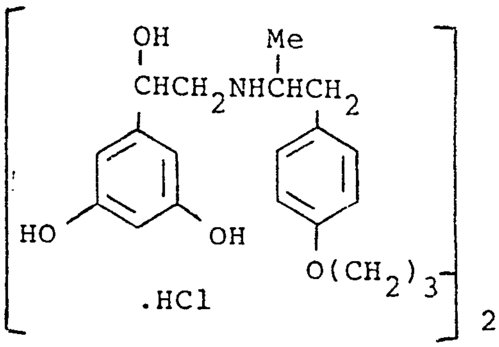

A mixture of [R,R,R,R]-α,α'-[1,6-Hexanediylbis [oxy-4-1-phenylene(1-methyl-2,1-ethanediyl) iminomethylene]]bis[3,5-dibenzyloxybenzenemethanol], dihydrochloride, (1.4g) and 10% Pd-C (0.3g) in methanol (20ml) was shaken under a hydrogen atmosphere at ambient pressure and temperature until hydrogen uptake had ceased. The catalyst was removed by filtration and the solvent evaporated to give [R,R,R,R]-5,5'-[1,6-Hexanediylbis[oxy-4-1-phenylene(1-methyl-2,1-ethanediyl)imino(1-hydroxy-2,1-ethanediyl)]]bis [benzene-1,3-diol], dihydrochloride.

$^1H$ nmr ($d_6$-DMSO), ppm

1.08(6H,d); 1.4-1.55(4H,m); 1.65-1.80(4H,m); 2.5-2.65 (2H,m); 2.85-3.6(8H,m); 3.94(4H,t); 4.60(2H,dd); 6.10(2H,bs, replaceable by $D_2O$); 6.15-6.35(6H,m); 6.85(4H,d); 7.15(4H,d); 8.65(2H, broad, replaceable by $D_2O$); 9.31(6H,broad, replaceable by $D_2O$).

Example 23

[R,R,R,R]-α,α'[1,7-Heptanediylbis[oxy-4,1-phenylene-(1-methyl-2,1-ethanediyl)iminomethylene]]bis[3-chloro-benzenemethanol], dihydrochloride.

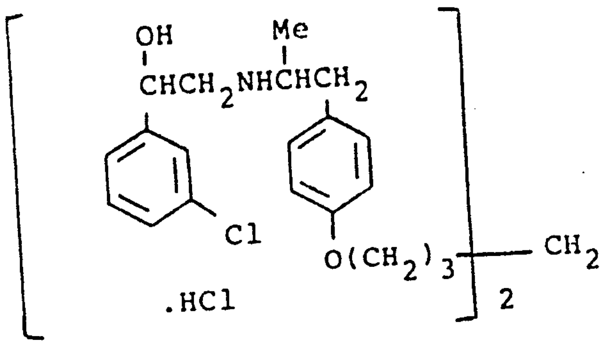

[R,R,R,R]-α,α'[1,7-Heptanediylbis[oxy-4,1-phenylene-(1-methyl-2,1-ethanediyl)iminomethylene]]bis[3-chlorobenzenemethanol], dihydrochloride m.p. 184-7° was prepared using [R,R,R,R]-N,N'-[1,7-heptanediylbis[oxy-4,1-phenylene(1-methyl-2,1-ethanediyl)]]bis[3-chloro-α-hydroxybenzeneacetamide] (20.1g) in an analogous procedure to that described in Example 3.

$[\alpha]_D^{25}$ (MeOH) = -37.0

1H nmr (DMSO-$d_6$), ppm.

1.1(6H,d); 1.4(6H,m); 1.7(4H,m); 2.6(2H,t), 3.0-3.5(8H,m); 3.9(4H,t); 5.1-5.2(2H,broad d); 6.3(2H, broad d, exch $D_2O$); 6.8(4H,d); 7.1(4H,d); 7.3-7.5(8H,m); 8.7-9.5(2H, very broad, exch $D_2O$); 9.5-9.8 (2H, very broad, exch $D_2O$).

Example XI

[R,R,R,R]-N,N'-[1,2-ethanediylbis[imino[2-oxo-2,1-ethanediyl)oxy-4,1-phenylene(1-methyl-2,1-ethanediyl)]]bis[3-chloro-α-hydroxybenzeneacetamide].

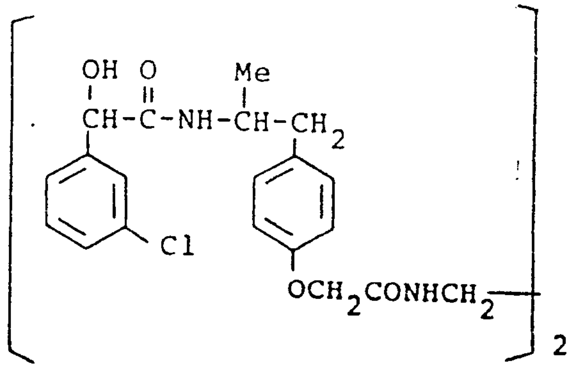

(XI)

A mixture of [R-(R*,R*)]-methyl 2-[4-[2-[(2-(3- chlorophenyl)-2-hydroxy-1-oxoethyl)amino]propy]phenoxy]-acetate (1.95g) and [R-(R*,R*)]-N-[2-[4-[2-(aminoethylamino)-2-oxoethoxyphenyl]-1-methylethyl]-3-chloro-α-hydroxybenzeneacetamide, (2.1g) in methanol (20ml) was stirred at ambient temperature for 2 days. The solvent was evaporated and the residue chromatographed on silica using chloroform/methanol (95:5) as eluent to give [R,R,R,R]-N,N'-[1,2-ethanediylbis[imino[2-oxo-2,1-ethanediyl)oxy-4,1-phenylene(1-methyl-2,1-ethanediyl)]]bis[3-chloro-α-hydroxybenzeneacetamide] as a white solid (1.2g).

$^{1}H$ nmr ($CDCl_3/CD_3OD$) ppm

1.1(6H,d); 2.7(4H,m); 3.45(4H,bs); 4.05(2H,m); 4.4(4H,s); 4.9(2H,s); 6.7-7.5(16H,m).

Example X2

[R-(R*,R*)]-N-[2[4[2-(aminoethylamino)-2-oxoethoxy]phenyl]-1-methylethyl]-3-chloro-α-hydroxybenzeneacetamide].

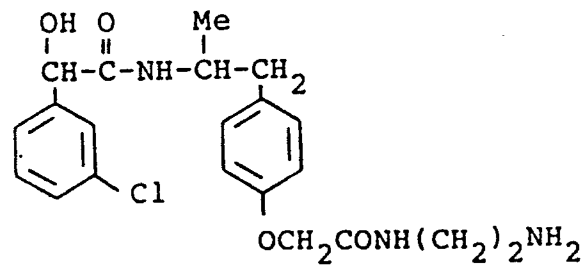

To a solution of [R-(R*,R*)]-methyl 2-[4-[2-[(2-(3-chlorophenyl)-2-hydroxy-1-oxoethyl)amino]propy] phenoxy] acetate (3g, 7.6 mmol) in methanol (20ml) was added ethylene diamine (0.46g, 7.6 mmol). The solution was heated at reflux for 5 h and then allowed to cool to ambient temperature overnight. The solvent was evaporated and the residue chromatographed on silica gel using chloroform/methanol/ammonia solution (90:9:1) as eluent to give [R-(R*,R*)]-N-[2[4[2-(aminoethylamino)-2-oxoethoxy]phenyl]-1-methylethyl]-3-chloro-α-hydroxybenzeneacetamide] as a white solid (2.3g), m.p. 126-130°.

$^1H$ nmr ($CDCl_3/d_6$-DMSO/$D_2O$) ppm

1.1(3H,s); 2.5-3.0(4H,m); 3.35(2H,t); 4.05(1H,m); 4.45(2H,s); 4.9(1H,s); 6.8-7.7(8H,m).

Example X3

[R-(R*,R*)]-Methyl 2-[4-[2-[(2-(3-chlorophenyl)-2-hydroxy-1-oxoethyl)amino]propyl]phenoxy]acetate.

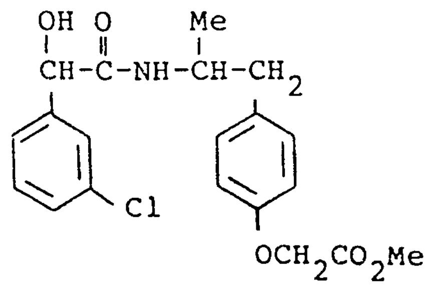

Dicyclohexylcarbodiimide (7.39g, 35.9 mmol) was added in portions to a stirred, ice cooled solution of (R)-3-chloromandelic acid (6.7g, 35.9 mmol), (R)-methyl 2-[4-[(2-amino)propyl]phenoxy]acetate (8g, 35.9 mmol) in dry dimethylformamide (100ml). The mixture was allowed to warm to ambient temperature overnight. The dicyclohexylurea was removed by filtration and the filtrate evaporated to leave a brown oil. This was dissolved in ethyl acetate, washed successively with aqueous potassium carbonate solution, 2M HCl, aqueous potassium carbonate solution, water, dried ($MgSO_4$) and evaporated to give [R-(R*,R*)]-methyl 2-[4-[2-[(2-(3-chlorophenyl)-2-hydroxy-1-oxoethyl)amino]-propyl]phenoxy]acetate as an oil (11.5g).

$^1H$ nmr ($CDCl_3$) ppm

1.1(3H,t); 3.65(2H,m); 3.85(3H,s); 4.1(1H,m); 4.65(2H,s); 4.9(1H,s); 6.05(1H,bd), 6.6-7.0(4H,m), 7.2-7.5(4H,m).

Example X4

[R,R,R,R]-N,N'-[1,6-Hexanediylbis[oxy-4,1-phenylene (1-methyl-2,1-ethanediyl)]]bis[3-chloro-α-hydroxybenzeneacetamide].

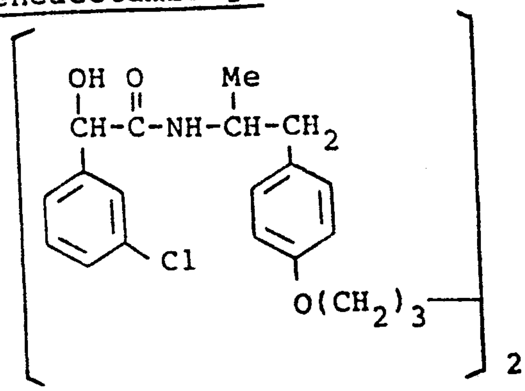

To an ice cooled solution of [R-(R*,R*)]-4,4'-[1,6-hexanediylbis(oxy)]bis[α-methylbenzene ethanamine] (1.9g), (R)-3-chloromandelic acid (1.85g) and 1-hydroxybenzotriazole (1.33g) in dry dimethylformamide (30ml), was added dicyclohexylcarbodiimide (2.03g), portionwise over 5 minutes. The mixture was stirred for 3 days at ambient temperature, filtered, and the solvent evaporated. The residue was partitioned between ethyl acetate and potassium carbonate solution. The organic extract was washed with 2M hydrochloric acid, saturated brine, dried over magnesium sulphate and evaporated to give [R,R,R,R]-N,N'-[1,6-hexanediylbis[oxy-4,1-phenylene (1-methyl-2,1-ethanediyl)]]bis[3-chloro-α-hydroxybenzeneacetamide] which crystallised from ethanol, m.p. 114-117°C.

$[\alpha]_D^{25}$ : -13.8° (MeOH)

$^1H$ nmr ($d_6$-DMSO) ppm

1.01(6H,d); 1.47(4H,m); 1.72(4H,m); 2.5-2.71(4H,m); 3.91(6H,m); 4.86(2H,d-collapses to singlet with $D_2O$);

6.1(2H,d, disappears with $D_2O$); 6.7(4H,d); 7.1(4H,d); 7.25-7.4(8H,m); 7.8(2H,d, disappears with $D_2O$).

Example X5

[R-(R*,R*)]-4,4'-[1,6-Hexanediylbis(oxy)]bis[α-methylbenzeneethanamine], dihydrochloride.

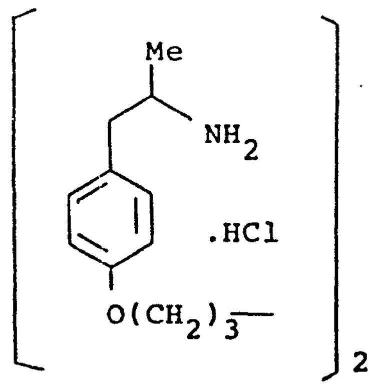

A solution of [R,R,R,R]-4,4'-[1,6-hexanediylbis(oxy)] bis[N-(α-methylbenzyl) -α-methylbenzeneethanamine], dihydrochloride (2.9g) in methanol (100ml) was treated with 10% Pd-C (1g) and the mixture hydrogenated at 50 p.s.i with steam heating for 8 hours. The mixture was cooled to ambient temperature, filtered, and the solvent evaporated to give [R-(R*,R*)]-4,4'-(1,6-hexanediylbis(oxy)]bis[α-methylbenzene-ethanamine], dihydrochloride as a white solid.

$^1H$ nmr, $d_6$-DMSO; ppm.
1.1(6H,d), 1.25-1.95(8H,m), 2.5-3.6(6H,m), 3.95(4H,m), 6.9(4H,d), 7.25(4H,d), 7.85(6H, broad, disappears with $D_2O$).

Example X6

[R,R,R,R]-4,4'-[1,6-Hexanediylbis(oxy)]bis[N-(α-methylbenzyl)-α-methylbenzeneethanamine], dihydrochloride.

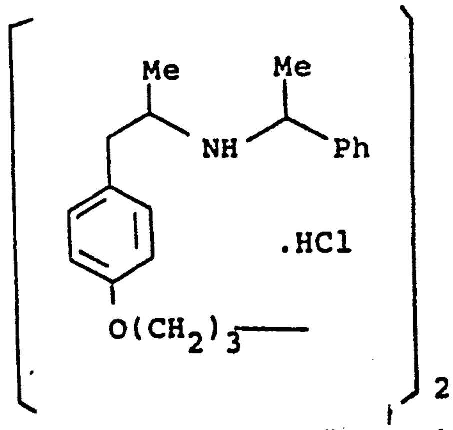

A mixture of [R-(R*,R*)]-4-[2-methyl-2-[(α-methylbenzyl)amino]ethyl]phenol,hydrochloride (5.0g), 1,6-dibromohexane (2.09g) and potassium carbonate (5g) in dry dimethylformamide (50ml) was stirred and heated at 70°C for three days. The mixture was cooled to ambient temperature, filtered and evaporated to dryness. The residue was dissolved in dichloromethane and washed successively with aqueous sodium carbonate solution, water, saturated brine, dried ($MgSO_4$) and evaporated to leave an oil. The residual oil was chromotographed on silica using chloroform-methanol (99:1) as eluent and converted into the hydrochloride salt to afford [R,R,R,R]-4,4'-[1,6-hexanediylbis(oxy)]bis[N-(α-methylbenzyl)-α-methylbenzeneethanamine], dihydrochloride which crystallised from ethanol m.p. 195-201°C.

$[\alpha]_D^{25}$ : +39.3° (C 0.94, MeOH)

$^1H$ nmr, $d_6$-DMSO;ppm

1.11(6H,d), 1.43(4H,m), 1.65(6H,d), 1.65(4H,m), 2.54(2H,m), 2.86(2H,m), 3.32(2H,m), 3.9(4H,m),

4.6(2H,m), 6.8(4H,d), 6.9(4H,d), 7.3-7.55(6H,m), 7.7-7.8(4H,m), 9.37 and 10.3 (4H, broad, disappears with $D_2O$).

Example X7

[R-(R*,R*)]-[2-methyl-2-[(α-methylbenzyl)amino]-ethyl]phenol,hydrochloride.

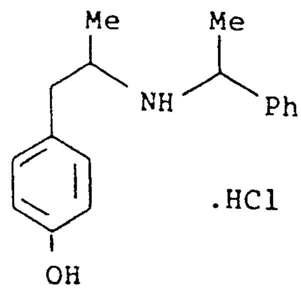

[R-(R*,R*)]-4-Benzyloxy-α-methyl-N-(α-methylbenzyl)-benzeneethanamine, hydrochloride (20g) was dissolved in absolute ethanol (200ml) and treated with 10% Pd-C (2g). The mixture was hydrogenated at ambient temperature and atmospheric pressure until hydrogen uptake had ceased. The mixture was filtered and evaporated in vacuo to give [R-(R*,R*)]-[2-methyl-2-[(α-methylbenzyl)amino]ethyl]phenol,hydrochloride as a crystalline solid which was recrystallised from ethyl acetate, mp. 204-228°C.

$[\alpha]_D^{25}$: +35.7°(C 0.7, MeOH)

$^1H$ nmr, $d_6$-DMSO;ppm

1.1(3H,d), 1.65(3H,d), 2.3-3.45(3H,m), 4.6(1H,m), 6.4-6.9(4H,m), 7.3-7.9(5H,m), 9.4(1H,s, disappears with $D_2O$), 9.4 and 10.15 (2H,broad, disappears with $D_2O$).

BAD ORIGINAL

Example X8

[R-(R*,R*)]-4-Benzyloxy-N-(α-methylbenzyl)-α-methylbenzeneethanamine, hydrochloride.

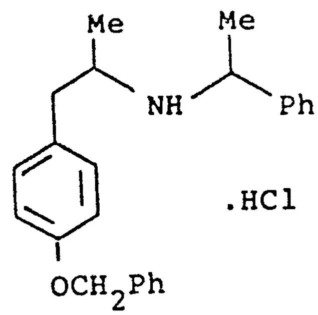

A mixture of (4-benzyloxyphenyl)-2-propanone (99g) and (R)-α-methylbenzylamine (50g) in benzene (400ml) was heated at reflux for 16 hours with removal of water by a Dean and Stark trap. The solution was cooled to ambient temperature and evaporated to dryness to leave a viscous oil. The oil was dissolved in ethanol (800ml) and divided into two equal portions. Each portion was treated with Raney Nickel (20ml) and subjected to hydrogenation at 60 p.s.i. and ambient temperature until reduction was complete. The batches were filtered, concentrated slightly *in vacuo*, treated with ethanolic hydrogen chloride solution until the solutions were acidic, and then, evaporated to dryness to give [R-(R*,R*)]-4-benzyloxy-N- (α-methylbenzyl)-α-methylbenzeneethanamine, hydrochloride which crystallised from ethyl acetate, mp. 154-158°C.

$[\alpha]_D^{25}$: + 37.9° (MeOH)

$^1$H nmr, $d_6$-DMSO; ppm.
1.1(3H,d), 1.65(3H,d), 2.5-3.6(3H,m), 4.55(1H,m), 5.0(2H,s), 6.90(4H,s), 7.2-7.95(10H,m), 9.5 and 10.3(2H, broad, disappears with $D_2O$).

Example X9

1,1'-Oxybis[4-(2-nitro-1-propenyl)benzene]

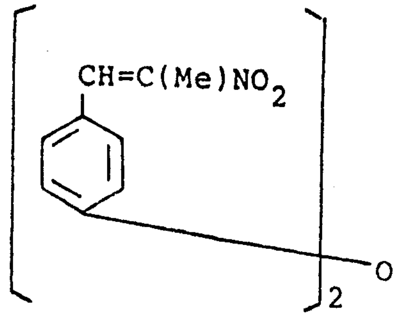

4,4'-Oxybis-[benzaldehyde] (23g) and 4-butanamine (40ml) in toluene (300ml) were heated under reflux for 2h using a Dean and Stark apparatus. The mixture was cooled, evaporated and the residue was dissolved in a mixture of acetic acid (150ml) and nitroethane (26.8ml). After 1h at 100° the mixture was poured into water (400ml) filtered, washed with water and diethylether to yield 1,1'-Oxybis[4-(2-nitro-1-propenyl)benzene] as a yellow solid.

1H-nmr ($CDCl_3$), ppm.

2.38(6H,s); 7.20(4H,d); 7.72(4H,d); 8.14(2H,s).

Example X10

1,1'-[Oxybis(4,1-phenylene)]bis[2-propanone]

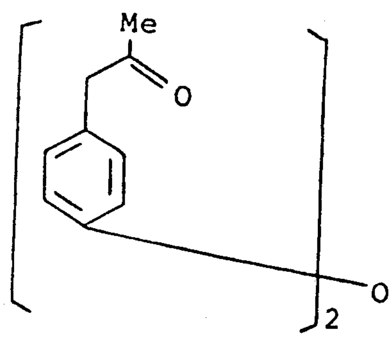

To a suspension of 1,1'-Oxybis-[4-(2-nitro-1-propenyl)-benzene] (19g) and iron powder (32g) in methanol (80ml) and water (25ml) under reflux was added, dropwise acetic acid (160ml). After 3h the mixture was cooled, the methanol evaporated and the aqueous residue was made acidic by the addition of hydrochloric acid and then extracted with dichloromethane. The organic layer was separated, dried and evaporated to yield 1,1'-[Oxybis(4,1- phenylene)]bis[2-propanone].

<u>$^1$H nmr ($CDCl_3$), ppm.</u>

2.20(6H,s); 3.69(4H,s); 6.98(4H,d), 7.22(4H,d).

<u>Example XII</u>

<u>[R-(R*,R*)]-4,4'-Oxybis[α-methyl-N-(α-methylbenzyl)-benzeneethanamine, dihydrochloride</u>

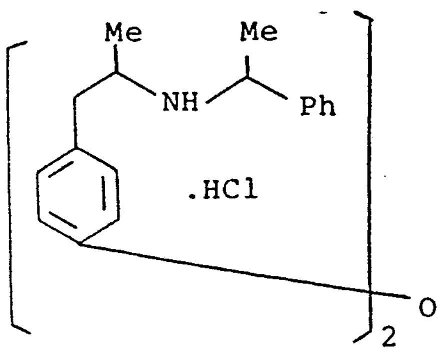

1,1'-[Oxybis(4,1-phenylene)]bis[2-propanone] (10g) and (R)-α-methylbenzylamine (8.5g) in toluene was heated under reflux for 3h using a Dean and Stark apparatus. The solution was cooled and evaporated to give a yellow oil which was dissolved in methanol (100ml) and hydrogenated at NTP in the presence of platinum oxide. After 18h the solution was filtered through diatomaceous earth and the solvent removed under vacuum. The crude product was treated with ethereal hydrogen chloride from which [R-(R*,R*)]-4,4'-Oxybis[α-methyl-N-(α-methylbenzyl)benzeneethanamine, dihydrochloride was obtained after crystallisation from ethanol-ethyl acetate.

$^1H$ nmr ($d_6$-DMSO), ppm.

1.12(6H,d); 1.65(6H,d); 2.7-3.5(6H,m); 4.6(2H,m, collapses to a quartet with $D_2O$); 6.87(4H,d); 7.05(4H,d); 7.4-7.6(6H,m); 7.7-7.9(4H,m); 9.4-9.7(2H,bs); 10.1-10.5(2H,bs).

Example X12

[R-(R*,R*)]-4,4'-Oxybis[α-methylbenzeneethanamine]

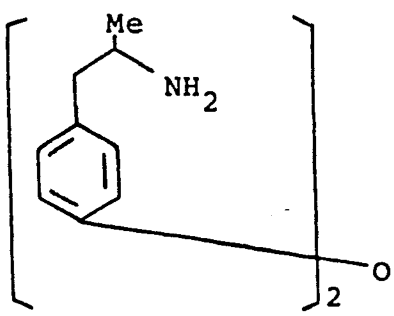

[R-(R*,R*)]-4,4'-Oxybis[α-methyl-N-(α-methylbenzyl)-benzeneethanamine, dihydrochloride (5.5g) in methanol (200ml) containing 10% palladium on charcoal (0.5g) was hydrogenated at 100° under 50 p.s.i. pressure.

After 24h the mixture was filtered through diatomaceous earth and the solvent removed under vacuum. The crude product was partitioned between aqueous 10% sodium hydroxide and dichloromethane, the organic layer was separated, dried and evaporated to yield [R-(R*,R*)]-4,

4'-Oxybis[α-methylbenzeneethanamine] as a colourless oil.

<u>1H nmr ($CDCl_3$), ppm.</u>

1.16(6H,d); 2.7-3.6(6H,m); 7.02(4H,d); 7.33(4H,d), 8.2-8.5(4H,bs, exchanges with $D^2O$).

<u>Example X13</u>

<u>[R,R,R,R]-N,N'-[4,4'-Oxybis[4,1-phenylene(1-methyl-2,1-ethanediyl)]]bis[3-chloro-α-hydroxybenzeneacetamide].</u>

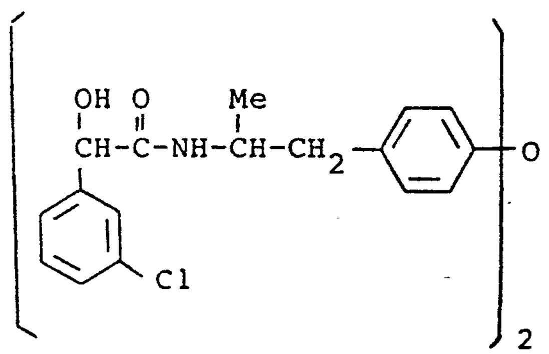

[R,R,R,R]-N,N'-[4,4'-Oxybis[4,1-phenylene(1-methyl-2,1-ethanediyl)]]bis[3-chloro-α-hydroxybenzeneacetamide] was prepared, after chromatography over silica gel (methanol/dichloromethane), from (R)-3-chloromandelic acid (2.1g) and [R-(R*,R*)]-4,4'-oxybis-(α-methylbenzeneethanamine] (1.6g) by an analogous procedure to that described in Example X3.

<u>$^1$H nmr ($CDCl_3$),ppm.</u>

1.15(6H,d); 2.72(4H,d); 4.0 (2H,m); 4.50(2H,d, exchanges with $D_2O$); 4.91(2H,d); 6.28(2H,d, exchanges with $D_2O$); 6.8-7.2(8H,m); 7.3-7.5(8H,m).

Example X14

1,1'-[Carbonyldi-4,1-phenylene]bis[2-propanone]

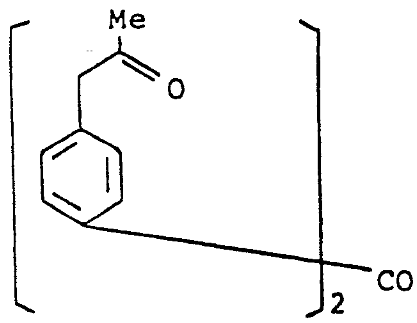

To magnesium metal (0.95g) was added dropwise a solution of (4-bromophenyl)-2-propanone, ethylene ketal (10g) in dry tetrahydrofuran (75ml). After all the magnesium had reacted the mixture was cooled to 5°C and a solution of (4-cyanophenyl)-2-propanone, ethylene ketal (7.9g) in dry tetrahydrofuran (25ml) was added dropwise. After stirring under reflux for 16h the mixture was cooled and poured into 15% aqueous hydrochloric acid. After 20h the organic solvent was removed under vacuum and the resultant aqueous layer extracted with dichloromethane. The organic phase was separated, dried and evaporated to yield an oil which was chromatographed on silica gel. Elution with hexane-ethyl acetate gave 1,1'-[Carbonyldi-4,1-phenylene]bis[2-propanone] as a yellow oil, which crystallised on standing.

$^1H$ nmr ($CDCl_3$), ppm.

2.20(6H,s); 3.80(4H,s); 7.31(4H,d); 7.79(4H,d).

Example X15

[R-(R*,R*)]-Di-[4-(2-(N-α-methylbenzyl)aminopropyl)-phenyl]methanone, dihydrochloride.

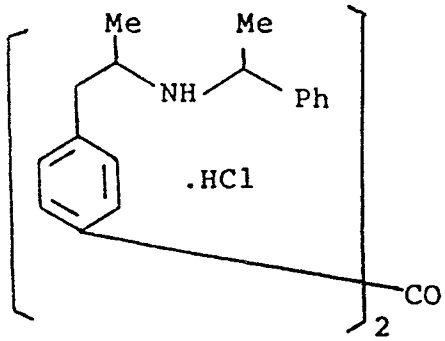

[R-(R*,R*)]-Di-[4-(2-(N-α-methylbenzyl)aminopropyl)-phenyl]methanone, dihydrochloride was prepared, after chromatography over silica gel (dichloromethane-methanol), from 1,1'-[carbonyldi-4,1-phenylene]bis [2-propanone] (7.2g) and (R)-α-methylbenzylamine (5.9g) by an analogous procedure to that described in Example X11.

$^1H$ nmr ($D_6$-DMSO), ppm.

1.15(6H,d); 1.69(6H,d); 2.6-3.6(6H,m); 4.5-4.8(2H,m collapses to a q with $D_2O$); 7.1-8.0(18H,m); 9.5-9.8(2H,bs, exchanges with $D_2O$); 10.0-10.5(2H,bs, exchanges with $D_2O$).

Example X16

[R-(R*,R*)]-4,4'-Methylenebis[α-methylbenzene ethanamine]

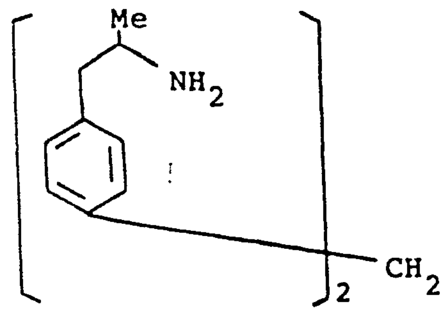

[R-(R*,R*)]-4,4'-Methylenebis[α-methylbenzene ethanamine] was prepared from [R-(R*,R*)]-Di-[4-(2-(N-α-methylbenzyl)aminopropyl)phenyl] methanone, dihydrochloride (5.2g) by an analogous procedure to that described in Example X12.

$^1H$ nmr ($d_6$-DMSO), ppm. dihydrochloride.

1.12(6H,d); 2.3-3.5(6H,bm); 3.87(2H,bs); 7.0-7.5(8H,m), 8.1-8.6(6H,bs, exchange with $D_2O$).

Example X17

[R,R,R,R]-N,N'-[Methylenebis[4,1-phenylene(1-methyl-2,1-ethanediyl]]bis[3-chloro-α-hydroxybenzeneacetamide]

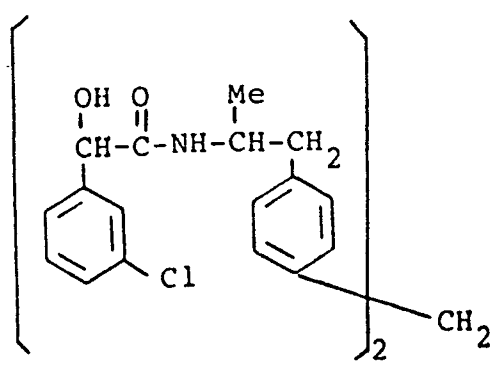

[R,R,R,R]-N,N'-[Methylenebis[4,1-phenylene(1-methyl-2,1-ethanediyl]]bis[3-chloro-α-hydroxybenzeneacetamide] was prepared after chromatography over silica gel (methanol/dichloromethane), from (R)-3-chloromandelic acid (3.17g) and [R-(R*,R*)]-4,4'-Methylenebis [α-methylbenzeneethanamine] (2.4g) by an analogous procedure to that described in example X3.

$^1H$ nmr ($CDCl_3$),ppm.

1.12(6H,d); 2.64(4H,d); 3.86(2H,s); 4.0-4.3(2H,m); 4.41(2H,d, exchanges with $D_2O$); 4.77(2H,s); 5.85(2H,d, exchanges slowly with $D_2O$); 6.8-7.5 (16H,m).

Example X18

4,4'-Thiobis-[benzaldehyde]

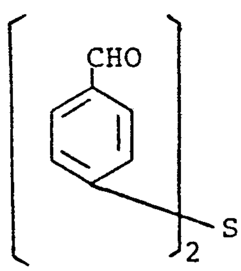

(EX18)

To a solution of 4-fluorobenzaldehyde (50g) in dimethylsulphoxide (150ml) was added, portionwise with stirring, sodium sulphide nonahydrate (41g). After 5h at 100° the mixture was cooled and poured into water (400ml). After filtration 4,4'-Thiobis-[benzaldehyde] was obtained as a pink solid.

$^1H$ nmr ($CDCl_3$), ppm.

7.45(4H,d); 7.87(4H,d), 10.03(2H,s).

Example X19

1,1'-Thiobis[4-(2-nitro-1-propenyl)benzene]

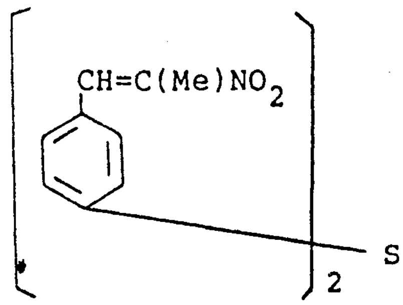

1,1'-Thiobis[4-(2-nitro-1-propenyl)benzene] was prepared from 4,4'-thiobis-[benzaldehyde] (28g) by an analogous procedure to that described in Example X9.

$^1H$ nmr ($CDCl_3$-$d_6$-DMSO), ppm.

2.43(6H,s); 7.46(4H,d); 7.64(4H,d); 8.08(2H,s).

Example X20

1,1'-[(Thiobis(4,1-phenylene)]bis[2-propanone].

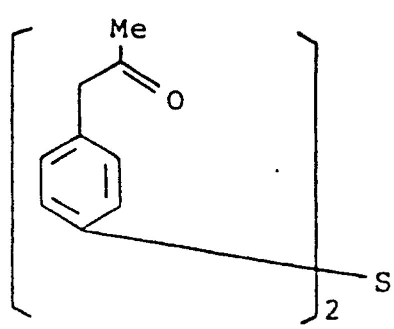

1,1'-[(Thiobis(4,1-phenylene)]bis[2-propanone] was prepared from 1,1'-Thiobis[4-(2-nitro-1-propenyl)-benzene] (32g) by an analogous procedure to that described in Example X10.

$^1H$ nmr ($CDCl_3$), ppm.

2.14(6H,s); 3.66(4H,s); 7.10(4H,d); 7.33(4H,d).

Example X21

1,1'-[Sulphonylbis(4,1-phenylene)]bis[2-propanone

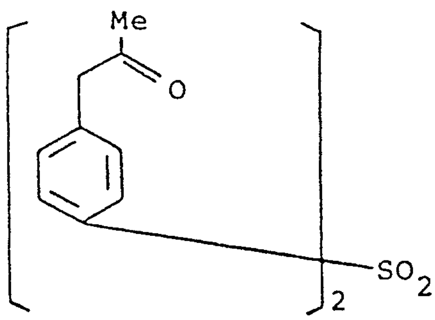

To a solution of 1,1'-[Thiobis(4,1-phenylene)]bis [2-propanone] (25g) in dichloromethane (250ml) was added, portionwise with stirring, 3-chloro perbenzoic acid (28g), keeping the temperature of the reaction mixture below 10°. After stirring at room temperature for 1.5h the mixture was filtered and the filtrate was washed sequentially with 10% aqueous sodium metabisulphite solution and 10% aqueous sodium carbonate. After drying and evaporation the crude product was chromatographed on silica gel. Elution with acetone-hexane gave 1,1'-[Sulphonylbis (4,1-phenylene)]bis[2-propanone as a white solid.

$^1H$ nmr ($CDCl_3$), ppm

2.18(6H,s); 3.78(4H,s); 7.33(4H,d); 7.91(4H,d).

Example X22

[R-(R*,R*)]-4,4'-Sulphonylbis[α-methyl-N-(α-methylbenzyl)benzeneethanamine, dihydrochloride.

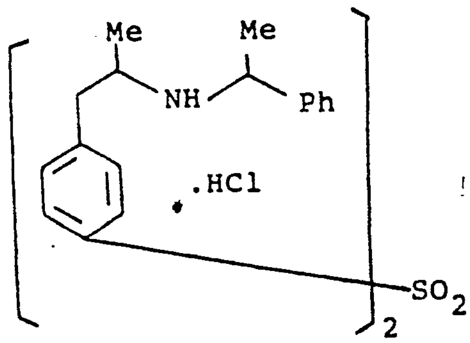

[R-(R*,R*)]-4,4'-Sulphonylbis[α-methyl-N-(α-methylbenzyl)benzeneethanamine, dihydrochloride was prepared, after chromatography on silica gel (dichloromethane-methanol), from 1,1'-[sulphonylbis (4,1-phenylene)]bis [2-propanone] (14g) and (R)-α-methylbenzylamine (10.2g) by an analogous procedure to that described in Example X11.

1H nmr ($d_6$-DMSO), ppm.

1.11(6H,d); 1.62(6H,d); 2.7-3.6(6H,m); 4.3-4.7(2H,bm, collapses to q with $D_2O$); 7.30(4H,d); 7.4-7.6(6H,m); 7.6-7.8(4H,m); 7.88(4H,d); 9.4-9.8(2H,bs, exchanges with $D_2O$); 10.2-10.5 (2H,bs, exchanges with $D_2O$).

Example X23

[R-(R*,R*)]-4,4'-Sulphonylbis[α-methylbenzene, ethanamine]

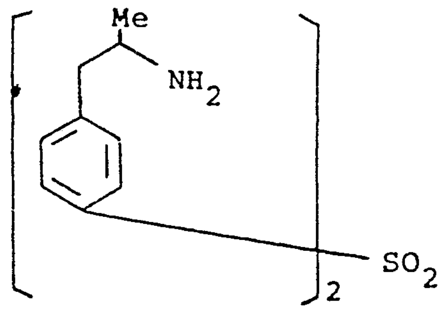

[R-(R*,R*)]-4,4'-Sulphonylbis[α-methylbenzene ethanamine] was prepared from [R-(R*,R*)]-4,4'-Sulphonylbis[α-methyl-N-(α-methylbenzyl) benzeneethanamine]dihydrochloride (18g) by an analogous procedure to that described in Example X12.

$^1H$ nmr ($d_6$-DMSO), ppm (dihydrochloride)

1.15(6H,d); 2.7-3.5(6H,m); 7.55(4H,d); 7.94(4H,d); 8.2-8.7(6H,bs exchanges with $D_2O$).

Example X24

[R,R,R,R]-N,N'-[Sulphonylbis[4,1 -phenylene(1-methyl -2,1-ethanediyl)]]bis[3-chloro-α-hydroxybenzene acetamide

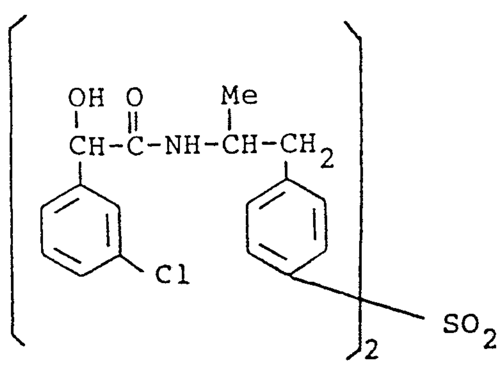

24,

[R,R,R,R]-N,N'-[Sulphonylbis[4,1 -phenylene(1-methyl-2,1-ethanediyl)]]bis[3-chloro-α-hydroxybenzene acetamide was prepared, after chromatography on silica gel (dichloromethane-methanol), from [R-(R*,R*)]-4,4'-Sulphonylbis[α-methylbenzeneethanamine] (5.5g) and (R)-3-chloromandelic acid (6.17g) by an analogous procedure to that described in Example X4.

$^1H$ nmr ($CDCl_3$), ppm

1.11(6H,d); 2.65(4H,d); 3.9-4.3(4H,m, 2H exchanges with $D_2O$); 4.76(2H,d); 6.65(2H,d, exchanges slowly with $D_2O$); 7.0-7.5(12H,m); 7.77(4H,d).

Example X25

Ethyl di[4-(2-oxopropyl)phenyl]-α-hydroxyacetate

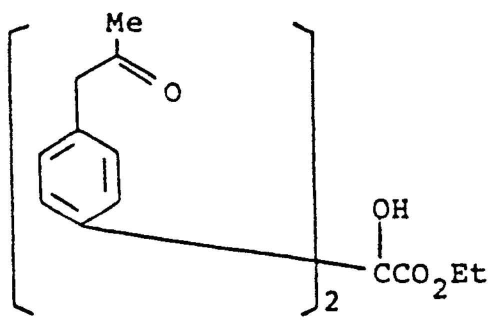

To magnesium metal(1.58g) was added a solution of (4-bromophenyl)-2-propanone ethylene ketal (17.0g) in dry tetrahydrofuran (60 ml) dropwise with stirring. After all the magnesium had reacted the mixture was transferred to a dropping funnel and added with stirring to a solution of diethyl oxalate (4.8g) in dry tetrahydrofuran (60ml) under reflux. After 18 hours the mixture was cooled and added to methanol (50ml) and 6N hydrochloric acid (50ml) and stirred for 12 hours. The organic solvents were removed under vacuum and the residue extracted with dichloromethane.

The organic phase was separated, dried and evaporated to yield the crude product which was chromatographed on silica gel. Elution with ethyl acetate-hexane gave ethyl di[4-(2-oxopropyl)phenyl]-α-hydroxyacetate as an oil which crystallised on standing.

$^1H$ nmr ($CDCl_3$), ppm

1.21(3H,t); 2.12(6H,s); 3.65(4H,s); 4.30(2H,q); 4.36(1H,s, exchanges with $D_2O$); 7.17(4H,d); 7.41(4H,d).

Example X26

[R-(R*,R*)]-Ethyl di[4-[[2-(α-methylbenzyl)amino]propyl]phenyl] -α-hydroxyacetate

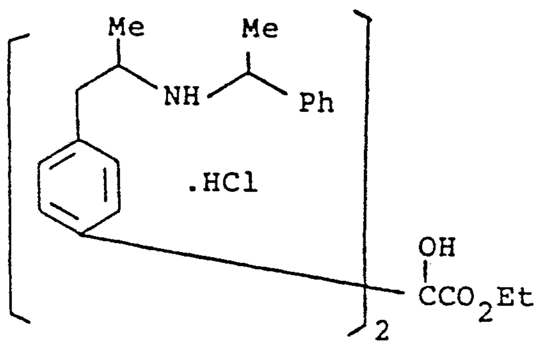

[R-(R*,R*)]-Ethyl di[4-[[2-(α-methylbenzyl)amino]propyl]phenyl]-α-hydroxyacetate was prepared from ethyl di[4-(2-oxopropyl)phenyl]-α-hydroxyacetate (3.0g) in an analogous manner to the compound described in Example X11.

$^1H$ nmr ($CDCl_3$), ppm

1.12(6H,d); 1.20(3H,t); 1.68(6H,d); 2.6-3.5(7H,m 1H exchanges with $D_2O$); 4.17(2H,q); 4.4-4.7(2H,m collaspes to q with $D_2O$); 6.98(4H,d); 7.23(4H,d); 7.4-7.6(6H,m);

7.6-7.9(4H,m); 8.5-8.8(2H, bs, exchanges with $D_2O$), 9.4-9.7(2H, bs, exchanges with $D_2O$).

Example X27

[R-(R*,R*)]-Ethyl di[4-(2-aminopropyl)phenyl] -α-hydroxyacetate

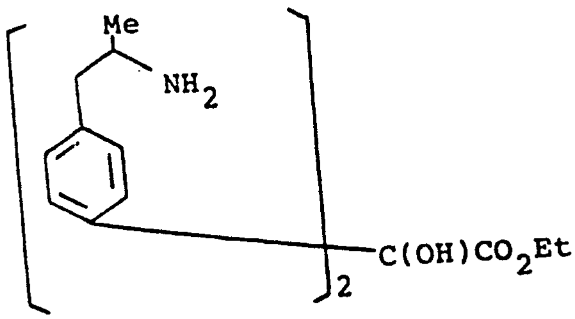

[R-(R*,R*)]-Ethyl di[4-(2-aminopropyl]phenyl] -α-hydroxyacetate was prepared from [R-(R*,R*)]-ethyl di[4-[[2-(α-methylbenzyl)amino]propyl]phenyl] -α-hydroxyacetate, dihydrochloride (4.2g) in an analogous manner to that described in Example X12.

$^1H$ nmr ($d_6$-DMSO), ppm(dihydrochloride)

1.13(6H,d); 1.20(3H,t); 2.6-3.5(7H,m, 1H exchanges with $D_2O$); 4.18(2H,q); 7.1-7.6(8H,m), 8.2-8.6(6H, bs, exchanges with $D_2O$).

Example X28

[R,R,R,R]-Ethyl di[4-[2-[[2-(3-chlorophenyl)-2-hydroxy-1-oxoethyl] amino]propyl)phenyl]-α-hydroxyacetate

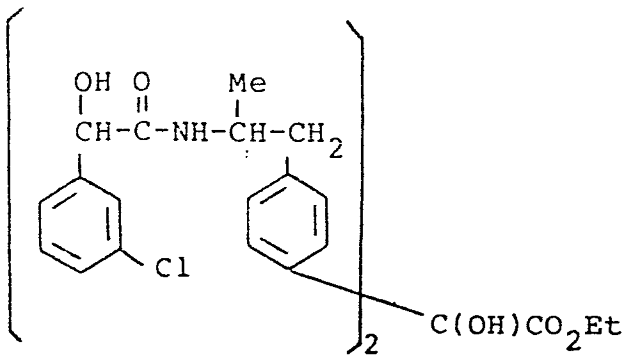

[R,R,R,R]-Ethyl di[4-[2-[[2-(3-chlorophenyl)-2-hydroxy-1-oxoethyl] amino]propyl)phenyl]-α-hydroxyacetate was prepared from [R-(R*,R*)]-ethyl di[(4-(2-aminopropyl)phenyl]-α-hydroxyacetate (1.1g) and (R)-3-chloromandelic acid (1.1g) by an analogous procedure to that described in Example X4.

$^1$H nmr ($CDCl_3$), ppm

1.12(6H,d); 1.20(3H,t); 2.77(4H,d); 4.34(2H,q); 4.1-4.7(5H,m); 3H exchanges with $D_2O$); 4.89(2H,d); 6.65(2H, d slowly exchanges with $D_2O$); 7.0-7.6(16H,m).

Example X29

[R,R,R,R]-4,4'[1,8-Octanediylbis(oxy)]bis[α-methyl-N-(α-methylbenzyl)benzene ethanamine], dihydrochloride

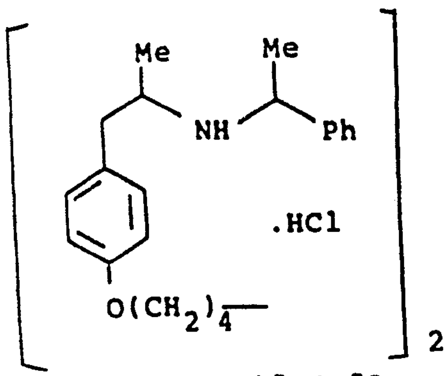

A mixture of [R-(R*,R*)]-4-[2-methyl-2-[(α-methylbenzyl) amino]ethyl]phenol, hydrochloride (5.0g); 1,8-dibromooctane (2.35g) and potassium tert-butoxide (3.9g) in dry dimethylformamide (60ml) was stirred and heated at 80°C for 48 hours. The mixture was coled, filtered and evaporated to dryness. The residue was chromatographed on silica gel. Elution with chloroform-methanol (98:2) gave a colourless oil which was converted into the hydrochloride salt to afford [R,R,R,R]-4,4'[1,8-Octanediylbis(oxy)]bis [α-methyl- N-(α-methylbenzyl)benzene ethanamine], dihydrochloride m.p. 187-191°C.

$^1H$ nmr ($d_6$-DMSO); ppm

1.07(6H,d); 1.34(8H,m); 1.60(6H,d); 1.65(4H,m); 2.89(2H,m); 3.25(2H,m); 3.89(4H,m); 4.59(2H,m); 6.81(4H,d); 6.92(4H,d); 7.44(6H,m); 7.66(4H,m); 9.15 and 9.70(4H,broad, disappears with $D_2O$).

0233686

Example X30

[R-(R*, R*)]-4,4'-[1,8-Octanediylbis(oxy)]bis[α-methylbenzeneethanamine], dihydrochloride.

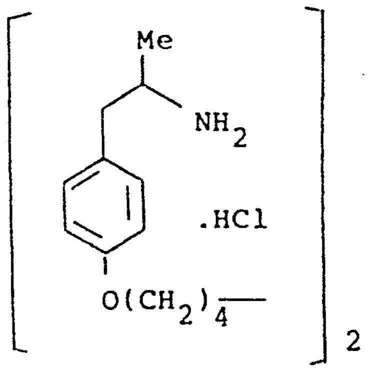

[R-(R*, R*)]-4,4'-[1,8-Octanediylbis(oxy)]bis[α-methylbenzeneethanamine], dihydrochloride.
was prepared from [R,R,R,R,]-4,4'-[1,8-octanediylbis(oxy)]bis[N-(α-methylbenzyl)-α-methylbenzene ethanamine], dihydrochloride (2.35g) by an analogous procedure to that described in Example X5.

$^1H$ NMR, $d_6$-DMSO; ppm.

1.1 (6H, d); 1.25-1.95 (12H, m); 2.60-3.60 (6H, m); 3.92 (4H, m); 6.85 (4H, d); 7.20 (4H, d); 8.10 (6H, broad, disappears with $D_2O$).

Example X32

[R,R,R,R,]-N, N′-[1,4-Butanediylbis[oxy-4,1-phenylene (1-methyl-2,1-ethanediyl)]]bis[3-chloro-α-hydroxybenzeneacetamide].

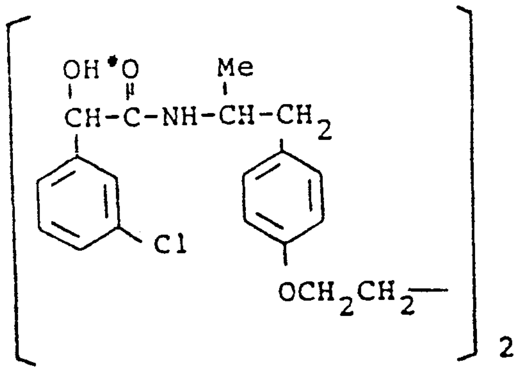

[R,R,R,R,]-N′ N′-[1,4-Butanediylbis[oxy-4,1-phenylene (1-methyl-2,1-ethanediyl)]]bis[3-chloro-α-hydroxybenzeneacetamide] was prepared using [R-(R*, R*)]-4,4′-[1,4-butanediyl-bis(oxy)]bis[α-methyl benzeneethanamine] (0.8g) and (R)-3-chloromandelic acid (0.83g) by an analogous method to that described in Example X4.

$^1$H NMR, ($CDCl_3$); ppm.

1.0 (6H, d); 1.4-2.1 (6H, m); 2.4-2.9 (4H, m); 3.7-4.4 (6H, m); 4.8 (2H, s); 4.9-5.4 (2H, broad, s); 6.5-7.6 (16H, m).

Example X31

[R,R,R,R,]-N,N'-[1,8-Octanediyl]bis[oxy-4,1-phenylene (1-methyl-2,1-ethanediyl)]]bis[3-chloro-α-hydroxy-benzeneacetamide].

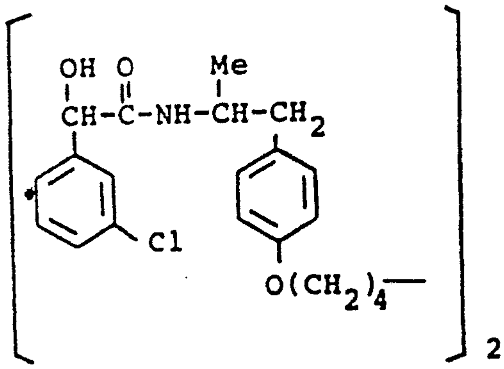

[R,R,R,R,]-N,N'-[1,8-Octanediyl[bis[oxy-4,1-phenylene (1-methyl-2,1-ethanediyl)]]bis[3-chloro-α-hydroxybenzeneacetamide] was prepared by an analogous procedure to that described in Example X4.

$^1H$ NMR, $d_6$-DMSO; ppm.

1.02 (6H, d); 1.49 (8H, m); 1.75 (4H, m); 2.52-2.73 (4H, m); 3.93 (6H, m); 4.88 (2H, d, collapses to singlet with $D_2O$); 6.13 (2H, d, disappears with $D_2O$); 6.72 (4H, d); 7.13 (4H, d); 7.26-7.42 (8H, m); 7.81 (2H, d, disappears with $D_2O$).

Example X33

[R-(R*, R*)]-4,4'-[1,4-Butanediylbis(oxy)]bis[α-methylbenzeneethanamine].

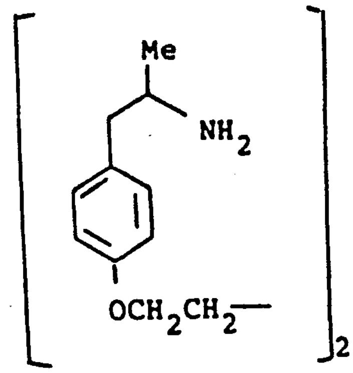

A solution of [R,R,R,R,]-4,4'-[1,4-butanediylbis(oxy)]bis[N-(α-methylbenzyl)-α-methylbenzeneethanamine (4.1g) in methanol (50ml) was acidified (pH 3-4) with methanolic hydrogen chloride solution and hydrogenated at 100 psi and 80°C over 10% Pd-C (1g) for 8 h. The mixture was cooled to ambient temperature, filtered and the solvent evaporated. The residual oil was suspended in chloroform, treated with excess triethylamine and purified by column chromatography on silica. Elution with chloroform: methanol: ammonia (94:5:1) gave [R-(R*, R*)]-4,4'-[1,4-butanediylbis(oxy)]bis[α-methylbenzene ethanamine] as an oil.

$^1H$ nmr ($CDCl_3$), ppm.

1.1 (6H, d); 1.9 (4H, m); 2.2-3.2 (6H, m); 3.2 (4H, s); 3.7-4.4 (4H, m); 6.6-7.4 (8H, m).

Example X34

[R,R,R,R]-4,4'-[1,4-Butanediylbis(oxy)]bis-N-(α-methylbenzyl)[α-methylbenzeneethanamine

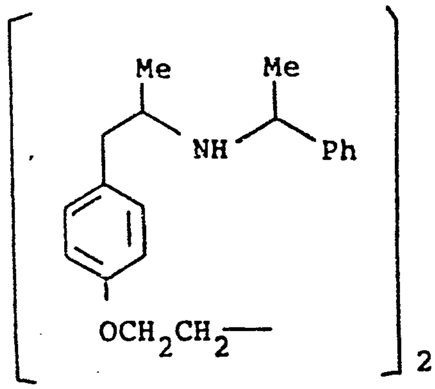

(EX34

A mixture of [R-(R*,R*)-4-[2-methyl-2-[(α-methylbenzyl)amino]ethyl]phenol,hydrochloride (5.0g), 1,4-dibromobutane (1.85g), potassium t-butoxide (3.9g) and 18-crown-6 (0.1g) in dry dimethylformamide (50ml) under a nitrogen atmosphere, was stirred and heated to 80$^{o}$ for 3 days. The mixture was cooled to ambient temperature, filtered and evaporated to dryness *in vacuo*. The residue was dissolved in dichloromethane and washed successively with aqueous sodium bicarbonate solution, water and saturated brine, dried ($MgSO_4$), filtered and evaporated to dryness. The residual oil was chromatographed on silica. Elution with chloroform methanol (99:1) gave [R,R,R,R]-4,4'-[1,4-Butanediylbis-(oxy)]bis-N- (α-methylbenzyl)[α-methylbenzeneethanamine as an oil.

$^{1}H$ nmr ($CDCl_3$),ppm.

0.9(6H,d); 1.3(6H,d); 1.4(2H,m); 1.9(4H,m); 2.4-3.1(6H,m); 3.7-4.3(6H,m); 6.7-7.2(8H,m); 7.2-7.7(10H,m).

Example X35

[R,R,R,R]-N,N'-[1,3-Propanediylbis[oxy-4,1-phenylene-(1-methyl-2,1-ethanediyl)]]bis[3-chloro-α-hydroxy-benzeneacetamide].

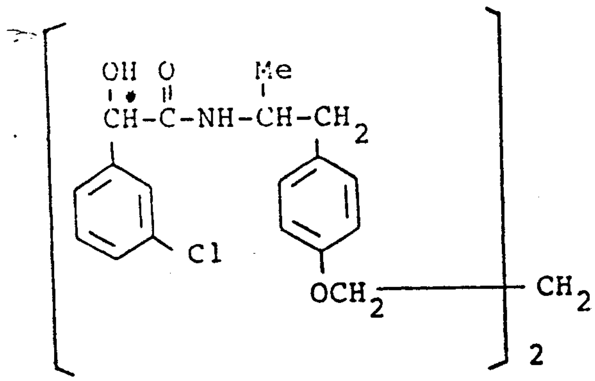

[R,R,R,R]-N,N'-[1,3-Propanediylbis[oxy-4,1-phenylene-(1-methyl-2,1-ethanediyl)]]bis[3-chloro-α-hydroxybenzeneacetamide] was prepared using [R-(R*,R*)]-4,4'-[1,3-propanediylbis(oxy)]bis[α-methylbenzeneethanamine] (1.8g) and (R)-3-chloromandelic acid (1.6g) by an analogous procedure to that described in example X4.

$^1$H nmr ($CDCl_3$) ppm.

1.1 (6H,d); 1.2-2.4 (6H,m); 2.5-3.0 (3H,m); 3.7-4.3 (5H,m); 4.7-4.9 (2H,m); 4.9-5.3 (2H,m); 6.5-7.5 (16H,m).

Example X36

[R-(R*,R*)]-4,4'-[1,3-Propanediylbis(oxy)]bis[α-methylbenzeneethanamine]

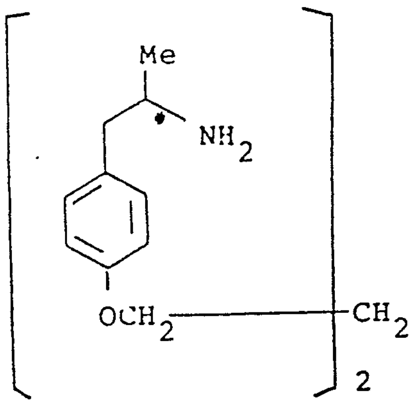

[R-(R*,R*)]-4,4'-[1,3-Propanediylbis(oxy)]bis[α-methylbenzeneethanamine] was prepared using [R,R,R,R]-4,4'-[1,3-propanediylbis(oxy)]bis[N-(α-methylbenzyl)-α-methylbenzeneethanamine] (3.3g), in an analogous procedure to that described in Example X33.

$^1H$ nmr (MeOH $d_4$) ppm.

1.2(6H,d); 1.6-2.3(4H,m); 2.5-3.4(4H,m); 4.1(4H,t); 6.8-7.4(8H,m).

Example X37

[R,R,R,R]-4,4'-[1,3-Propanediylbis(oxy)]bis[α-methyl-N-(α-methylbenzyl)benzeneethanamine].

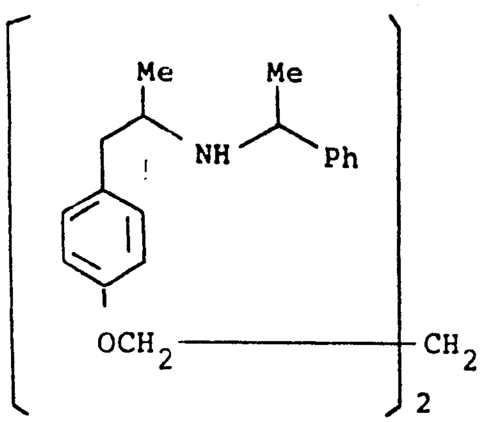

[R,R,R,R]-4,4'-[1,3-Propanediylbis(oxy)]bis[α-methyl-N-(α-methylbenzyl)benzeneethanamine] was prepared using [R-(R*,R*)]-4-[2-methyl-2-[(α-methylbenzyl)amino]-ethyl]phenol, hydrochloride (5.0g) and 1,3-dichloropropane (1.0g) in an analogous procedure to that described in Example X34.

$^1H$ nmr $CDCl_3$ ppm.

0.9(6H,d); 1.3(6H,d); 1.7-3.0(10H,m); 3.6-4.3(6H,m); 6.6-7.6(18H,m).

Example X38

[R,R,R,R]-4,4'-[1,9-Nonanediylbis(oxy)]bis[α-methyl-N-(α-methylbenzyl)benzeneethananamine].

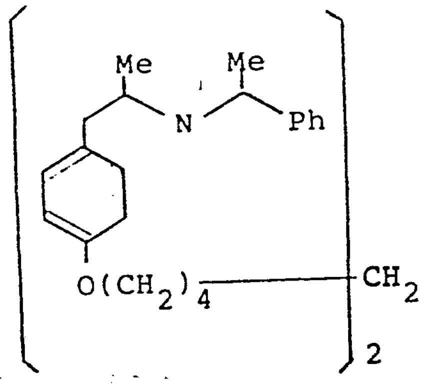

[R,R,R,R]-4,4'-[1,9-Nonanediylbis(oxy)]bis[α-methyl-N-(α-methylbenzyl)benzeneethananamine] was prepared using 1,9-dibromononane (1.62g) in an analogous manner to that described in Example X6.

$^1H$ nmr ($CDCl_3$) ppm.

0.95 (6H,d); 1.1-2.1(22H,m); 2.3-3.1(6H,m); 3.7-4.3(6H,m); 6.7-7.2(8H,m); 7.3(10H,s)

Example X39

[R-(R*,R*)]-4,4'-[1,9-Nonanediylbis(oxy)]bis[α-methyl-benzeneethanamine]

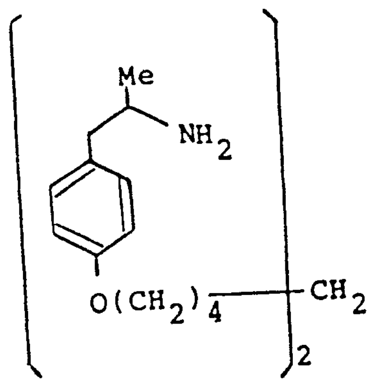

[R-(R*,R*)]-4,4'-[1,9-Nonanediylbis(oxy)]bis[α-methylbenzeneethanamine] was prepared in an analogous manner to that described in Example X5.

Example X40

[R,R,R,R]-N,N'-[1,9-Nonanediylbis(oxy)-4,1-phenylene-(1-methyl-2 , 1-ethanediyl]]bis[3-chloro-α-hydroxy-benzeneacetamide].

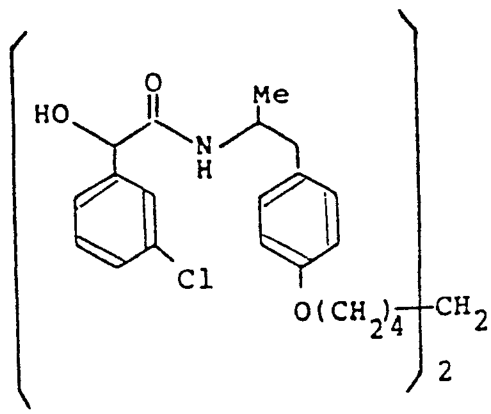

[R,R,R,R]-N,N'-[1,9-Nonanediylbis(oxy)-4,1-phenylene-(1-methyl-2-, 1-ethanediyl)]bis[3-chloro-α-hydroxybenzeneacetamide] was prepared in an analogous manner to that described in Example X4.

$^1H$ nmr ($CDCl_3$) ppm.

1.1(6H,d); 1.1-2.1(14H,m); 2.5-2.8(4H,m); 3.7-4.2(6H,m); 4.2-4.6(2H,broad); 4.8(2H,s); 6.3(2H,bd); 6.7-7.1(8H,m); 7.2-7.6(8H,m).

Example X41

[R-(R*, R*)]-3-Chloro-α-hydroxy-N-[2-(4-hydroxyphenyl)-1-methylethyl]benzeneacetamide.

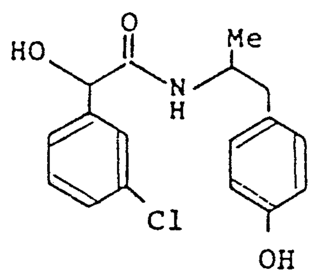

To a mixture of (R)-4-(2-aminopropyl]phenol (2.3g), (R)-3-chloromandelic acid (2.9g) and 1-hydroxybenzotriazole (2.07g) in dry dimethylformamide (40ml), was added dicyclohexylcarbodiimide (3.2g). The mixture was stirred for 16h at ambient temperature, filtered, and the solvent evaporated. The residue was dissolved in ethyl acetate, washed successively with sodium bicarbonate solution, 2M hydrochloric acid, brine, and dried ($MgSO_4$). Evaporation of the solvent gave [R-(R*, R*)]-3-chloro-α-hydroxy-N-[2-(4-hydroxyphenyl)-1-methyl ethyl] benzeneacetamide

$^1H$ nmr, ($CDCl_3$ and $CD_3OD$), ppm.

1.15 (3H, d), 2.65 (2H, d), 3.9-4.4 (1H, m), 4.9 (1H,s) 6.7-7.5 (8H, m).

Example X42

(R)-4-(2-Aminopropyl)phenol, hydrochloride

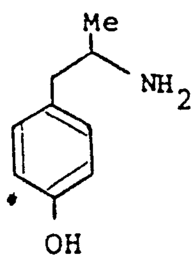

.HCl

(EX42)

To a solution of [R-(R*, R*)]-4-[2-(α-methylbenzylamino)propyl]phenol, hydrochloride (11.5g) in ethanol (100ml), was added 10% Pd-C (2g) and the mixture hydrogenated at 50 p.s.i and 60°C for 16h. The catalyst was filtered off, and the ethanol evaporated to give (R)-4-(2-Aminopropyl)phenol, hydrochloride

$^1H$ nmr ($d_6$ DMSO), ppm.

1.10 (3H, d); 2.56 (1H, dd); 2.95 (1H, dd); 3.30 (1H, m) 6.72 (2H, d); 7.03 (2H, d); 8.17 (3H, bs, replaceable by $D_2O$) 9.40 (1H, s, replaceable by $D_2O$)

Example X43

[R-(R*, R*)]-3-Chloro-α-[[[2-(4-hydroxyphenyl)-1-methylethyl]amino]methyl]benzenemethanol.

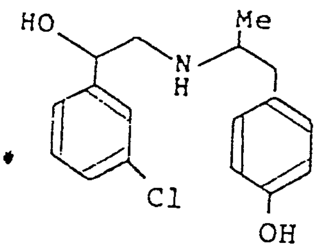

To a solution of [R-(R*, R*)]-3-chloro-α-hydroxy-N-[2-(4-hydroxyphenyl)-1-methylethyl]benzene acetamide (2.8g) in dry tetrahydrofuran (50ml), was added dropwise, a solution of borane-methyl sulphide (4.4ml, 44 mmol) in dry tetrahydrofuran (5ml). The solution was stirred and heated at 60°C for 2 h. The solution was cooled to ambient temperature and methanol added cautiously to destroy excess borane-methyl sulphide. After excess reagent was destroyed, methanolic-hydrogen chloride solution was added until the solution was acidic. The solvent was evaporated to leave a colourless oil. The amine free base was liberated by shaking with aqueous sodium bicarbonate and extracting with ethyl acetate. The material was purified by chromatography on silica using chloroform/methanol (98:2) as eluant, and crystallised from ether, m.p. 107-109°C.

$[\alpha]_D^{25}$ -44.1° methanol (C = 0.25).

$^1H$ nmr ($CDCl_3$), ppm.

1.08 (3H, d), 2.5-2.95 (5H, m), 4.26 (3H, broad, disappears with $D_2O$) 4.52 (1H, dd), 6.74 (2H, d), 6.95 (2H, d), 7.1-7.3 (4H, m).

Example X44

[R-(R*, R*)]-4-Benzyloxy-α-hydroxy-N-[2-(4-hydroxyphenyl)-1-methylethyl]benzeneacetamide

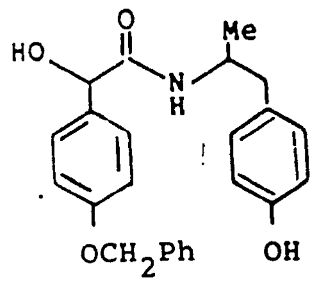

[R-(R*, R*)]-4-benzyloxy-α-hydroxy-N-[2-(4-hydroxyphenyl)-1-methylethyl]benzeneacetamide was prepared in an analogous manner to that described in Example X41.

$^1H$ nmr ($CDCl_3$), ppm.

1.2 (3H, d,), 2.65 (2H, bd), 3.9-4.4 (1H, m), 4.85 (1H, s), 5.1 (2H, s), 6.4-7.4 (11H, m), 7.6 (5H, s).

Example X45

[R-(R*, R*)]-4-Benzyloxy-α-[[[2-(4-hydroxyphenyl)-1-methylethyl]amino]methyl]benzenemethanol.

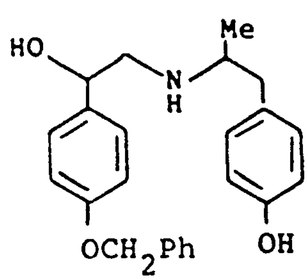

To a slurry of lithium aluminium hydride (0.58g) in dry tetrahydrofuran (20ml) was added dropwise, a solution of [R-(R*, R*)]-4-benzyloxy-α-hydroxy-N-[2-

(4-hydroxyphenyl)-1-methylethyl]benzeneacetamide (1.5g) in dry tetrahydrofuran (10ml). The mixture was stirred and heated at reflux under a nitrogen atmosphere for 16 h. After cooling to ambient temperature, excess reducing agent was destroyed by the careful addition of saturated sodium sulphate solution. The mixture was filtered, the filtrate evaporated, and the residue chromatographed on silica using chloroform/methanol (98:2) as eluant.

$[\alpha]_D^{25}$ : -36.8° (methanol).

<u>$^1$H nmr( $d_6$-DMSO), ppm.</u>

0.9 (3H, d), 1.6 (1H, broad, disappears with $D_2O$), 2.25-2.4 (1H, m), 2.5-2.8 (4H, m), 4.5 (1H, dd), 5.08 (2H, s) 6.65 (2H, d), 6.85-7.0 (4H, m), 7.15-7.25 (2H, m) 7.3-7.5 (5H, m), 9.11 (2H, broad, disappears with $D_2O$).

<u>Example X46</u>

<u>[R-(R*, R*)]-α-[[[2-[4-(6-Bromohexyloxy)phenyl]-1-methylethyl]amino]methyl]-3-chlorobenzenemethanol.</u>

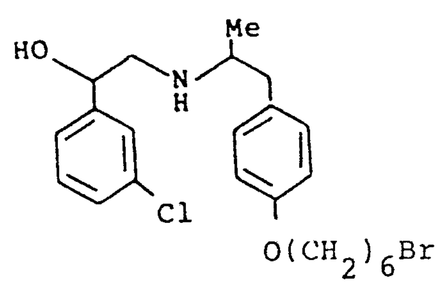

To a suspension of 99% sodium hydride (45 mg) in dry tetrahydrofuran (20ml) was added [R-(R*,R*)]-3-chloro-α-[[[2-(4-hydroxyphenyl)-1-methylethyl]amino]methyl]-benzenemethanol (0.5g). After stirring for 5 minutes

ambient temperature, dibromohexane (0.5g). was added together with a trace of 18-crown-6. The reaction mixture was heated at reflux for 3 h. left to cool to ambient temperature overnight, filtered, and the solvent evaporated. The residue was dissolved in chloroform, washed successively with water and brine, the dried over magnesium sulphate. Evaporation of the solvent gave an oil which was purified by chromatography on silica using chloroform/methanol (98:2). The [R-(R*, R*)]-α-[[[2-[4-(6-Bromohexyloxy) phenyl]-1-methylethyl]amino]methyl]-3-chlorobenzenemethanol was obtained as an oil.

<u>$^1$H nmr ($CDCl_3$), ppm.</u>

1.05 (3H, d), 1.3-2.1 (8H, m), 2.4-2.9 (5H, m), 3.0 (2H, bs, disappears on $D_2O$), 3.4 (2H, t), 3.95 (2H, t), 4.6 (1H, dd), 6.8-7.5 (8H, m).

Example X47

[R,R,R,R]-α-[[[[2-[4-[6-[4-[2-[2-(4-Benzyloxyphenyl)-2-hydroxyethyl]amino]propyl]phenoxy]hexyloxy]phenyl]-1-methylethyl]amino]methyl]-3-chlorobenzenemethanol.

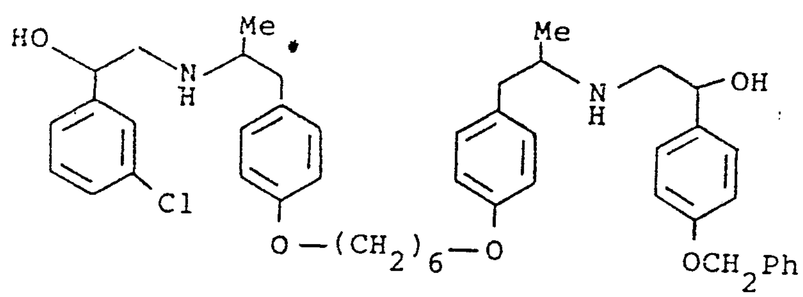

[R,R,R,R]-α-[[[[2-[4-[6-[4-[2-[2-(4-Benzyloxyphenyl)-2-hydroxyethyl]amino]propyl]phenoxy]hexyloxy]phenyl]-1-methylethyl]amino]methyl]-3-chlorobenzenemethanol was prepared by an identical procedure described in Example

X46 from R-[(R*,R*)]-α-[[[2-[4-(6-bromohexyloxy) phenyl]-1-methylethyl]amino]methyl]-3-chlorobenzenemethanol (1.2g) and R-[(R*,R*)]-4-benzyloxy-α-[[[2-(4-hydroxyphenyl)-1-methylethyl] amino]methyl]benzenemethanol (1.0g).

$^1$H nmr ($CD_3OD$), ppm.

1.1(6H,d); 1.25-2.1(8H,m); 2.35-3.1(10H,m), 3.95(4H,t); 4.5-4.80(2H,m); 5.15(2H,s); 6.65-7.6(21H,m).

Example X48

[R,R,R,R]-N,N'-[1,2-Ethanediylbis[iminocarbonyl-4, 1-phenylene(1-methyl-2,1-ethanediyl)]]bis[3-chloro-α-hydroxybenzeneacetamide]

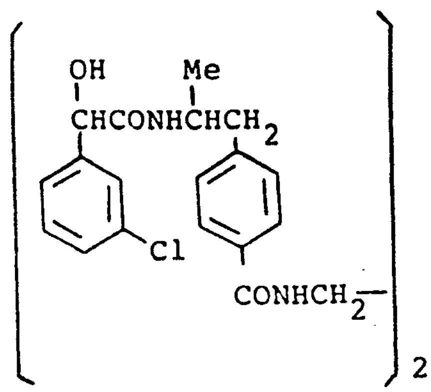

Dicyclohexylcarbodiimide (0.593g, 2.9mmol) was added portionwise to a solution of 1-hydroxybenzotriazole hydrate (0.427g, 3.2 mmol), ethylene diamine (0.077g, 1.5 mmol) and R-[(R*,R*)]-4-[2-[[2-(3-chlorophenyl)-2-hydroxy-1-oxoethyl]amino]propyl]benzoic acid (0.91g, 2.9 mmol) in dimethylformamide (25ml) and the mixture stirred overnight. The reaction mixture was filtered and the filtrate concentrated to give [R,R,R,R]-N,N'-[1,2-Ethanediyl-bis[iminocarbonyl-4, 1-phenylene(1-methyl-2,1-ethanediyl)]]bis [3-chloro-α-hydroxybenzeneacetamide] 0.6g as a white

solid m.p. 257-259° (dioxan-methanol)-$[\alpha]_D^{25}$: 12.6°(DMSO).

$^1H$ nmr ($DMSOd_6$), ppm.

1.05(6H,d); 2.75(4H,d.d.d.); 3.3(2H,s); 3.45(4H,bs); 4.0(2H,m); 4.6(2H,d); 6.2(2H,d); 7.1-7.9(18H,m); 8.5(2H,brs).

Example X49

[R-(R*,R*)]-4-[2-[[2-(3-Chlorophenyl)-2-hydroxy-1-oxoethyl]amino]propyl]benzoic acid

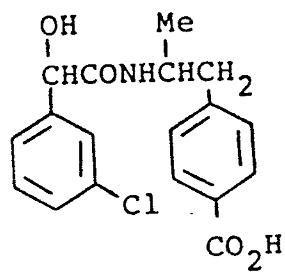

[R-(R*,R*)]-Methyl 4-[2-[[2-(3-chlorophenyl)-2-hydroxy-1-oxoethyl]amino]propyl]benzoate (9.31g, 25.7 mmol) was dissolved in tetrahydrofuran (90ml) and added to a solution of sodium hydroxide (1.0g, 25.3 mmol) in water (90ml) and the mixture stirred for 18 h and then extracted with ethyl acetate. The aqueous layer was acidified with hydrochloric acid (2N) and extracted with ethy acetate. The organic layer was dried and concentrated to an oil which was chromatographed on silica. Elution with chloroform/ methanol (9:1) gave [R-(R*,R*)]-4-[2-[[2-(3-chloro phenyl)-2-hydroxy-1-oxoethyl]amino]propyl]benzoic acid, 7.73g, as a white foam.

$[\alpha]_D^{25}$: -10.2°(MeOH)

$^1$H nmr ($CDCl_3$), ppm.

1.2(3H,d); 2.6(2H,d.d.d.); 4.35(1H,m); 5.0(1H,s); 6.0(1H,d); 7.05(2H,d); 7.15-7.35(4H,m); 7.9(2H,d).

Example X50

[R-(R*,R*)]-Methyl 4-[2-[[2-(3-chlorophenyl)-2-hydroxy-1-oxoethyl]amino]propyl]benzoate

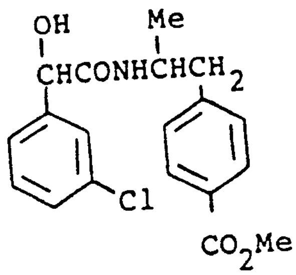

Dicyclohexylcarbodiimide (5.66g, 27.5 mmol) was added in portions to a stirred, ice cooled solution of (R)-3-chloromandelic acid (5.17g, 27.7 mmol), (R)-methyl 4-[2-aminopropyl]benzoate (5.31g, 27.5 mmol), and 1-hydroxybenzotriazole hydrate (4.07g, 30 mmol) in dry dimethylformamide (50ml). The mixture was allowed to rise to ambient temperature overnight, filtered and concentrated in vacuo. The residue was dissolved in ethyl acetate and washed successively with sodium bicarbonate solution and brine. The organic layers were combined, dried ($MgSO_4$) and evaporated to give [R-(R*,R*)]-methyl4-[2-[[2-(3-chlorophenyl)-2-hydroxy-1-oxoethyl]amino]propyl]benzoate (9.31g, 94%) as a white solid, m.p. 131-132°C.

$[\alpha]_D^{25}$: -11.8° (MeOH)

$^1$H nmr ($CDCl_3$), ppm.

1.15(3H,d); 2.75(2H,d.d.); 3.9(3H,s); 4.2(1H,m), 4.5(1H,brs); 4.8(1H,s); 6.4(1H,d); 7.05(2H,d); 7.1-7.3(4H,m); 7.85(2H,d).

Example X51

[R,R,R,R]-N,N'-[1,4-Butanediylbis[iminocarbonyl-4,1-phenylene(1-methyl-2,1-ethanediyl)]]bis[3-chloro-α-hydroxybenzeneacetamide.

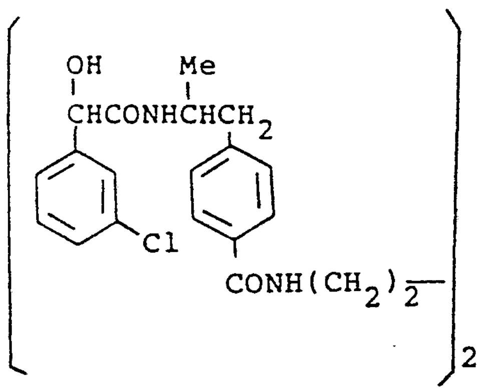

[R,R,R,R]-N,N'-[1,4-Butanediylbis[iminocarbonyl-4,1-phenylene(1-methyl-2,1-ethanediyl)]]bis[3-chloro-α-hydroxybenzeneacetamide was prepared in an analogous manner to the compound described in Example X48.

m.p. 220-222°C, $[\alpha]_D^{25}$: -7.0° (DMSO)

$^1H$ nmr ($d_6$-DMSO) ppm

1.05(6H,d); 1.55(4H,brs); 2.75(4H,m); 3.3(4H,m), 4.0(2H,m); 4.8(2H,d); 6.2(2H,d); 7.15-7.5(12H,m); 7.70(4H,d); 7.9(2H,d); 8.4(2H,m).

Example X52

[R,R,R,R,]-N,N'-[1,6-Hexanediylbis[iminocarbonyl-4,1-phenylene(1-methyl-2,1-ethandiyl)]]bis[3-chloro-α-hydroxybenzeneacetamide.

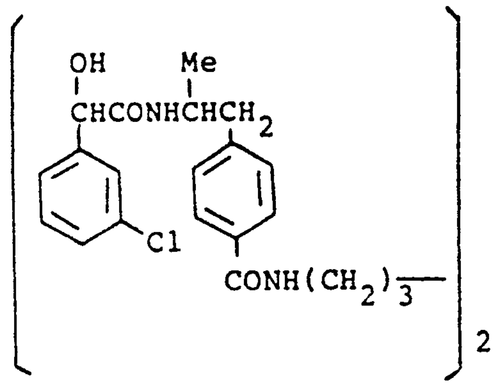

[R,R,R,R,]-N,N'-[1,6-Hexanediylbis[iminocarbonyl-4,1-phenylene(1-methyl-2,1-ethandiyl)]]bis[3-chloro-α-hydroxybenzeneacetamide m.p. 160-161°C was prepared in an analogous manner to the compound described in Example X48.

$^1H$ nmr ($d_6$-DMSO), ppm.

1.1 (6H, d); 1.3 (4H, br s); 1.55 (4H, br s); 2.75 (4H, m) 3.25 (4H, m); 4.0 (2H, m); 4.8 (2H, s); 6.2 (2H, br s) 7.0-7.5 (12H, m); 7.7 (4H, d); 7.85 (2H, d); 8.4 (2H, m).

Example X53

[R,R,R,R]-N, N'-[1,2-Ethanediylbis[4,1-phenylene (1-methyl-2,1-ethanediyl]]bis[3-chloro-α-hydroxybenzeneacetamide].

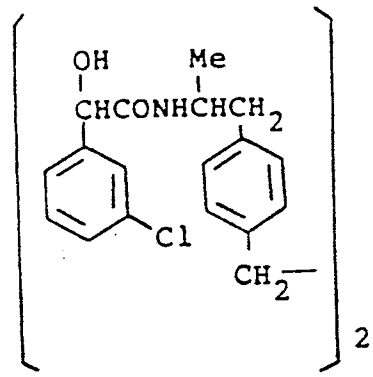

[R,R,R,R]-N, N'-[1,2-Ethanediylbis[4,1-phenylene (1-methyl-2,1-ethanediyl]]bis[3-chloro-α-hydroxybenzeneacetamide] was prepared in an analogous manner to the compound described in Example X4.

$^1H$ nmr ($CDCl_3$) ppm.

1.1 (6H, d); 2.4-3.0 (8H, m); 4.55 (2H, m); 4.8 (2H, s) 6.3 (2H, br); 6.7-7.4 (18H, m).

Example X54

[R-(R*, R*)]-4,4'-(1,2-Ethanediyl)bis [α-methylbenzeneethanamine]dihydrochloride.

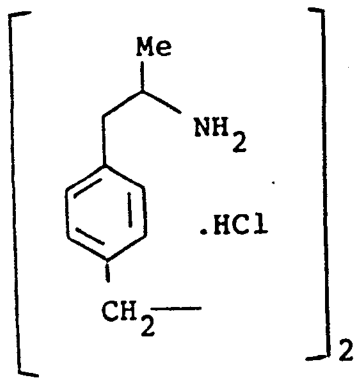

[R-(R*, R*)]-4,4'-(1,2-Ethanediyl)bis [α-methylbenzeneethanamine]dihydrochloride was prepared in an analogous manner to the compound described in Example X5 except that a pressure of 100 psi was used.

$^1H$ nmr, ($d_6$-DMSO); ppm.

1.15 (6H, d); 2.7-3.1 (4H, m); 3.1-3.5 (6H, m); 7.05 (8H, s); 8.2 (6H, br).

Example X55

[R,R,R,R]-4,4'-(1,2-Ethanediyl)bis[α-methyl-N-(α-methylbenzyl)benzeneethanamine, dihydrochloride

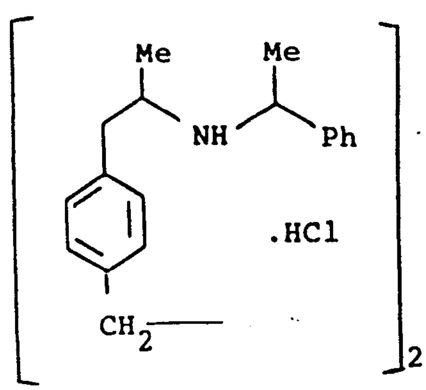

[R,R,R,R]-4,4′-(1,2-Ethanediyl)bis[α-methyl-N-(α-methylbenzyl)benzeneethanamine, dihydrochloride was prepared in an analogous manner to the compound described in Example X8 except that methanol was used as solvent and platinum oxide as the catalyst. Hydrogenation was carried out at NTP.

$[\alpha]_D^{25}$: +35.2 (DMSO).

<u>$^1$H nmr, ($d_6$-DMSO); ppm.</u>

1.1 (6H, d); 1.6 (6H, d); 2.5-3.0 (8H, m); 3.4 (2H, m) 4.55 (2H, m); 6.8 (4H, d); 7.05 (4H, d); 7.25-7.8 (10H, m) 9.25-10.1 (4H, br).

<u>Example X56</u>

<u>1,1′-[1,2-Ethanediylbis(4,1-phenylene)]bis[2-propanone].</u>

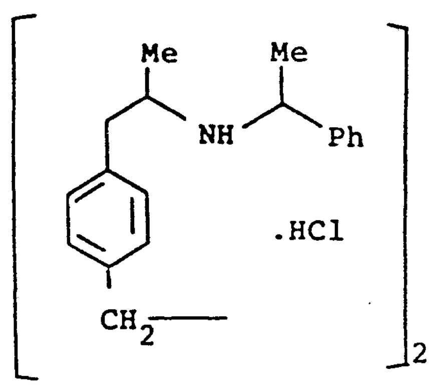

To a stirred suspension of magnesium turnings (0.886g, 36.9 mmol) in tetrahydrofuran (150ml) was added (4-bromomethylphenyl)-2-propanone, ethylene ketal (20.1g, 74 mmol) slowly such that the mixture refluxed gently. After the addition was complete the mixture was refluxed for a further 2 h, allowed to cool and dilithium tetrabromocuprate (18 mmol) added. The solution was stirred for 16 h then diluted with ethyl acetate and washed sequentially with dilute hydrochloric acid, saturated sodium bicarbonate

solution and brine. The organic fraction was dried ($MgSO_4$) and concentrated to an oil. Chromatographic purification on silica (40-60 petrol-4:1 petrol/ether) gave an oil (5.32g, 38%). This was dissolved in tetrahydrofuran (30ml) and 2N hydrochloric acid (50ml) and the solution stirred for 2.5 h after which time the mixture was extracted with ethyl acetate and the organic layers washed with saturated sodium bicarbonate solution, dried ($MgSO_4$) and concentrated. Chromatography of the residues on silica (chloroform) provided 1,1'-[1,2-Ethanediylbis(4,1-phenylene)] bis[2-propanone], (3.74g, 92%).

<u>$^1H$ nmr ($CDCl_3$) ppm.</u>

2.15 (6H, s); 2.55 (4H, m); 3.7 (4H, s); 7.15 (8H, s).

<u>Example X57</u>

<u>[R,R,R,R]-N,N'-[1,6-Hexanediylbis[4,1-phenylene (1-methyl-2,1-ethanediyl]]bis[3-chloro-α-hydroxybenzeneacetamide].</u>

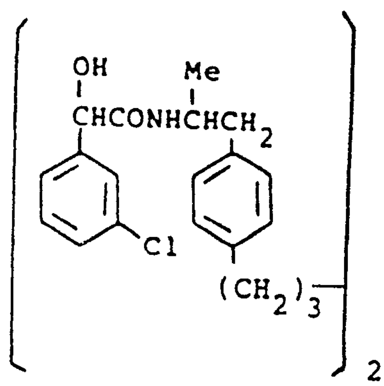

[R,R,R,R]-N,N'-[1,6-Hexanediylbis[4,1-phenylene (1-methyl-2,1-ethanediyl]]bis[3-chloro-α-hydroxybenzeneacetamide] was prepared in an analogous manner to the compound described in Example X4.

$^1H$ nmr $(CDCl_3)$ ppm.

1.1 (6H, d); 1.25 (4H, br s); 1.55 (4H, br s); 2.45-3.1 (8H, m); 4.6 (2H, m); 4.8 (2H, s); 6.5 (2H, d); 6.8-7.4 (18H, m).

Example X58

[R-(R*, R*)]-4,4'-(1,6-Hexanediyl)bis [α-methylbenzeneethanamine], dihydrochloride.

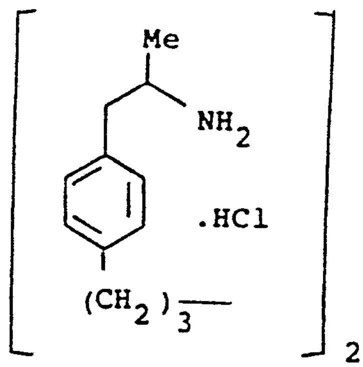

[R-(R*, R*)]-4,4'-(1,6-Hexanediyl)bis [α-methylbenzeneethanamine], dihydrochloride was prepared in an analogous manner to the compound described in Example X54.

$^1H$ nmr, ($d_6$-DMSO); ppm.

1.0 (6H, d); 1.0-1.7 (8H, m); 2.6-3.6 (10H, m) 7.0 (8H, s); 8.2 (6H, br).

Example X59

[R,R,R,R]-4,4'-(1,6-hexanediyl)bis[α-methyl-N-(α-methylbenzyl)benzeneethanamine, dihydrochloride

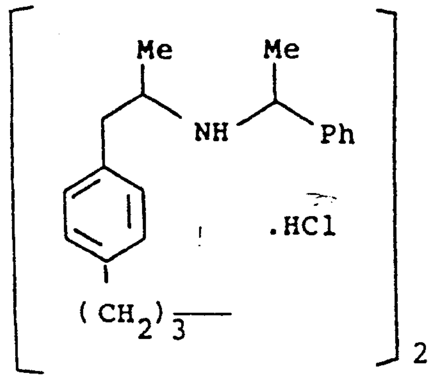

[R,R,R,R]-4,4'-(1,6-hexanediyl)bis[α-methyl-N-(α-methylbenzyl)benzeneethanamine, dihydrochloride was prepared in an analogous manner to the compound described in Example X55.

$[\alpha]_D^{25}$: +30.46° (DMSO).

$^1H$ nmr, ($d_6$-DMSO); ppm.

1.05 (6H, d); 1.25 (4H, br s); 1.5 (4H, br s); 1.6 (6H, d); 2.45-2.6 (4H, m); 2.8 (2H, br s); 3.4 (4H, m) 4.6 (2H, m); 6.8 (4H, d); 7.05 (4H, d); 7.25-7.55 (6H, m); 7.7 (4H, d); 9.3 (2H, br); 10.2 (2H, br).

Example X60

1,1'-[1,6-Hexanediylbis(4,1-phenylene)]bis[2-propanone]

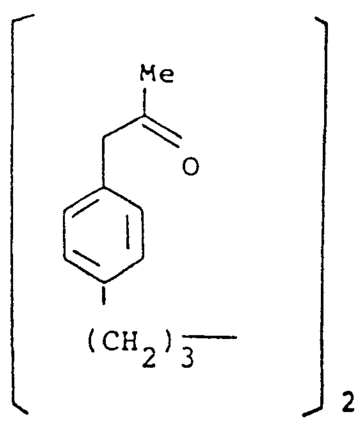

A solution of 1,1'-[(1,6-dihydroxy-1,6-hexanediyl)bis(4,1-phenylene)]bis[2-propanone, ethylene ketal] (6.14g, 13.1 mmol) in methanol (250ml) was hydrogenated at 100 psi in the presence of 10% Pd/C (0.6g) for 8 h then filtered through a pad of celite and concentrated in vacuo. The residues were dissolved in acetone (80ml) and hydrochloric acid (2N), (80ml) added and the solution stirred for 16 h after which time it was extracted with ethyl acetate. The organic layers were washed with saturated sodium bicarbonate solution, brine, dried ($MgSO_4$) and concentrated to provide 1,1'-[1,6-hexanediylbis(4,1-phenylene)]bis[2-propanone], (3.41g, 90%).

$^1H$ nmr ($CDCl_3$) ppm.

1.0-1.8 (8H, m); 2.25 (6H, s); 2.6 (4H, br t); 3.7 (4H, s); 7.15 (8H, s).

Example X61

1,1'-[(1,6-Dihydroxy-1,6-hexanediyl)bis(4,1-phenylene)]bis[2-propanone, ethylene ketal]

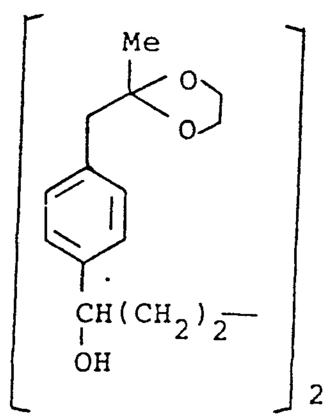

1,4-dibromobutane (6.0g, 27.8 mmol) was added slowly to a suspension of magnesium turnings (1.47g, 61.3 mmol)

in tetrahydrofuran (80ml) at such a rate as to maintain a gentle reflux. After completion of the addition the mixture was refluxed for an additional 3 h, cooled to $^{o}C$ and 4-(2-oxopropyl)benzaldehyde, ethylene ketal (11.41g, 55.4 mmol) added. The solution was stirred for 16 h and then saturated ammonium chloride solution (80ml) was added with stirring. The mixture was extracted with ether and the organic fractions dried ($MgSO_4$) and concentrated to an oil. Chromatographic purification on silica (gradient elution petrol-1:1 petrol/ether) gave 1,1'-[(1,6-Dihydroxy-1,6-hexanediyl) bis(4,1-phenylene)]bis[2-propanone, ethylene ketal], (6.19g, 48%).

<u>$^{1}H$ nmr ($CDCl_3$) ppm.</u>

1.2 (6H, s); 1.2-2.05 (8H, m); 2.85 (4H, br s); 3.8 (10H, m); 4.6 (2H, m); 7.2 (8H, m).

<u>Example X62</u>

<u>[R,R,R,R]-N,N'-[1,5-Pentanediyl]bis[oxy-4,1-phenylene (1-methyl-2,1-ethanediyl)]]bis[3-chloro-α-hydroxybenzeneacetamide].</u>

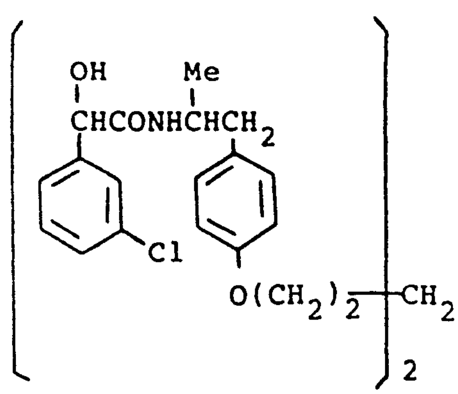

[R,R,R,R]-N,N'-[1,5-Pentanediyl]bis[oxy-4,1-phenylene (1-methyl-2,1-ethanediyl)]]bis[3-chloro-α-hydroxybenzeneacetamide] was prepared in an analogous

manner to the compound described in Example X4.

$^1$H nmr ($CDCl_3$) ppm.

1.1 (6H, d); 1.65 (2H, m); 1.8 (4H, m); 2.6 (4H, d); 3.95 (4H, t); 4.2 (2H, m); 4.8 (2H, s); 5.95 (2H, d); 6.7 (4H, d); 6.8 (4H, d); 7.2-7.45 (8H, m).

Example X63

[R-(R*,R*)]-4,4'-(1,5-Pentanediylbis(oxy)]bis [α-methylbenzeneethanamine]

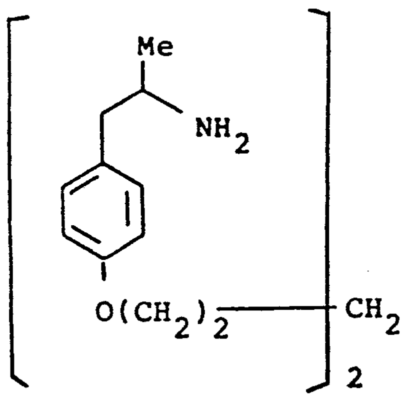

[R-(R*,R*)]-4,4'-(1,5-Pentanediylbis(oxy)]bis [α-methylbenzeneethanamine] was prepared in an analogous manner to the compound described in Example X5.

$^1$H nmr ($CDCl_3$) ppm.

1.15 (6H, d); 1.65 (2H, m); 1.8 (8H, m); 2.5 (2H, dd) 2.7 (2H, dd); 3.15 (2H, m); 3.95 (4H, t); 6.8 (4H, d); 7.1 (4H, d).

Example X64.

[R,R,R,R]-4,4'-(1,5-Pentanediyl)bis[α-methyl-N-(α-methylbenzyl)benzeneethanamine, dihydrochloride

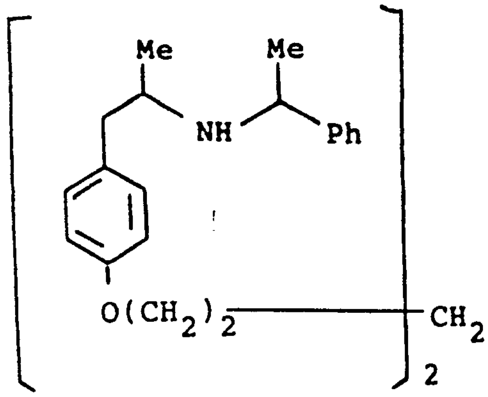

[R,R,R,R]-4,4'-(1,5-Pentanediyl)bis[α-methyl-N-(α-methylbenzyl)benzeneethanamine, dihydrochloride was prepared in an analogous manner to the compound described in Example X55.

$[\alpha]_D^{25}$: +23.3° (DMSO).

$^1H$ nmr, ($d_6$-DMSO); ppm.

1.15 (6H, d); 1.35-1.95 (10H, m); 2.85 (2H, m); 3.35 (4H, m); 3.9 (4H, t); 4.6 (2H, m); 6.85 (8H, q); 7.5 (6H, m); 7.75 (4H, m); 9.5 (2H, m); 10.25 (2H, m).

Examples X65

[R,R,R,R ]-N,N'-[Oxybis[2,1-ethanediyloxy-4, 1-phenylene(1-methyl-2,1-ethanediyl)]]bis[3-chloro-α-hydroxybenzeneacetamide].

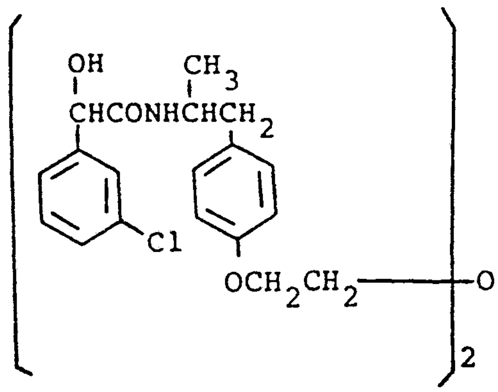

[R,R,R,R]-N,N'-[Oxybis[2,1-ethanediyloxy-4, 1-phenylene(1-methyl-2,1-ethanediyl)]]bis[3-chloro-α-hydroxybenzeneacetamide] was prepared by an analogous procedure to that described in Example X4.

$^1H$ nmr ($CDCl_3$) ppm.

1.1 (6H; d); 2.2-3.8 (10H, complex, 4H exchanges) 3.7-4.3 (8H, complex); 4.8 (2H,); 6.7 (4H, d); 6.9 (4H, d); 7.2 (8H, complex).

Examples X66

[R-(R*, R*)]-4,4'-Oxybis[(2,1-ethanediyloxy)]bis [α-methylbenzeneethanamine], dihydrochloride.

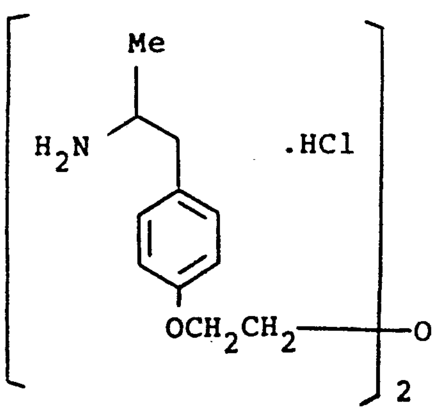

[R-(R*, R*)]-4,4'-Oxybis[(2,1-ethanediyloxy)]bis[α-methylbenzeneethanamine], dihydrochloride, mp. 77-81°C (chloroform-ether) was prepared in an analogous manner to the compound described in Example X5.

$[\alpha]_D^{25}$ +15.2° (Ethanol).

<u>$^1$H nmr, ($d_6$-DMSO); ppm.</u>

1.15 (6H, d); 2.2-4 (6H, complex); 3.8 (4H, complex), 4.15 (4H, complex); 6.9 (4H, d); 7.1 (4H, d); 7.6-8.8 (6H, broad s, exchanges).

<u>Example X67.</u>

<u>[R,R,R,R]-4,4'-Oxybis(2,1-ethanediyloxy]bis[α-methyl-N-(α-methylbenzyl)benzeneethanamine], dihydrochloride</u>

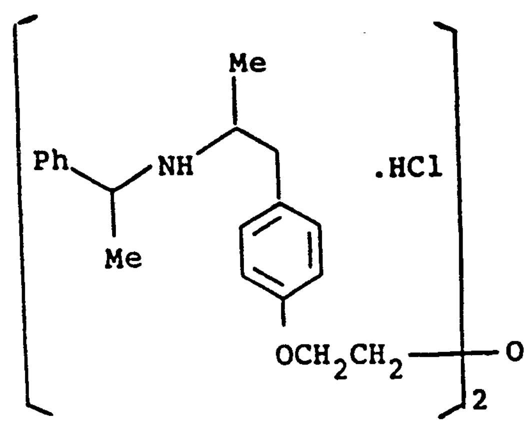

[R,R,R,R]-4,4'-Oxybis(2,1-ethanediyloxy]bis[α-methyl-N-(α-methylbenzyl)benzeneethanamine], dihydrochloride was prepared in an analogous manner to the compound described in Example X6.

Example X68.

1,1'-Oxybis[2,1-ethanediyloxy-4,1-phenylene]bis [2-propanone].

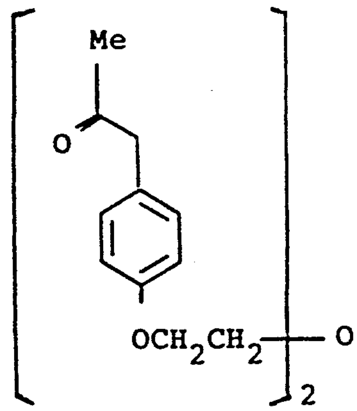

1,1'-Oxybis[2,1-ethanediyloxy-4,1-phenylene]bis [2-propanone], mp. 64-65°C, was prepared in an analogous manner to the compound described in Example X10.

$^1$H nmr ($CDCl_3$) ppm.

2.2 (6H, s); 3.6 (4H, s); 3.85 (4H, complex); 4.1 (4H, complex); 6.8 (4H, d); 7.1 (4H, d).

Example X69.

1,1'-Oxybis[2,1-ethanediyloxy[4-(2-nitro-1-propenyl) benzene].

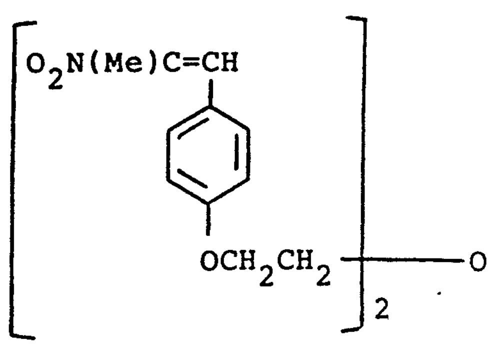

1,1'-Oxybis[2,1-ethanediyloxy[4-(2-nitro-1-propenyl) benzene], mp. 124°C, was prepared in an analogous

manner to the compound described in Example X9.

$^1H$ nmr, ($d_6$-DMSO); ppm.

2.4 (6H, s); 3.85 (4H, complex); 4.2 (4H, complex); 7.1 (4H, d); 7.55 (4H, d); 8.05 (2H, s).

Example 70.

4,4'-Oxybis[2,1-ethanediyloxybenzaldehyde].

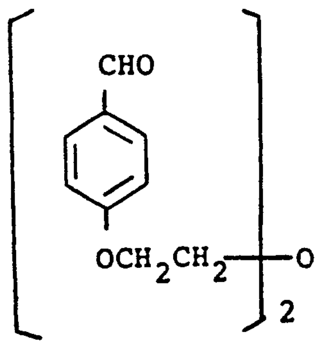

Sodium hydride (4g, 60% dispersion in oil) was added portionwise to a stirred solution of 4-hydroxybenzaldehyde (12.2g) in dry dimethylformamide (100ml) under nitrogen and at ambient temperature. When gas evolution ceased a solution of 2,2'-dichlorodiethylether (7.5g) in dry dimethylformamide (50ml) was added slowly. The mixture was heated at 120°C for 3 h, cooled and poured into cold water. The solid was filtered, washed with water, dried under vacuum and crystallised from IMS to give 4,4'-Oxybis[2,1-ethanediyloxybenzaldehyde], mp. 139-40°C.

$^1H$ nmr ($CDCl_3$) ppm.

3.65 (4H, complex); 4.05 (4H, complex), 7.0 (4H, d); 7.8 (4H, d); 9.8 (2H, s).

Example X71.

[R,R,R,R]-2,2'-[1,6-Hexanediylbis[oxy-4,1-phenylene (1-methyl-2,1-ethanediyl)iminomethylene]] bis[3,5-dibenzyloxy-benzenemethanol], dihydrochloride.

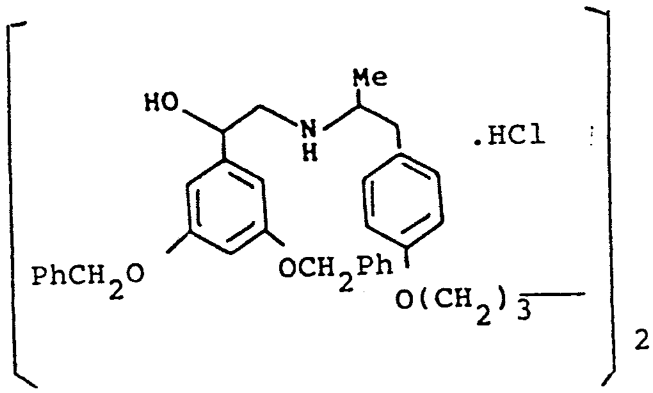

[R,R,R,R]-2,2'-[1,6-Hexanediylbis[oxy-4,1-phenylene (1-methyl-2,1-ethanediyl)iminomethylene]]bis [3,5-dibenzyloxy-benzenemethanol], dihydrochloride was prepared in an analogous manner to that described in Example 3.

$^1H$ nmr, ($d_6$-DMSO); ppm.

1.10 (6H, d); 1.45 (4H, m); 1.71 (4H, m) 2.55-2.70 (2H, m); 2.95-3.5 (8H, m); 3.93 (4H, t); 5.04 (2H, dd), 5.09 (8H, s);6.25 (2H, broad, replaceable by $D_2O$); 6.60-6.75 (6H, m); 6.87 (4H, d); 7.14 (4H, d); 7.25-7.5 (20H, m); 8.83 (2H, broad, replaceable by $D_2O$); 9.48 (2H, broad, replaceable by $D_2O$).

Example X72.

N,N'-[1,6-Hexanediylbis[oxy-4,1-phenylene(1-methyl-2,1-ethanediyl)]]bis[3,5-dibenzyloxy-α-hydroxybenzene-acetamide].

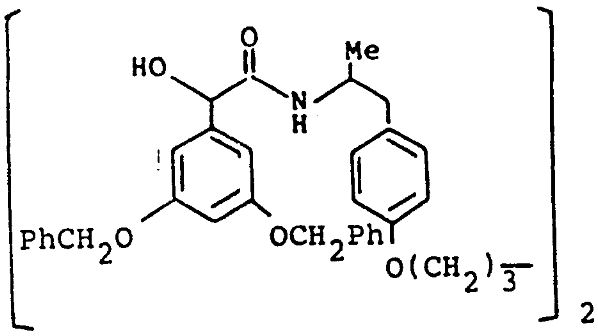

N,N'-[1,6-Hexanediylbis[oxy-4,1-phenylene(1-methyl-2,1-ethanediyl)]]bis[3,5-dibenzyloxy-α-hydroxybenzeneacetamide] was prepared in an analogous manner to that described in example 4 using (R)-3,5-dibenzyloxymandelic acid in place of (R)-3-chloromandelic acid.

$^1H$ nmr, ($CDCl_3$) ppm.

1.05 (6H, d); 1.2-2.0 (8H, m); 2.55 (4H, d); 3.8 , (4H, t), 4.0-4.3 (2H, m); 4.8 (2H, s) 4.95 (8H, s); 6.0 (2H, bd, replaceable by $D_2O$),; 6.6-7.0 (16H, m includes 2H replaceable by $D_2O$); 7.35 (20H, s).

Example X73.

[R,R,R,R]-4,4'-(1,7-Heptanediylbis(oxy)]bis[N-(α-methylbenzyl)-α-methylbenzeneethanamine], dihydrochloride.

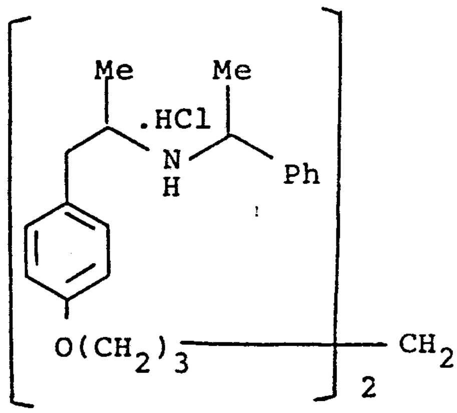

[R,R,R,R]-4,4'-(1,7-Heptanediylbis(oxy)]bis[N-(α-methylbenzyl)-α-methylbenzeneethanamine], dihydrochloride was prepared using [R(R*, R*)]-4-[2-methyl-2-[(α-methylbenzyl)amino]ethyl]phenol, hydrochloride (5.0g) and 1,7-dibromoheptane (1.61g) in an analogous procedure to that described in Example X34.

$^1H$ nmr, (DMSO), ppm.

1.1 (6H, d); 1.3 (6H, m); 1.5-1.75 (10H, m); 2.5 (2H, t); 2.8 (2H, broad s); 3.3 (2H, m); 3.85 (4H, m); 4.6 (2H, broad s); 6.8 (4H, d); 6.9 (4H, d); 7.4 (6H, m); 7.7 (4H, m); 9.3 (2H broad s, exchanges $D_2O$); 10.1 (2H, broad s exch $D_2O$).

Example X74.

[R-(R*, R*)]-4,4'-[(1,7-Heptanediylbis(oxy)]bis-[α-methylbenzeneethanamine], dihydrochloride.

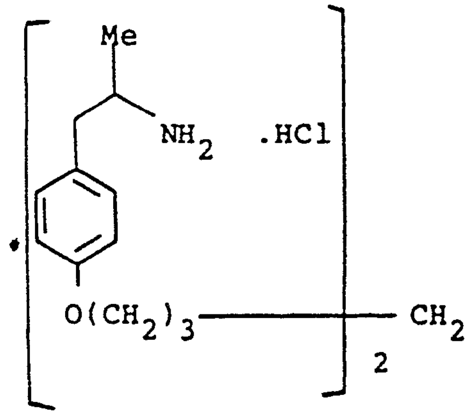

[R-(R*, R*)]-4,4'-[(1,7-heptanediylbis(oxy)]bis-[α-methylbenzeneethanamine], dihydrochloride was prepared using [R,R,R,R]-4,4'-[1,7-heptanediylbis(oxy)] bis[N-(α-methylbenzyl)-α-methylbenzeneethanamine], dihydrochloride (5g) in an analogous fashion to that described in Example X33.

$^1H$ nmr, (DMSO-$d_6$), ppm.

1.2 (6H, d); 1.3-1.5 (6H, m); 1.7 (4H, m); 2.6 (2H, dd); 3.0 (2H, dd); 3.2-3.5 (2H, m); 3.9 (4H, t); 6.8 (4H, d); 7.15 (4H, d); 8.0-8.5 (6H, broad s, exchanges $D_2O$).

Example 75

[R,R,R,R]-N,N'-[1,7-Heptanediylbis[oxy-4,1-phenylene (1-methyl-2,1 ethanandiyl)]]bis[3-chloro-α-hydroxy-benzeneacetamide].

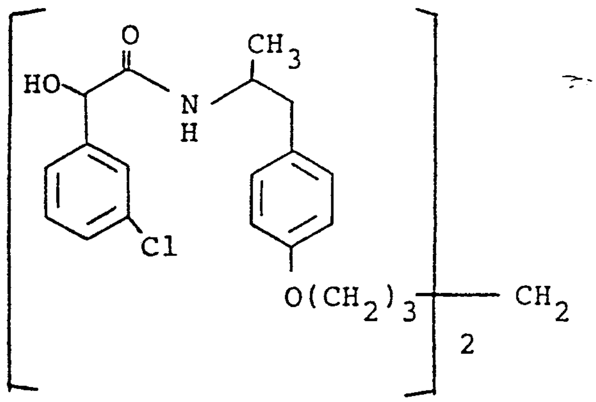

[R,R,R,R]-N,N'-[1,7-heptanediylbis[oxy-4,1-phenylene (1-methyl-2,1 ethanandiyl)]]bis[3-chloro-α-hydroxybenzeneacetamide] was prepared using [R-(R*,R*)]-4,4'-[1,7-heptanediylbis(oxy)]bis[α-methylbenzeneethanamine] (2.5g) and (R)-3-chloromandelic acid (2.02g), by an analogous procedure to that described in Example X4.

$^1H$ nmr ($CDCl_3$), ppm.

1.1 (6H, d); 1.3-2.1 (10H, m); 2.6-2.9 (4H, m); 3.8-4.4 (6H, m); 4.5-4.7 (2H, m); 4.7-5.0 (2H, m) 6.3-6.5 (2H, d); 6.6-7.1 (8H, m); 7.1-7.5 (8H, m).

0233686

## DEMONSTRATION OF EFFECTIVENESS OF COMPOUNDS

### Effect on Energy Expenditure

The effect of the compounds on the energy expenditure of rats was demonstrated by means of the following procedure:

Male Sprague-Dawley rats each weighing between 170-200g were deprived of food for 16 hours before, and during the experiment. Water was provided *ad lib* at all times. The compounds were administered orally in water to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dosing from the volume of air leaving the boxes and its oxygen content, following the principles described by J.B. de V. Weir, J. Physiol. (London) 109, 1-9 (1949). The results are expressed as a percentage of the rate of energy expenditure of the rats dosed with water. Results are given in Table 1.

| Compound of Example No. | Dose µmol/Kg.p.o | Mean Energy Expenditure (0-3h) | (3-6h) | (0-21h) |
|---|---|---|---|---|
| 1 | 5 | 132 | 133 | 127 |
| 2 | 5 | 110 | 133 | 117 |
| 3 | 10 | 122 | 130 | 126 |
| 4 | 100 | 117 | 115 | 115 |
| 5 | 10 | 112 | 114 | 109 |
| 6 | 10 | 131 | 124 | 124 |
| 7 | 10 | 120 | 112 | 114 |
| 8 | 20 | 107 | 110 | 111 |

| Compound of Example No. | Dose µmol/Kg.po. | Mean Energy Expenditure (0-3h) | (3-6h) | (0-21h) |
|---|---|---|---|---|
| 9 | 5 | 118 | 112 | 111 |
| 10 | 5 | 145 | 123 | 117 |
| 11 | 3 | 122 | 121 | 121 |
| 12 | 3 | 113 | 106 | 110 |
| 13 | 30 | 110 | 116 | 115 |
| 14 | 3 | 118 | 113 | 114 |
| 15 | 20 | 111 | 115 | 118 |
| 16 | 20 | 116 | 112 | 109 |
| 17 | 100 | 106 | 108 | 109 |
| 18 | 100 | 127 | 116 | 117 |
| 19 | 20 | 107 | 106 | 106 |
| 20 | 3 | 115 | 119 | 112 |
| 21 | 3 | 128 | 125 | 119 |
| 22 | 100 | 115 | 117 | 107 |
| 23 | 10 | 110 | 117 | 112 |

2. <u>Hypoglycaemic Activity</u>

Female CFLP mice, weighing approximately 25g, were fasted for 24 hours prior to the study. The compounds under study were administered orally as an aqueous solution to each of 6 mice. 30 minutes later a blood sample (20µl) was obtained from the tail for the analysis of blood glucose. Immediately after taking this blood sample, glucose (1g/kg body weight) was administered subcutaneously to each mouse. 6 mice were given water as a control. Blood samples were then obtained from each mouse at 30 minute intervals for 120 minutes.

Compounds that produced a significant ($p>0.05$) reduction of blood glucose, compared with control mice given water, at any time interval were considered

active. The area under the blood glucose curve over the 2 hour period after the administration of the glucose was calculated for each compound and compared with the value for control animals.

| Compounds of Example No. | Dose µmol/Kg p.o. | % reduction in area under blood glucose curve |
|---|---|---|
| 1 | 12.5 | 43 |
| 3 | 1.0 | 55 |
| 6 | 3 | 30 |
| 7 | 2.5 | 17 |
| 13 | 3 | 34 |
| 16 | 3 | 8 |
| 19 | 10 | 11 |
| 20 | 0.1 | 51 |

Claims

1. A compound of formula (I):

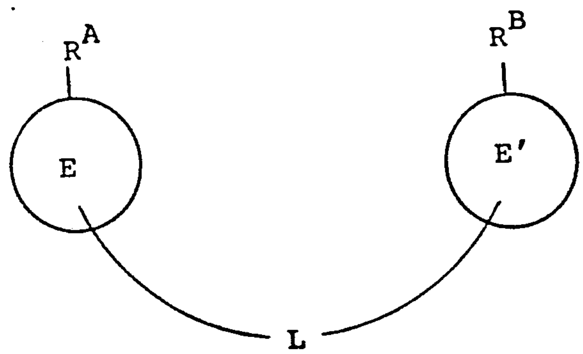

(I)

or a pharmaceutically acceptable salt, ester or amide thereof,

characterized in that $R^A$ represents a moiety of formula (a):

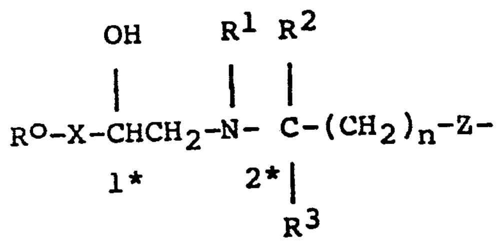

(a)

and $R^B$ represents a moiety of formula (b):

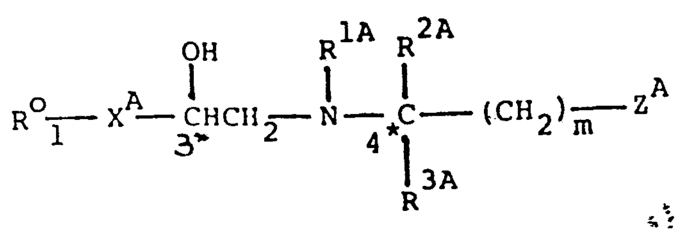

(b)

wherein

$R^o$ and $R^o_1$ each independently represents a substituted or unsubstituted aryl group or a substituted or unsubstituted benzofuranyl group,

X and $X^A$ each independently represents a bond or $-O-CH_2-$ ,

$R^1$ represents a hydrogen atom or a moiety:

$$\begin{array}{c} OH \\ | \\ R^o\text{-}X\text{-}\underset{5*}{C}HCH_2\text{-} \end{array}$$ wherein X and $R^o$ are as defined above;

$R^{1A}$ represents a hydrogen atom or a moiety:

$$\begin{array}{c} OH \\ | \\ R^o_1\text{-}X^A\text{-}\underset{6*}{C}HCH_2\text{-} \end{array}$$ wherein $X^A$ and $R^o_1$ are as defined above;

$R^2$, $R^3$, $R^{2A}$ and $R^{3A}$ each independently represent a hydrogen atom or an alkyl group,

Z and $Z^A$ each independently represent a bond or a moiety $-CH_2-O-$,

n and m each independently represent an integer 1 or 2;

E and E′ each independently represent substituted or unsubstituted aryl; and L represents a linking moiety.

2. A compound according to claim 1, wherein E or E′ represents a substituted or unsubstituted phenylene or naphthylene group.

3. A compound according to claim 1 or claim 2, wherein $R^o$ and $R^o_1$ each independently represent a moiety of formula (c):

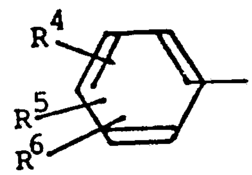

(c)

wherein $R^4$, $R^5$ and $R^6$ each independently represent hydrogen, halogen, alkyl, alkenyl, alkynyl, phenyl, alkoxy, trifluoromethyl, hydroxyalkyl, hydroxy, alkoxy amino, nitro, nitrile or carboxy.

4. A compound according to any one of claims 1 to 3, wherein $R^4$, $R^5$ and $R^6$ each independently represent hydrogen, halogen, trifluoromethyl, amino or hydroxy.

5. A compound according to any one of claims 1 to 4, wherein L comprises a substituted or unsubstituted hydrocarbon; or a chain of at least two atoms in length comprising at least one hetero atom selected from oxygen or substituted or unsubstituted nitrogen or sulphur; or L represents oxygen, an amino group or $SO_z$ wherein z is zero or 1 or 2.

6. A compound according to any one of claims 1 to 5, wherein L is a substituted or unsubstituted alkylene, alkenylene or alkynylene group.

7. A compound according to any one of claims 1 to 5, wherein L is a chain of from 2 to 30 atoms in length comprising at least one hetero atom selected from oxygen, nitrogen or sulphur and a substituted or unsubstituted alkylene, alkenylene or alkynylene group, preferably an alkylene group.

8. A compound according to any one of claims 1 to 5, wherein the moiety L comprises

-O-, -S-, -SO-, $-SO_2-$, -C(O)-, -CR(OH)-, -CO.O-, -CON(R′)- or -N(R′)-, wherein R represents hydrogen, alkyl or hydroxyalkyl and R′ represents hydrogen or alkyl, as part of the chain of from 2 to 30 atoms, especially as part of a chain also comprising a substituted or unsubstituted alkylene, alkenylene or alkynylene groups.

9. A compound according to any one of claims 1 to 8, wherein L is of formula $-X^1- X^2- X^3-$ wherein $X^1$ and $X^3$ each

independently represent a bond, -C(O)-, RCOH, -CO.O-

$-OX^{2A}CO_2-$, -CO.N(R′)-, $-X^{2A}CO.N(R')-$, $-OX^{2A}CO.N(R')-$, $-OX^{2B}O-$, $-X^2N(R')-$, $-OX^{2A}N(R')-$, O, S, $-SO_2$, -N(R′)-, $RC(OH)X^{2A}-$ or $-N(R')X^{2B}O-$; wherein R and R′are as defined above, $X^{2A}$ represents alkylene and $X^{2B}$ represents $C_{2-10}$ alkylene; and $X^2$ represents a substituted or unsubstituted alkylene, alkenylene, alkynylene or a moiety $-X^4-Z^1-X^5-$ wherein $X^4$ and $X^5$ each independently represent a bond, $C_{1-6}$ alkylene, $C_{2-6}$ alkylene or $C_{2-6}$ alkynylene, and

wherein $Z^1$ represents -O-,-S,-SO,$-SO_2$ or-NR′ wherein R′ is defined above.

10. A compound according to any one of claims 1 to 9, wherein L represents:

$-O(CH_2)_yCONH-(CH_2)_x-NHCO(CH_2)_yO-$,

$-O(CH_2)_yCONH-(CH_2)_x-NH(CH_2)_xO-$,

$-O(CH_2)_xNH-(CH_2)_x-NH(CH_2)_xO-$,

$-CONH(CH_2)_xNHCO-$,

$-(CH_2)_yCONH-(CH_2)_x-NHCO(CH_2)_y-$,

$-(CH_2)_yCONH-(CH_2)_x-NH(CH_2)_y-$,

$-(CH_2)_yNH-(CH_2)_x-NH(CH_2)_y-$,

$-O-(CH_2)_{y+1}-O-$,

$-(CH_2)_y-O-(CH_2)_y-$,

$-O(CH_2)_{y+1}-O-(CH_2)_{y+1}-O-$,

$-CO.O-(CH_2)_{y+1}-O.OC-$,

$-(CH_2)_y-$,

|

$-C(OH)-CH_2OH$,

$-O-$, or

$SO_z$; wherein

x represents an integer from 2 to 6;
y represents an integer from 1 to 10; and
z represents zero or an integer 1 or 2.

11. A compound according to any one of claims 1 to 10, wherein L represents $-O(CH_2)_{y+1}O-$, wherein y is an integer from 1 to 10.

12. A compound according to any one of claims 1 to 11, wherein L represents $-O(CH_2)_6O-$

13. A compound according to formula (I) selected from the list consisting of:

[R,R,R,R]-N,N′-(1,2-ethanediyl)bis[2-[4-[2-[(2-(3-chlorophenyl)-2-hydroxyethyl)amino]propyl]phenoxy] acetamide];

[R,R,R,R]-α,α'[1,2-ethanediylbis(imino-2,1-ethanediyloxy-4,1-phenylene (1-methyl-2,1-ethanediyl) iminomethylene]]bis[3-chlorobenzenemethanol];

[R,R,R,R]-α,α'[1,6-hexanediylbis[oxy-4,1-phenylene(1-methyl-2,1-ethanediyl)iminomethylene]]bis[3-chlorobenzene methanol],

[R,R,R,R]-α,α'-[oxybis[4,1-phenylene(1-methyl-2,1-ethanediyl)iminomethylene]]bis[3-chlorobenzenemethanol];

[R,R,R,R]-α,α'-[methylenebis[4,1-phenylene(1-methyl-2,1-ethanediyl)iminomethylene]]bis[3-chlorobenzenemethanol];

[R,R,R,R]-α,α'-[sulphonylbis[4,1-phenylene(1-methyl-2,1-ethanediyl)iminomethylene]]bis[3-chlorobenzenemethanol];

[R,R,R,R]-1,1-di[4-[2-[[2-(3-chlorophenyl)-2-hydroxyethyl]amino]propyl]phenyl]-1,2-ethanediol;

[R,R,R,R]-α,α'-[1,8-octanediylbis[oxy-4,1-phenylene (1-methyl-2,1-ethanediyl)iminomethylene]]bis[3-chlorobenzenemethanol];

1,2-ethanediyl di[4-[2-[[2-hydroxy-2-(3-trifluoromethyl)phenylethyl]amino]propyl]benzoate];

1,2-ethanediyl di[4-[2-[[2-hydroxy-2-phenylethyl]amino] propyl] benzoate];

[R,R,R,R,]-α,α'[1,4-butanediylbis[oxy-4,1-phenylene (1-methyl-2,1-ethanediyl)iminomethylene]]bis[3-chlorobenzenemethanol], dihydrochloride.

[R,R,R,R,]-α,α'[1,3-propanediylbis[oxy-4,1-phenylene (1-methyl-2,1-ethanediyl)iminomethylene]]bis[3-chlorobenzenemethanol];

[R,R,R,R,]-α,α'[1,9-nonanediylbis[oxy-4,1-phenylene (1-methyl-2,1-ethanediyl)iminomethylene]]bis[3-chlorobenzenemethanol];

[R,R,R,R,]-3-chloro-α-[[[[2-[4-[6-[4-[2-[2-(4-hydroxyphenyl)-2-hydroxyethyl]amino]propyl]phenoxy] hexyloxy]phenyl]-1-methylethyl]amino]methyl]-benzenemethanol;

[R,R,R,R,]-α,α'-[1,2-ethanediylbis[iminomethylene-4,1-phenylene(1-methyl-2,1-ethanediyl)iminomethylene]]bis [3-chlorobenzenemethanol;

[R,R,R,R]-α,-α'-[1,4-butanediylbis[iminomethylene-4,1-phenylene(1-methyl-2,1-ethanediyl)iminomethylene]]bis [3-chlorobenzenemethanol;

[R,R,R,R]-α,α'-[1,6-hexanediylbis[iminomethylene-4,1-phenylene (1-methyl-2,1-ethanediyl)iminomethylene]] bis[3-chlorobenzenemethanol];

[R,R,R,R]-α,α'-[1,2-ethanediylbis[4,1-phenylene (1-methyl-2,1-ethanediyl)iminomethylene]] bis[3-chlorobenzenemethanol];

[R,R,R,R]-α,α'-[1,6-hexanediylbis[4,1-phenylene (1-methyl-2,1-ethanediyl)iminomethylene]] bis[3-chlorobenzenemethanol];

[R,R,R,R]-α,α'-[1,5-pentanediyl bis[oxy-4,1-phenylene (1-methyl-2,1-ethanediyl)iminomethylene]] bis[3-chlorobenzenemethanol];

[R,R,R,R]-α,α'-[oxybis[2,1-ethanediyloxy-4,1-phenylene (1-methyl-2,1-ethanediyl)iminomethylene]] bis[3-chlorobenzenemethanol],

[R,R,R,R]-5,5'-[1,6-hexanediylbis[oxy-4-1-phenylene (1-methyl-2,1-ethanediyl)imino(1-hydroxy-2,1-ethanediyl)]]bis[benzene-1,3-diol]; and

[R,R,R,R]-α,α'[1,7-heptanediylbis[oxy-4,1-phenylene-(1-methyl-2,1-ethanediyl)iminomethylene]]bis[3-chlorobenzenemethanol]; or a pharmaceutically acceptable acid addition salt thereof.

14. A process for preparing a compound of formula (I) characterized in that:

(A) a compound of formula (III):

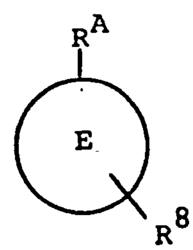

(II)

is reacted with with a compound of formula (IV):

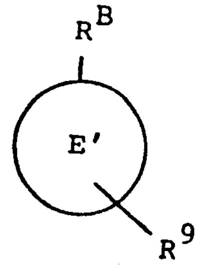

(IV)

wherein $R^A$ and $R^B$ are as defined in relation to formula (I) or may be protected forms thereof E and E′ are as defined in relation to formula (I); and either $R^8$ represents a nucleophilic group and $R^9$ represents a moiety-$L^1$ - $R^x$ wherein $R^x$ represents a leaving group, $L^1$ representing a moiety such that -$R^8$-$L^1$- represents the linking group L; or $R^8$ represents the above defined moiety-$L^1$ - $R^x$, $R^9$ represents a nucleophilic group and $L^1$ is a moiety such that -$R^9$-$L^1$- represents L;

or

(B) a compound of formula (IX) is converted to a compound of formula (I) or a pharmaceutically acceptable salt, ester or amide thereof,

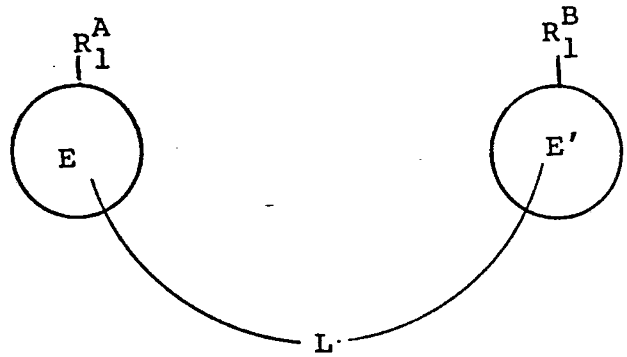

(IX)

wherein L E and E′ are as defined in relation to formula (I), $R^A{}_1$ represents $R^A$ as defined in relation to formula (I) or a moiety convertible to a moiety $R^A$; and $R^B{}_1$ represents $R^B$ as defined in relation to formula (I) or a moiety convertible to a group $R^B$, providing that $R^A{}_1$ is not $R^A$ when $R^B{}_1$ is $R^B$;

by, where appropriate;

(a) converting any group $R^A{}_1$ to $R^A$: and/or

(b) converting any group $R^B{}_1$ to $R^B$;

and thereafter if necessary carrying out one or more of the following steps:

(i) removing any protecting group;

(ii) converting a compound of formula (I) into a further compound of formula (I);

(iii) converting a salt of formula (I) into a free compound of formula (I);

(iv) preparing a pharmaceutically acceptable ester or amide of a compound of formula (I);

(v) preparing a pharmaceutically acceptable salt of a compound of formula (I) or an ester or amide thereof.

15. A pharmaceutical composition comprising a compound of the general formula (I), or a pharmaceutically acceptable salt, ester or amide thereof, and a pharmaceutically acceptable carrier therefor.

16. A compound of the general formula (I), or a pharmaceutically acceptable salt, ester or amide thereof, for use in the treatment of obesity and/or hyperglycaemia in human or non-human animals.

17. A compound of the general formula (I), or a pharmaceutically acceptable salt, ester or amide thereof, for use in the treatment of atherosclerosis in humans.

18. The use of a compound of formula (I), or a pharmaceutically acceptable salt, ester or amide thereof, for the manufacture of a medicament for the treatment of obesity and/or hyperglycaemia in humans and non-human animals.

19. A method for increasing weight gain and/or improving the feed utilisation efficiency and/or increasing lean body mass and/or decreasing birth mortality rate and increasing the post-natal survival rate; of livestock, which method comprises the administration to livestock of an effective non-toxic amount of a compound of formula (I) or a veterinarily acceptable salt, ester or amide thereof.

20. A veterinarily acceptable premix formulation comprising a compound of formula (I), or a veterinarily acceptable salt, ester or amide thereof, and a veterinarily acceptable carrier therefor.

Claims

1. A process for preparing a compound of formula (I):

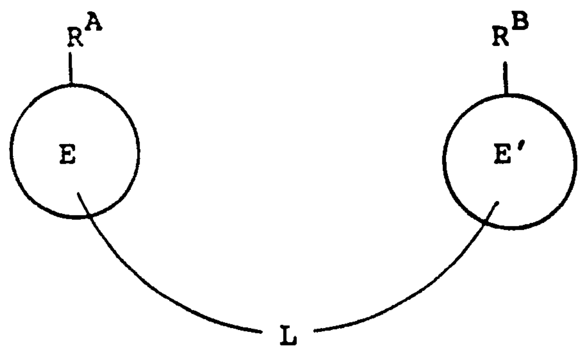

(I)

or a pharmaceutically acceptable salt, ester or amide thereof,

wherein $R^A$ represents a moiety of formula (a):

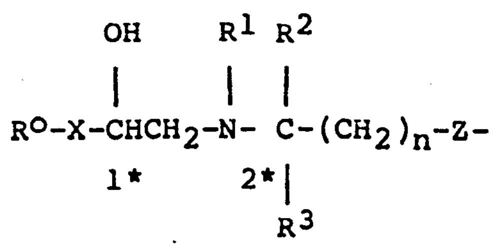

(a)

and $R^B$ represents a moiety of formula (b):

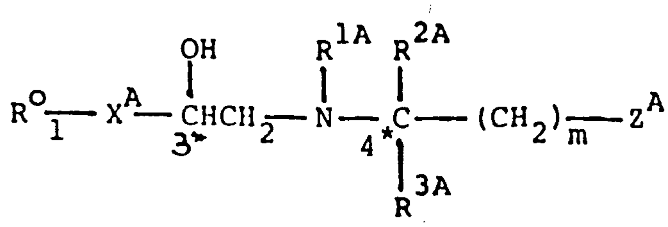

(b)

wherein

$R^{o}$ and $R^{o}_{1}$ each independently represents a substituted or unsubstituted aryl group or a substituted or unsubstituted benzofuranyl group,

X and $X^{A}$ each independently represents a bond or $-O-CH_2-$ ,

$R^1$ represents a hydrogen atom or a moiety:

$$\overset{\displaystyle OH}{\underset{5*}{R^{o}-X-CHCH_2-}}$$ wherein X and $R^{o}$ are as defined above;

$R^{1A}$ represents a hydrogen atom or a moiety:

$$\overset{\displaystyle OH}{\underset{6*}{R^{o}_{1}-X^{A}-CHCH_2-}}$$ wherein $X^{A}$ and $R^{o}_{1}$ are as defined above;

$R^2$, $R^3$, $R^{2A}$ and $R^{3A}$ each independently represent a hydrogen atom or an alkyl group,

Z and $Z^{A}$ each independently represent a bond or a moiety $-CH_2-O-$,

n and m each independently represent an integer 1 or 2;

E and E′ each independently represent substituted or unsubstituted aryl; and L represents a linking moiety;

(A) from a compound of formula (III):

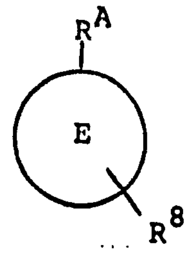

(III)

by reaction with a compound of formula (IV):

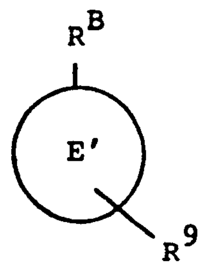

(IV)

wherein $R^A$ and $R^B$ are as defined in relation to formula (I) or may be protected forms thereof E and E′are as defined in relation to formula (I); and either $R^8$ represents a nucleophilic group and $R^9$ represents a moiety-$L^1$ - $R^x$ wherein $R^x$ represents a leaving group, $L^1$ representing a moiety such that -$R^8$-$L^1$- represents the linking group L; or $R^8$ represents the above defined moiety-$L^1$ - $R^x$ and $R^9$ represents a nucleophilic group and $L^1$ is a moiety such that -$R^9$-$L^1$- represents L; or

(B) from a compound of formula (IX)

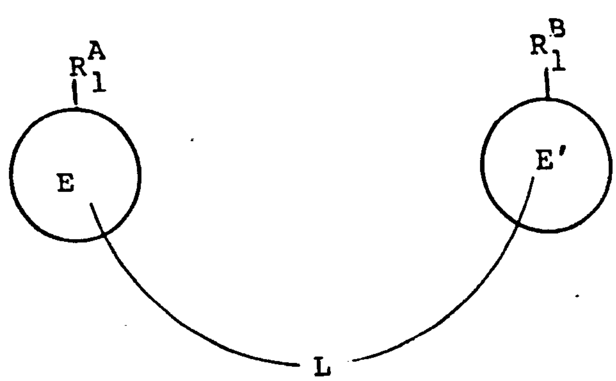

(IX)

wherein L E or E′ are as defined in relation to formula (I), $R^{A}_{1}$ represents $R^{A}$ as defined in relation to formula (I) or a moiety convertible to a moiety $R^{A}$; and $R^{B}_{1}$ represents $R^{B}$ as defined in relation to formula (I) or a moiety convertible to a group $R^{B}$, providing that $R^{A}_{1}$ is not $R^{A}$ when $R^{B}_{1}$ is $R^{B}$;

by, where appropriate;
(a) converting any group $R^{A}_{1}$ to $R^{A}$: and/or
(b) converting any group $R^{B}_{1}$ to $R^{B}$;

and thereafter if necessary carrying out one or more of the following steps:

(i) removing any protecting group;

(ii) converting a compound of formula (I) into a further compound of formula (I);

(iii) converting a salt of formula (I) into a free compound of formula (I);

(iv) preparing a pharmaceutically acceptable ester or amide of a compound of formula (I);

(v) preparing a pharmaceutically acceptable salt of a compound of formula (I) or an ester or amide thereof.

2. A process according to claim 1, for preparing a compound of formula (I) wherein E or E′ represents a substituted or unsubstituted phenylene or naphthylene group.

3. A process according to claim 1 or claim 2, for preparing a compound of formula (I) wherein $R^{O}$ and $R^{O}_{1}$ each independently represent a moiety of formula (c):

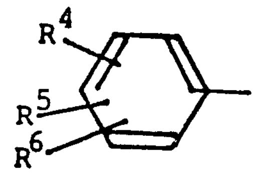

(c)

wherein $R^4$, $R^5$ and $R^6$ each independently represent hydrogen, halogen, alkyl, alkenyl, alkynyl, phenyl, alkoxy, trifluoromethyl, hydroxyalkyl, hydroxy, alkoxy amino, nitro, nitrile or carboxy.

4. A process according to any one of claims 1 to 3, for preparing a compound of formula (I) wherein $R^4$, $R^5$ and $R^6$ each independently represent hydrogen, halogen, trifluoromethyl, amino or hydroxy.

5. A process according to any one of claims 1 to 4, for preparing a compound of formula (I) wherein L comprises a substituted or unsubstituted hydrocarbon; or a chain of at least two atoms in length comprising at least one hetero atom selected from oxygen or substituted or unsubstituted nitrogen or sulphur; or L represents oxygen, an amino group or $SO_z$ wherein z is zero or 1 or 2.

6. A process according to any one of claims 1 to 5, for preparing a compound of formula (I) wherein L is a substituted or unsubstituted alkylene, alkenylene or alkynylene group.

7. A process according to any one of claims 1 to 5, for preparing a compound of formula (I) wherein L is a chain of from 2 to 30 atoms in length comprising at least one hetero atom selected from oxygen, nitrogen or

sulphur and a substituted or unsubstituted alkylene, alkenylene or alkynylene group preferably an alkylene group.

8. A process according to any one of claims 1 to 5, for preparing a compound of formula (I) wherein the moiety L comprises -O-, -S-, -SO-, $-SO_2-$, -C(O)-, -CR(OH)-, -CO.O-, -CON(R')- or -N(R')-, wherein R represents hydrogen, alkyl or hydroxyalkyl and R' represents hydrogen or alkyl, as part of the chain of from 2 to 30 atoms, especially as part of a chain also comprising a substituted or unsubstituted alkylene, alkenylene or alkynylene groups.

9. A process according to any one of claims 1 to 8, for preparing a compound of formula (I) wherein L is of formula $-X^1- X^2- X^3-$ wherein $X^1$ and $X^3$ each independently represent a bond, -C(O)-, RCOH, -CO.O- $-OX^{2A}CO_2-$, -CO.N(R')-, $-X^{2A}CO.N(R')-$, $-OX^{2A}CO.N(R')-$, $-OX^{2B}O-$, $-X^2N(R')-$, $-OX^{2A}N(R')-$, O, S, $-SO_2$, -N(R')-, $RC(OH)X^{2A}-$ or $-N(R')X^{2B}O-$; wherein R and R'are as defined above, $X^{2A}$ represents alkylene and $X^{2B}$ represents $C_{2-10}$ alkylene; and $X^2$ represents a substituted or unsubstituted alkylene, alkenylene, alkynylene or a moiety $-X^4-Z^1-X^5-$ wherein $X^4$ and $X^5$ each independently represent a bond, $C_{1-6}$ alkylene, $C_{2-6}$ alkylene or $C_{2-6}$ alkynylene, and wherein $Z^1$ represents -O-, S, -SO, $-SO_2$ or-NR' wherein R' is defined above.

10. A process according to any one of claims 1 to 9, for preparing a compound of formula (I) wherein L represents:

$-O(CH_2)_yCONH-(CH_2)_x-NHCO(CH_2)_yO-$,
$-O(CH_2)_yCONH-(CH_2)_x-NH(CH_2)_xO-$,
$-O(CH_2)_xNH-(CH_2)_x-NH(CH_2)_xO-$,
$-CONH(CH_2)_xNHCO-$,
$-(CH_2)_yCONH-(CH_2)_x-NHCO(CH_2)_y-$,
$-(CH_2)_yCONH-(CH_2)_x-NH(CH_2)_y-$,
$-(CH_2)_yNH-(CH_2)_x-NH(CH_2)_y-$,
$-O-(CH_2)_{y+1}-O-$,
$-(CH_2)_y-O-(CH_2)_y-$,
$-O(CH_2)_{y+1}-O-(CH_2)_{y+1}-O-$,
$-CO.O-(CH_2)_{y+1}-O.OC-$,
$-(CH_2)_y-$,
|
$-C(OH)-CH_2OH$,
$-O-$, or
$SO_z$; wherein

x represents an integer from 2 to 6;
y represents an integer from 1 to 10; and
z represents zero or an integer 1 or 2.

11. A process according to any one of claims 1 to 10, for preparing a compound of formula (I) wherein L represents $-O(CH_2)_{y+1}O-$, wherein y is an integer from 1 to 10.

12. A process according to any one of claims 1 to 11, for preparing a compound of formula (I) wherein L represents $-O(CH_2)_6O-$.

13. A process according to claim 1, for preparing a compound selected from the list consisting of:

[R,R,R,R]-N,N′-(1,2-ethanediyl)bis[2-[4-[2-[(2-(3-chlorophenyl)-2-hydroxyethyl)amino]propyl]phenoxy] acetamide];

[R,R,R,R]-α,α′[1,2-ethanediylbis(imino-2,1-ethanediyloxy-4,1-phenylene (1-methyl-2,1-ethanediyl) iminomethylene]]bis[3-chlorobenzenemethanol];

[R,R,R,R]-α,α'[1,6-hexanediylbis[oxy-4,1-phenylene(1-methyl-2,1-ethanediyl)iminomethylene]]bis[3-chlorobenzene methanol],

[R,R,R,R]-α,α′-[oxybis[4,1-phenylene(1-methyl-2,1-ethanediyl)iminomethylene]]bis[3-chlorobenzenemethanol];

[R,R,R,R]-α,α'-[methylenebis[4,1-phenylene(1-methyl-2,1-ethanediyl)iminomethylene]]bis[3-chlorobenzenemethanol];

[R,R,R,R]-α,α'-[sulphonylbis[4,1-phenylene(1-methyl-2,1-ethanediyl)iminomethylene]]bis[3-chlorobenzenemethanol];

[R,R,R,R]-1,1-di[4-[2-[[2-(3-chlorophenyl)-2-hydroxyethyl]amino]propyl]phenyl]-1,2-ethanediol;

[R,R,R,R]-α,α'-[1,8-octanediylbis[oxy-4,1-phenylene (1-methyl-2,1-ethanediyl)iminomethylene]]bis[3-chlorobenzenemethanol];

1,2-ethanediyl di[4-[2-[[2-hydroxy-2-(3-trifluoromethyl)phenylethyl]amino]propyl]benzoate];

1,2-ethanediyl di[4-[2-[[2-hydroxy-2-phenylethyl]amino] propyl] benzoate];

[R,R,R,R,]-α,α'[1,4-butanediylbis[oxy-4,1-phenylene (1-methyl-2,1-ethanediyl)iminomethylene]]bis[3-chlorobenzenemethanol], dihydrochloride.

[R,R,R,R,]-α,α'[1,3-propanediylbis[oxy-4,1-phenylene (1-methyl-2,1-ethanediyl)iminomethylene]]bis[3-chlorobenzenemethanol];

[R,R,R,R,]-α,α'[1,9-nonanediylbis[oxy-4,1-phenylene (1-methyl-2,1-ethanediyl)iminomethylene]]bis[3-chlorobenzenemethanol];

[R,R,R,R,]-3-chloro-α-[[[[2-[4-[6-[4-[2-[2-(4-hydroxyphenyl)-2-hydroxyethyl]amino]propyl]phenoxy] hexyloxy]phenyl]-1-methylethyl]amino]methyl]-benzenemethanol;

[R,R,R,R,]-α,α'-[1,2-ethanediylbis[iminomethylene-4,1-phenylene(1-methyl-2,1-ethanediyl)iminomethylene]]bis [3-chlorobenzenemethanol;

[R,R,R,R]-α,-α'-[1,4-butanediylbis[iminomethylene-4,1-phenylene(1-methyl-2,1-ethanediyl)iminomethylene]]bis [3-chlorobenzenemethanol;

[R,R,R,R]-α,α'-[1,6-hexanediylbis[iminomethylene-4,1-phenylene (1-methyl-2,1-ethanediyl)iminomethylene]] bis[3-chlorobenzenemethanol];

[R,R,R,R]-α,α'-[1,2-ethanediylbis[4,1-phenylene (1-methyl-2,1-ethanediyl)iminomethylene]] bis[3-chlorobenzenemethanol];

[R,R,R,R]-α,α'-[1,6-hexanediylbis[4,1-phenylene (1-methyl-2,1-ethanediyl)iminomethylene]] bis[3-chlorobenzenemethanol];

[R,R,R,R]-α,α'-[1,5-pentanediyl bis[oxy-4,1-phenylene (1-methyl-2,1-ethanediyl)iminomethylene]] bis[3-chlorobenzenemethanol];

[R,R,R,R]-α,α'-[oxybis[2,1-ethanediyloxy-4,1-phenylene (1-methyl-2,1-ethanediyl)iminomethylene]] bis[3-chlorobenzenemethanol];

[R,R,R,R]-5,5'-[1,6-hexanediylbis[oxy-4-1-phenylene (1-methyl-2,1-ethanediyl)imino(1-hydroxy-2,1-ethanediyl)]]bis[benzene-1,3-diol]; and

[R,R,R,R]-α,α'[1,7-heptanediylbis[oxy-4,1-phenylene-(1-methyl-2,1-ethanediyl)iminomethylene]]bis[3-chlorobenzenemethanol]; or a pharmaceutically acceptable acid addition salt thereof.